(19) Europäisches Patentamt
European Patent Office
Office européen des brevets

(11) Publication number: 0 044 212
B1

(12) **EUROPEAN PATENT SPECIFICATION**

(45) Date of publication of patent specification: **10.04.85**

(21) Application number: **81303179.6**

(22) Date of filing: **10.07.81**

(51) Int. Cl.[4]: **C 07 D 239/42,**
C 07 D 251/12,
C 07 D 491/048,
C 07 D 491/052,
C 07 D 239/52,
C 07 D 239/47,
C 07 C 143/828,
C 07 D 251/46,
C 07 D 239/70, C 07 D 253/06
// (C07D491/048, 239:00,
307:00),(C07D491/052,
239:00, 311:00)

(54) **Herbicidal o-alkylsulfonyloxy- and o-alkylsulfonylamino-benzenesulfonamides.**

(30) Priority: **11.07.80 US 168344**
**19.05.81 US 262813**

(43) Date of publication of application:
**20.01.82 Bulletin 82/03**

(45) Publication of the grant of the patent:
**10.04.85 Bulletin 85/15**

(84) Designated Contracting States:
**AT BE CH DE FR GB IT LI LU NL SE**

(56) References cited:
**EP-A-0 023 140**
**US-A-4 190 432**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Reap, James John**
**2505 Bona Road**
**Wilmington Delaware 19810 (US)**

(74) Representative: **Hildyard, Edward Martin et al**
**Frank B. Dehn & Co. Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 044 212 B1

**Description**

This invention relates to ortho(sulfonyl)oxybenzene sulfonamides and their use as herbicides.

Netherlands Patent 121,788, published September 15, 1966, teaches the preparation of compounds of Formula (I'), and their use as general or selective herbicides:

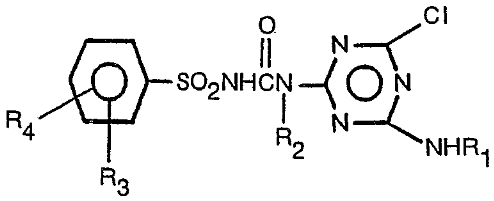
(I')

wherein

$R_1$ and $R_2$ may independently be alkyl of 1—4 carbon atoms; and

$R_3$ and $R_4$ may independently be hydrogen, chloride or alkyl of 1—4 carbon atoms.

Compounds of Formula (II'), and their use as antidiabetic agents, are reported in *J. Drug. Res. 6,* 123 (1974):

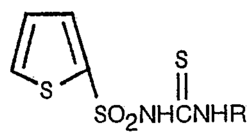
(II')

wherein

R is pyridyl.

In U.S. Patent 4,127,405, compounds are disclosed of the general formula:

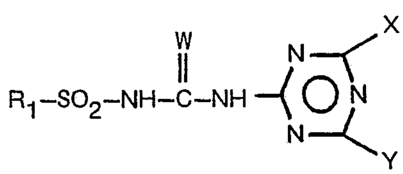

wherein

$R_1$ is

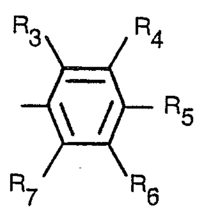 , 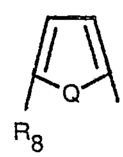 ,

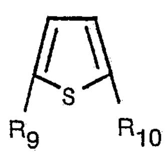 or 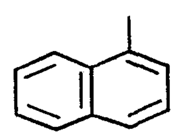 ;

$R_3$ and $R_6$ are independently hydrogen, fluorine, chlorine, bromine, iodine, alkyl of 1—4 carbon atoms, alkoxy of 1—4 carbon atoms, nitro, trifluoromethyl, cyano, $CH_3S(O)_n$— or $CH_3CH_2S(O)_n$—;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1—2 carbon atoms or alkoxy of 1—2 carbon atom;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1—3 carbon atoms, trifluoromethyl, $CH_3S$— or $CH_3OCH_2$—; and

Y is methyl or methoxy; or their agriculturally suitable salts; provided that:

(a) when $R_5$ is other than hydrogen at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of the $R_4$, $R_5$ and $R_6$ must be hydrogen.

In particular, the patent discloses ortho-substituted compounds wherein the substitution is $C_1$—$C_4$ alkyl.

Other herbicidal compounds of related structure to those claimed herein are disclosed in our US—A—4,190,432 and in our EP—A2—23,140. The compounds of the present invention are believed to be, in general, respectively more selective and more active than the compounds of these two earlier Specifications.

The presence of undesired vegetation causes substantial damage to useful crops, especially agricultural products that satisfy man's basic food needs, such as soybeans, corn, wheat and the like. The current population explosion and concomitant world food shortage demand improvements in the efficiency of producing these crops. Preventing or minimizing the loss of a portion of valuable crops by killing, or inhibiting the growth of undesired vegetation is one way of improving this efficiency.

A wide variety of materials useful for killing, or inhibiting (controlling) the growth of undesired vegetation is available; such materials are commonly referred to as herbicides. However, the need exists for still more effective herbicides that destroy or retard weeds without causing significant damage to useful crops.

We have now discovered novel compounds which may be used as general, as well as selective, pre- and post-emergence herbicides.

Thus, in one aspect, the invention provides compounds of general formula I

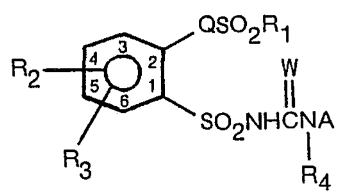

I

where

W is O or S;

Q is Q or $NR_5$;

$R_1$ is $C_1$—$C_4$ alkyl, $C_1$—$C_4$ alkyl substituted with 1—3 atoms of F, Cl or Br, $CH_2CH_2OCH_3$, $CH_2CH_2CH_2OCH_3$ or

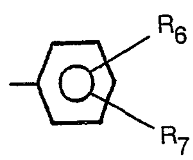

;

$R_2$ is H, F, Cl, Br, $OCH_3$, $NO_2$ or $C_1$—$C_2$ alkyl;

$R_3$ is H, F, Cl, Br or $CH_3$;

$R_4$ is H, $CH_3$ or $OCH_3$;

$R_5$ is $C_1$—$C_4$ alkyl;

$R_6$ and $R_7$ are independently H, F, Cl, Br, $CH_3$, $CF_3$, $NO_2$ or $OCH_3$;

A is

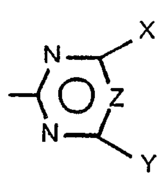

,

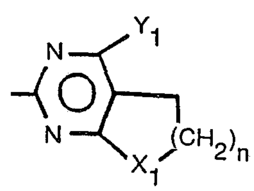

,

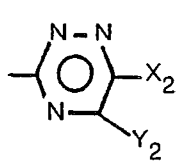

or

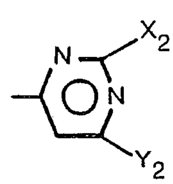

;

X is $NH_2$, $N(CH_3)_2$, $NHCH_3$, $C_1$—$C_4$ alkyl, $C_1$—$C_4$ alkyl substituted with 1—3 atoms of F, Cl or Br, $CH_2OCH_3$, $CH_2OCH_2CH_3$, $C_1$—$C_4$ alkoxy, $C_1$—$C_2$ alkylthio, $C_3$—$C_4$ alkenyloxy, $C_3$—$C_4$ alkynyloxy, $OCH_2CH_2OCH_3$ or $C_2$—$C_4$ alkoxy substitued with 1—3 atoms of F, Cl or Br;

n is 1 or 2;

Y is H, $CH_3$, $OCH_3$ or Cl;

$X_1$ is O or $CH_2$;

$Y_1$ is H, $CH_3$, $OCH_3$ or Cl;

$X_2$ and $Y_2$ are independently $CH_3$ or $OCH_3$; and

Z is CH, N, $CCH_3$, CBr, CCl, CF, CI, $CC_2H_5$, $CCH_2CH_2Cl$ or $CCH_2CH{=}CH_2$;

provided that

(1) when Y is Cl, then Z is other than N and X is $NH_2$, $NHCH_3$, $N(CH_3)_2$, $CH_3$ or $OCH_3$;

(2) when Y is H, then X is $OCH_3$, $CH_3$ or $CH_2OCH_3$, and Z is other than N; and

(3) when W is S, then $R_4$ is H.

Preferred for reasons of higher herbicidal activity and/or more favorable ease of synthesis are:

(1) Compounds of the generic scope where $R_5$ is $CH_3$, W is O, and $R_4$ is H or $CH_3$;

(2) Compounds of *Preferred (1)* where $R_1$ is $C_1$—$C_4$ alkyl, $CF_3$, $CH_2CH_2OCH_3$ or $CH_2CH_2CH_2OCH_3$;

(3) Compounds of *Preferred (2)* where $R_2$ is H;

(4) Compounds of *Preferred (3)* where $R_3$ is H;

(5) Compounds of *Preferred (4)* where

$R_1$ is $C_1$—$C_3$ alkyl or $CF_3$;

Q is O;

A is

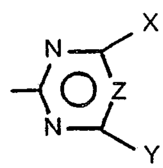 ;

and Z is CH or N.

(6) Compounds of *Preferred (5)* where $R_4$ is H; and

(7) Compounds of *Preferred (6)* where X and Y are independently $CH_3$ or $OCH_3$, and $R_1$ is $CH_3$.

Specifically preferred for particularly high herbicidal activity and/or particularly favorable ease of synthesis are:

N-[(4,6-dimethoxypyrimidin-2-yl)aminocarbonyl]-2-hydroxybenzenesulfonamide, methanesulfonate;

2-hydroxy-N-[(4-methoxy-6-methylpyrimidin-2-yl)aminocarbonyl]benzenesulfonamide, methanesulfonate; and

N-[(4,6-dimethylpyrimidin-2-yl)aminocarbonyl]-2-hydroxybenzenesulfonamide, methanesulfonate.

All of the above compounds show selectivity on wheat.

N-[(4,6-dimethoxy-1,3,5-triazin-2-yl)aminocarbonyl]-2-hydroxybenzenesulfonamide, methanesulfonate;

2-hydroxy-N-[(4-methoxy-6-methyl-1,3,5-triazin-2-yl)aminocarbonyl]benzenesulfonamide, methanesulfonate;

N-[(4,6-dimethyl-1,3,5-triazin-2-yl)aminocarbonyl]-2-hydroxybenzenesulfonamide, methanesulonate;

N-[(4,6-dimethoxy-1,3,5-triazin-2-yl)(methyl)aminocarbonyl]-2-hydroxybenzenesulfonamide, methanesulfonate;

N-[(4,6-dimethoxypropimidin-2-yl)aminocarbonyl]-2-hydroxybenzenesulfonamide, 1-propanesulfonate, which shows selectivity on rice and corn; and

2-hydroxy-N-[(4-methoxy-6-methyl-1,3,5-triazin-2-yl)aminocarbonyl]benzenesulfonamide, 1-propanesulfonate, which shows selectivity on soybeams.

This invention also relates to compounds of Formula II which are useful intermediates for the preparation of the herbicidal compounds of Formula I:

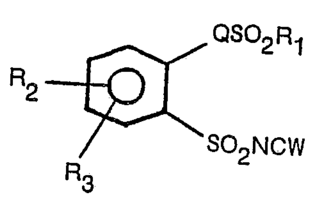

II

where W, Q, $R_1$, $R_2$ and $R_3$ are as defined above.

Preferred intermediates, for reasons of more favorable ease of synthesis and/or higher herbicidal activity of the compounds of Formula I, are:

(1) Compounds of Formula II where Q is O, $R_2$ and $R_3$ are H, $R_1$ is $CH_3$ or $CF_3$, and W is O; and

(2) Compounds of Formula II where Q is $NCH_3$, $R_2$ and $R_3$ are H, $R_1$ is $CH_3$ or $CF_3$, and W is O.

This invention also relates to compounds of Formula VII which are useful intermediates for the preparation of the herbicidal compounds of Formula I:

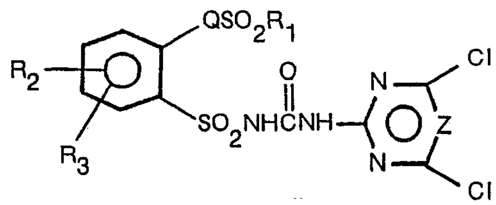

VII

where

Q is O or $NR_5$;

$R_1$ is $C_1$—$C_4$ alkyl, $C_1$—$C_4$ alkyl substituted with 1—3 atoms of F, Cl or Br, $CH_2CH_2OCH_3$; $CH_2CH_2CH_2OCH_3$, or

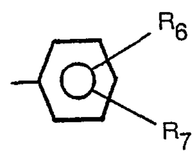

;

$R_2$ is H, F, Cl, Br, $OCH_3$, $NO_2$ or $C_1$—$C_2$ alkyl;
$R_3$ is H, F, Cl, Br or $OCH_3$;
$R_5$ is $C_1$—$C_4$ alkyl;
$R_6$ and $R_7$ are independently H, F, Cl, Br, $CH_3$, $CF_3$, $NO_2$ or $OCH_3$; and
Z is CH or N.

## Synthesis

The compounds of this invention can be made as outlined below. The compounds of Formula (IV), (VI), (XII) or (XIV) can be prepared by reacting an appropriate 2-aminopyrimidine, 2-aminotriazine or 2-aminobicyclopyrimidine of Formula (III), (V), (XI) or (XIII) with an appropriately substituted sulfonylisocyanate or isothiocyanate, of Formula (II), where Q, $R_1$, $R_2$, $R_3$, $R_4$, X, Y, Z, $X_1$, $X_2$, $Y_1$, $Y_2$, W and n were previously defined.

The reaction is best carried out in inert solvents such as methylene chloride and acetonitrile. The mode of addition is not critical, however, it is often convenient to add a solution of the isocyanate or isothiocyanate of Formula (II) to a stirred suspension of the aminoheterocycle (III), (V), (XI) or (XIII).

The reaction is generally exothermic. In some cases, the desired product is insoluble in the reaction medium and crystallizes from it in pure form. Products soluble in the reaction medium are isolated by evaporation of the solvent and trituration of the residue with solvents such as diethyl ether, 1-chlorobutane, or hexanes and filtration.

1.)

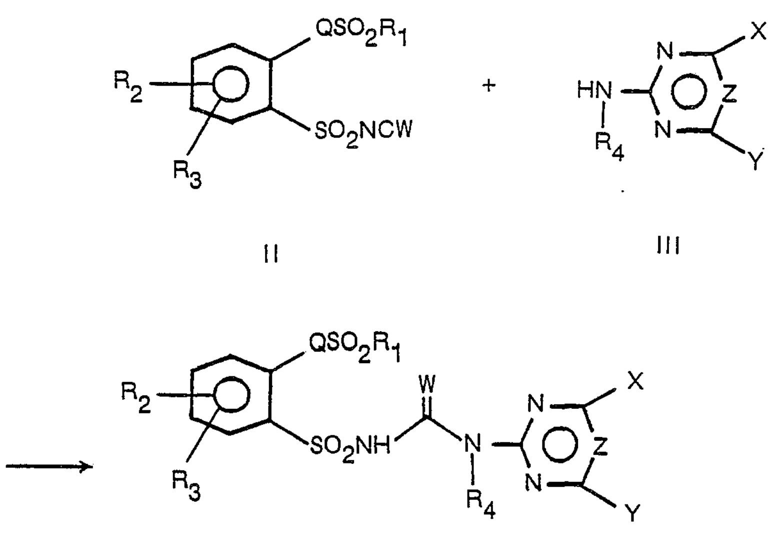

II

III

IV

2.)

II +

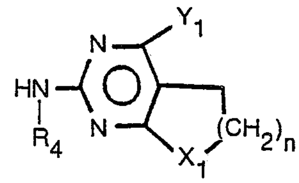

V

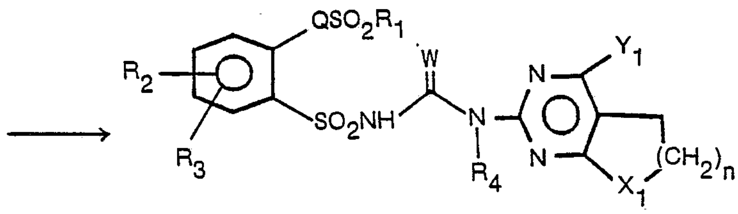

VI

3.)

II +

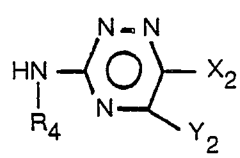

XI

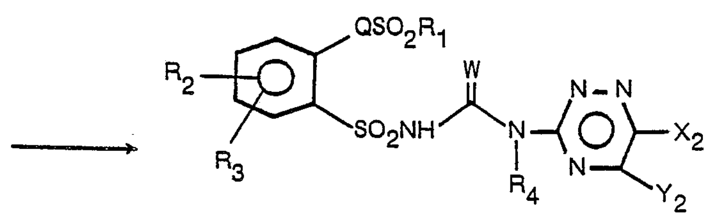

XII

4.)

II +

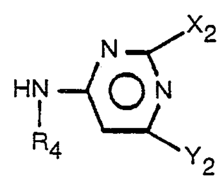

XIII

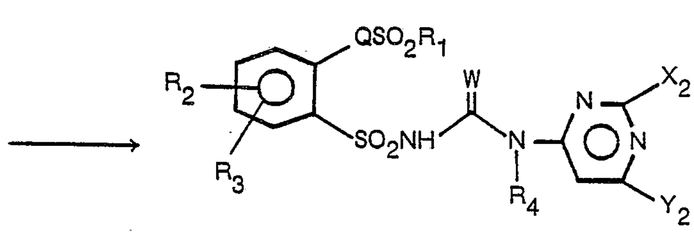

XIV

Compounds of this invention may also be prepared by reacting a sulfonamide of Formula VIII with a 4,6-dichloroheterocyclic isocyanate of Formula IX.

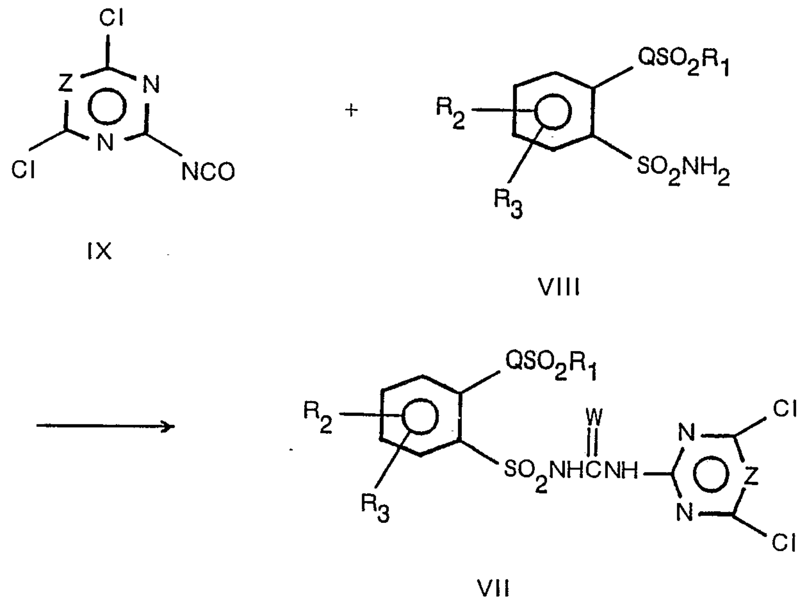

IX

VIII

VII

One or both of the halogen atoms on the heterocyclic ring of the compound of Formula VII can be displaced by an alkoxide of $OR_8$, to give compounds of Formula X, where $R_8$ is $C_1$—$C_4$ alkyl, $C_3$—$C_4$ alkenyl, $C_3$—$C_4$ alkynyl, $CH_2CH_2OCH_3$ or $C_2$—$C_4$ alkyl substituted with 1—3 atoms of F, Cl or Br, and Y'' is Cl or $OCH_3$.

VII

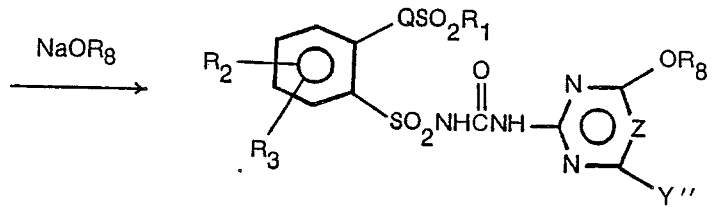


X

The process for the preparation of compounds of Formula X is described in our European Patent Specification No. 0030140, published 10 June 1981.

Sulfonamides, prepared as described in Research Disclosure, pg. 52, (1978), may conveniently be converted to the corresponding isocyanates or isothiocyanates of Formula II by methods described in U.S. Patent No. 4,127,405.

The synthesis of the heterocyclicamine derivatives has been reviewed in "The Chemistry of Heterocyclic Compounds", a series published in Interscience Publ., New York and London.

2-Aminopyrimidines are described by D. J. Brown in "The Pyrimidines", Vol. XVI of the above series which are herein incorporated by reference.

The preparation of amino bicyclopyrimidines are described in unexamined European Patent No. 15683.

The aminoheterocyclic intermediates of Formula III, V, XI or XIII in which $R_4$ is $CH_3$, may be prepared by the following procedure, or by obvious modifications thereof.

XV

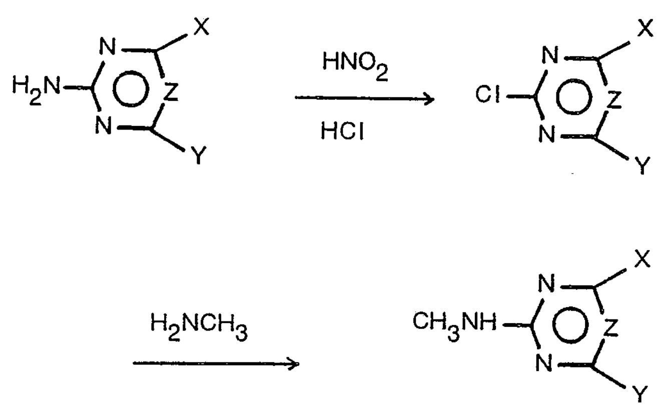


XVI

XVII

A solution of the amine XV in concentrated hydrochloric acid is contacted with an aqueous sodium nitrite solution and the chloro compound XVI is isolated by filtration of the acidic solution (see for example, Bee and Rose, *J. Chem. Soc. C.,* 2051 (1966) for the case in which Z is CH and X and Y are $OCH_3$). Displacement of the chlorine may be accomplished by heating with an excess of methylamine in water to obtain the methylaminoheterocycle XVII.

N-Methoxyamino heterocycles can be prepared by procedures reported on the literature [see, for example, Belgian Patent 618,563 and J. T. Shaw, et al., *J. Org. Chem., 27* 4054 (1962)] and the procedure illustrated below.

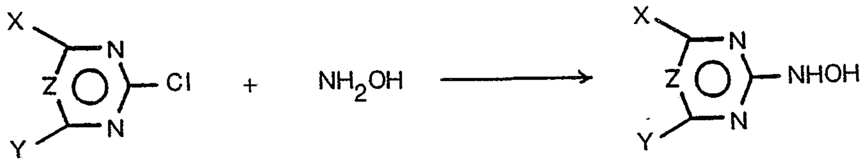


XVI

XVIII

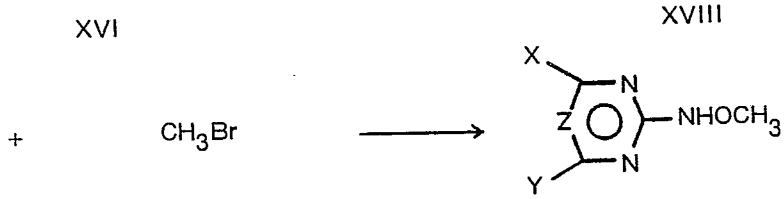


XIX

XVI + $NH_2OCH_3 \cdot HCl$ —NaOH→ XIX

Chloro compound XVI is reacted with hydroxylamine to form derivative XVIII which may be alkylated with methyl bromide to afford the N-methoxy heterocyclic amine XIX. This compound may alternatively be prepared in one step by treatment of XVI and O-methyl hydroxylamine hydrochloride with an alkali metal hydroxide such as sodium hydroxide.

The compounds of this invention and their preparation are further illustrated by the following examples, wherein temperatures are given in degrees centigrade and parts are by weight unless otherwise indicated.

Example 1

2-Hydroxybenzenesulfonyl isocyanate, methanesulfonate

To 10.0 g (.04 mole) 2-hydroxybenzenesulfonamide, methanesulfonate, suspended in 75 ml dry xylenes was added 4.0 g (.04 mole) N-butylisocyanate and a catalytic amount of 1,4-diazobicyclo-[2.2.2]octane (~.05 g). This mixture was rapidly heated to reflux temperature (~135°C) and 4.0 ml of phosgene was slowly added (at such as rate as to keep the reaction temperature greater than 128°C). The phosgene addition required ~1½ hours. The reaction was cooled to room temperature, filtered under $N_2$, and the solvent evaporated under reduced pressure. The infrared spectrum of the resultant oil was consistent for 2-hydroxybenzenesulfonyl isocyanate, methanesulfonate (2210 $cm^{-1}$).

Example 2

N-[(4,6-Dimethoxypyrimidin-2-yl)aminocarbonyl]-2-hydroxybenzenesulfonamide, methanesulfonate

To a suspension of .6 g 2-amino-4,6-dimethoxypyrimidine in 10 ml dry methylene chloride is added 1.1 g 2-hydroxybenzenesulfonylisocyanate, methanesulfonate. The reaction exotherms (21°C—29°C) and is then stirred ½ hour. The solvent is evaporated under reduced pressure. The resultant mixture is triturated with diethylether and filtered to give 1.2 g tan solid m.p. 165—166°C. The infrared spectrum shows absorption bands at 1740, 1615, 1590, 1360 $cm^{-1}$.

Calc. for C 38.9, H 3.73, N 12.9.
Found C 38.3, H 3.63, N 13.0.

Example 3

2-Hydroxy-N-[(4-methoxy-6-methylpyrimidin-2-yl)aminocarbonyl]benzenesulfonamide, methanesulfonate.

To a suspension of .6 g 2-amino-4-methoxy-6-methylpyrimidine in 10 ml dry methylene chloride is added 1.1 g 2-hydroxybenzenesulfonylisocyanate, methanesulfonate. The reaction exotherms mildly and

is then stirred for ½ hour. The solvent is evaporated under reduced pressure. The resultant mixture is triturated with diethylether and filtered to give .95 g white solid m.p. 189—190°C. The infrared spectrum shows adsorption bands at 1700, 1615, 1560, 1370 $cm^{-1}$.

Calc. for C 40.4, H 3.87, N 13.4.
Found C 40.2, H 3.77, N 13.6.

Example 4

N-[(4,6-dimethoxy-1,3,5-triazin-2-yl)aminocarbonyl]-2-hydroxybenzenesulfonamide, methanesulfonate

To a suspension of .6 g of 2-amino-4,6-dimethoxy-1,3,5-triazine in 10 ml dry methylene chloride is added 1.1 g 2-hydroxybenzenesulfonylisocyanate, methanesulfonate. The reaction exotherms mildly and is then stirred for about 1 hour. The solvent is evaporated under reduced pressure. The resultant mixture is triturated with diethylether and filtered to give 1.4 g tan solid m.p. 163—166°C. The infrared spectrum shows absorption bands at 1715, 1600, 1550, 1350 $cm^{-1}$.

Using the procedures similar to those of Examples 1—4, the following compounds can be prepared.

TABLE I

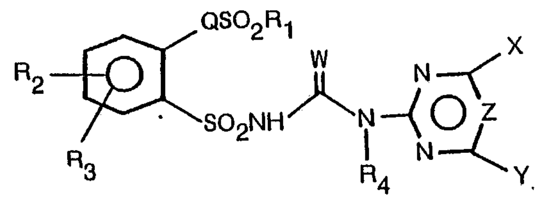

| $R_1$ | $R_2$ | $R_3$ | $R_4$ | Q | W | Y | X | Z | m.p. (°C) |
|---|---|---|---|---|---|---|---|---|---|
| $CH_3$ | H | H | H | O | O | $OCH_3$ | $OCH_3$ | CH | 165–166 |
| $CH_3$ | H | H | H | O | O | $OCH_3$ | $CH_3$ | CH | 189–190 |
| $CH_3$ | H | H | H | O | O | $CH_3$ | $CH_3$ | CH | 204–205 |
| $CH_3$ | H | H | H | O | O | $OCH_3$ | $OCH_3$ | N | 163–166 |
| $CH_3$ | H | H | H | O | O | $OCH_3$ | $CH_3$ | N | 170–173 |
| $CH_3$ | H | H | H | O | O | $CH_3$ | $CH_3$ | N | 181–184 |
| $(CH_2)_3CH_3$ | H | H | H | O | O | $OCH_3$ | $OCH_3$ | CH | 158–162 |
| $(CH_2)_2CH_2Cl$ | H | H | H | O | O | $OCH_3$ | $OCH_3$ | CH | |
| $CCl_3$ | H | H | H | O | O | $OCH_3$ | $OCH_3$ | CH | |
| 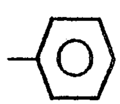 | H | H | H | O | O | $OCH_3$ | $CH_3$ | CH | 216–219 |

TABLE I (Continued)

| $R_1$ | $R_2$ | $R_3$ | $R_4$ | Q | W | Y | X | Z | m.p. (°C) |
|---|---|---|---|---|---|---|---|---|---|
| 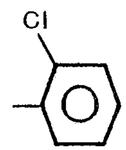 | H | H | H | O | O | $OCH_3$ | $CH_3$ | CH | |
| 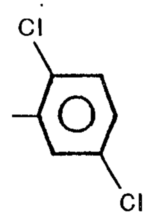 | H | H | H | O | O | $OCH_3$ | $CH_3$ | CH | |
| 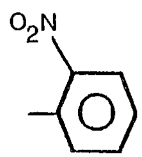 | H | H | H | O | O | $OCH_3$ | $CH_3$ | CH | |
| 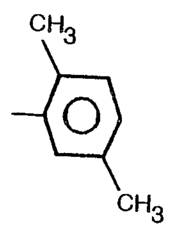 | H | H | H | O | O | $OCH_3$ | $CH_3$ | CH | |

TABLE I (Continued)

| $R_1$ | $R_2$ | $R_3$ | $R_4$ | Q | W | Y | X | Z | m.p. (°C) |
|---|---|---|---|---|---|---|---|---|---|
| 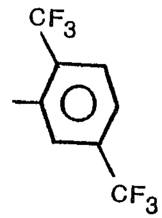 | H | H | H | O | O | $OCH_3$ | $CH_3$ | CH | |
| 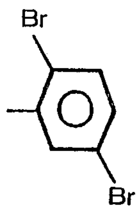 | H | H | H | O | O | $OCH_3$ | $CH_3$ | CH | |
| 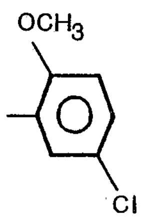 | H | H | H | O | O | $OCH_3$ | $CH_3$ | CH | |
| 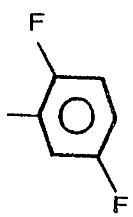 | H | H | H | O | O | $OCH_3$ | $CH_3$ | CH | |

TABLE I (Continued)

| R1 | R2 | R3 | R4 | Q | W | Y | X | Z | m.p. (°C) |
|---|---|---|---|---|---|---|---|---|---|
| $CH_3$ | 5—Cl | H | H | O | O | $OCH_3$ | $OCH_3$ | CH | 174—176 |
| $CH_3$ | 6—$CH_3$ | H | H | O | O | $OCH_3$ | $CH_3$ | CH | |
| $CH_3$ | 5—F | H | H | O | O | $OCH_3$ | $CH_3$ | CH | |
| $CH_3$ | 5—$NO_2$ | H | H | O | O | $OCH_3$ | $CH_3$ | CH | |
| $CH_3$ | 5—Br | H | H | O | O | $OCH_3$ | $CH_3$ | CH | |
| $CH_3$ | 5—$OCH_3$ | H | H | O | O | $OCH_3$ | $CH_3$ | CH | |
| $CH_3$ | 5—$CF_3$ | H | H | O | O | $OCH_3$ | $CH_3$ | CH | |
| $CH_3$ | 5—Cl | 4—Cl | H | O | O | $OCH_3$ | $CH_3$ | CH | |
| $CH_3$ | 5—$CH_3$ | 4—$CH_3$ | H | O | O | $OCH_3$ | $CH_3$ | CH | |
| $CH_3$ | H | H | $CH_3$ | O | O | $OCH_3$ | $OCH_3$ | N | 91—96 |
| $CH_3$ | H | H | H | —N—$CH_3$ | O | $OCH_3$ | $OCH_3$ | CH | |
| $CH_3$ | H | H | H | —N($CH_2$)$_3$ with $CH_3$ | O | $OCH_3$ | $OCH_3$ | CH | |
| $CH_3$ | H | H | H | O | O | Cl | $OCH_3$ | CH | |
| $CH_3$ | H | H | H | O | O | $OCH_3$ | —$(CH_2)_3CH_3$ | CH | |
| $CH_3$ | H | H | H | O | O | $CH_3$ | —$OCH_2CF_3$ | N | |

TABLE I (Continued)

| $R_1$ | $R_2$ | $R_3$ | $R_4$ | Q | W | Y | X | Z | m.p. (°C) |
|---|---|---|---|---|---|---|---|---|---|
| $CH_3$ | H | H | H | O | O | $CH_3$ | $-OCH_3CCl_3$ | N | |
| $CH_3$ | H | H | H | O | O | $CH_3$ | $-OCH_2CBr_3$ | N | |
| $CH_3$ | H | H | H | O | O | $CH_3$ | $OCH_2CH_2Cl$ | N | |
| $CH_3$ | H | H | H | O | O | $CH_3$ | H | CH | |
| $CH_3$ | H | H | H | O | O | $CH_3$ | $(CH_2)_3CH_3$ | CH | |
| $CH_3$ | H | H | H | O | O | $CH_3$ | $-CH_2CH_2Cl$ | CH | |
| $CH_3$ | H | H | H | O | O | $CH_3$ | $-CH_2CF_3$ | CH | |
| $CH_3$ | H | H | H | O | O | $CH_3$ | $-O(CH_2)_3CH_3$ | N | |
| $CH_3$ | H | H | H | O | O | $CH_3$ | $-OCH_2CH{=}CH_2$ | N | |
| $CH_3$ | H | H | H | O | O | $CH_3$ | $-OCH_2C(CH_3){=}CH_2$ | N | |
| $CH_3$ | H | H | H | O | O | $CH_3$ | $OCH_2C{\equiv}CH$ | N | |
| $CH_3$ | H | H | H | O | O | $CH_3$ | $OCH_2C{\equiv}CCH_3$ | N | |
| $CH_3$ | H | H | H | O | O | $CH_3$ | $-CH_2OCH_3$ | CH | |
| $CH_3$ | H | H | H | O | O | $CH_3$ | $SCH_3$ | CH | |
| $CH_3$ | H | H | H | O | O | $CH_3$ | $S(CH_2)CH_3$ | CH | |
| $CH_3$ | H | H | H | O | O | $CH_3$ | $CH_3$ | CBr | |

TABLE I (Continued)

| $R_1$ | $R_2$ | $R_3$ | $R_4$ | Q | W | Y | X | Z | m.p. (°C) |
|---|---|---|---|---|---|---|---|---|---|
| $CH_3$ | H | H | H | O | O | $CH_3$ | $CH_3$ | CCl |  |
| $CH_3$ | H | H | H | O | O | $CH_3$ | $CH_3$ | CF |  |
| $CH_3$ | H | H | H | O | O | $CH_3$ | $CH_3$ | CCl |  |
| $CH_3$ | H | H | H | O | S | $OCH_3$ | $OCH_3$ | N |  |
| $CH_3$ | H | H | H | O | S | $OCH_3$ | $CH_3$ | N |  |
| $CH_3$ | H | H | H | O | S | $CH_3$ | $CH_3$ | N |  |
| $CH_3$ | H | H | H | O | S | $OCH_3$ | $CH_3$ | CH |  |
| $CH_3$ | H | H | H | O | S | $OCH_3$ | $OCH_3$ | CH |  |
| $CH_3$ | H | H | H | O | S | $CH_3$ | $CH_3$ | CH |  |
| $CH_3$ | 3–Cl | H | H | O | O | $OCH_3$ | $OCH_3$ | CH | 134–142 |
| $CH_3$ | 3–Cl | H | H | O | O | $OCH_3$ | $OCH_3$ | N | 165–170 |
| $CH_3$ | 3–Cl | 5–Cl | H | O | O | $OCH_3$ | $CH_3$ | N | 210(dec) |
| $CH_3$ | 3–Cl | 5–Cl | H | O | O | $OCH_3$ | $OCH_3$ | N | 200(dec) |
| $CH_3$ | H | H | H | $NCH_2CH_3$ | O | $OCH_3$ | $OCH_3$ | CH |  |
| $CH_3$ | H | H | H | $N(CH_2)_2CH_3$ | O | $OCH_3$ | $OCH_3$ | CH |  |
| $CH_3$ | H | H | H | $NCH_3$ | O | $OCH_3$ | $CH_3$ | CH |  |
| $CH_3$ | H | H | H | $NCH_3$ | O | $CH_3$ | $CH_3$ | CH |  |
| $CH_3$ | H | H | H | $NCH_3$ | O | $OCH_3$ | $CH_3$ | N |  |

TABLE I (Continued)

| $R_1$ | $R_2$ | $R_3$ | $R_4$ | Q | W | Y | X | Z | m.p. (°C) |
|---|---|---|---|---|---|---|---|---|---|
| $CH_2CH_3$ | H | H | H | O | O | $OCH_3$ | $OCH_3$ | CH | 189–191 |
| $CH_2CH_3$ | H | H | H | O | O | $OCH_3$ | $CH_3$ | CH | 160–162 |
| $CH_2CH_3$ | H | H | H | O | O | $CH_3$ | $CH_3$ | CH | 196–198 |
| $CH_2CH_3$ | H | H | H | O | O | $OCH_3$ | $OCH_3$ | N | |
| $CH_2CH_3$ | H | H | H | O | O | $OCH_3$ | $CH_3$ | N | |
| $CH_2CH_3$ | H | H | H | O | O | $CH_3$ | $CH_3$ | N | |
| $CH_2CH_3$ | H | H | $CH_3$ | O | O | $OCH_3$ | $OCH_3$ | N | |
| $(CH_2)_3CH_3$ | H | H | H | O | O | $OCH_3$ | $CH_3$ | CH | |
| $(CH_2)_3CH_3$ | H | H | H | O | O | $CH_3$ | $CH_3$ | CH | 201–202 |
| $(CH_2)_3CH_3$ | H | H | H | O | O | $OCH_3$ | $OCH_3$ | N | 150–153 |
| $(CH_2)_3CH_3$ | H | H | H | O | O | $OCH_3$ | $CH_3$ | N | 161–163 |
| $(CH_2)_3CH_3$ | H | H | H | O | O | $CH_3$ | $CH_3$ | N | |
| $(CH_2)_3CH_3$ | H | H | H | O | O | H | $CH_3$ | CCl | 135–140 |
| $(CH_2)_2CH_3$ | H | H | H | O | O | $OCH_3$ | $OCH_3$ | CH | 172–174 |
| $(CH_2)_2CH_3$ | H | H | H | O | O | $OCH_3$ | $CH_3$ | CH | 183–186 |
| $(CH_2)_2CH_3$ | H | H | H | O | O | $CH_3$ | $CH_3$ | CH | 182–184 |
| $(CH_2)_2CH_3$ | H | H | H | O | O | $OCH_3$ | $OCH_3$ | N | |

TABLE I (Continued)

| $R_1$ | $R_2$ | $R_3$ | $R_4$ | Q | W | Y | X | Z | m.p. (°C) |
|---|---|---|---|---|---|---|---|---|---|
| $(CH_2)_2CH_3$ | H | H | H | O | O | $OCH_3$ | $CH_3$ | N | 144–147 |
| $(CH_2)_2CH_3$ | H | H | H | O | O | $CH_3$ | $CH_3$ | N | |
| $(CH_2)_2CH_3$ | H | H | $CH_3$ | O | O | $OCH_3$ | $OCH_3$ | CH | |
| $(CH_2)_2CH_3$ | H | H | H | O | O | H | $CH_3$ | CCl | 185–190 |
| $CF_3$ | H | H | H | O | O | $OCH_3$ | $OCH_3$ | CH | 129–131 |
| $CH_2CH_2Br$ | H | H | H | O | O | $OCH_3$ | $OCH_3$ | CH | |
| $CH_2CH_2OCH_3$ | H | H | H | O | O | $OCH_3$ | $OCH_3$ | CH | |
| $CH_2CH_2OCH_3$ | H | H | H | O | O | $OCH_3$ | $CH_3$ | CH | |
| $CH_2CH_2OCH_3$ | H | H | H | O | O | $CH_3$ | $CH_3$ | CH | |
| $CH_2CH_2OCH_3$ | H | H | H | O | O | $OCH_3$ | $CH_3$ | N | |
| $(CH_2)_2CH_2OCH_3$ | H | H | H | O | O | $OCH_3$ | $CH_3$ | CH | |
| $CH_3$ | H | H | H | O | O | Cl | $NH_2$ | CH | |
| $CH_3$ | H | H | H | O | O | Cl | $NHCH_3$ | CH | |
| $CH_3$ | H | H | H | O | O | Cl | $N(CH_3)_2$ | CH | |
| $CH_3$ | H | H | H | O | O | $CH_3$ | $CH_2OCH_3$ | CH | |
| $CH_2CH_3$ | H | H | H | O | O | $CH_3$ | $CH_2OCH_3$ | CH | |
| $(CH_2)_2CH_3$ | H | H | H | O | O | $CH_3$ | $CH_2OCH_3$ | CH | |
| $CH_3$ | H | H | H | O | O | $CH_3$ | $CH_2OCH_2CH_3$ | CH | |
| $CH_3$ | H | H | H | O | O | $CH_3$ | $CH_2CH_3$ | CH | |

TABLE I (Continued)

| $R_1$ | $R_2$ | $R_3$ | $R_4$ | Q | W | Y | X | Z | m.p. (°C) |
|---|---|---|---|---|---|---|---|---|---|
| $CH_3$ | H | H | H | O | O | $CH_3$ | $CH_2CH_2CH_3$ | CH | |
| $CH_3$ | H | H | H | O | O | $CH_3$ | $CH_2CH_2Br$ | CH | |
| $CH_3$ | H | H | H | O | O | $CH_3$ | $OCH_2CH_3$ | CH | |
| $CH_3$ | H | H | H | O | O | $CH_3$ | $O(CH_2)_2CH_3$ | CH | |
| $CH_3$ | H | H | H | O | O | $CH_3$ | $OCH_2CH=CH_2$ | CH | |
| $CH_3$ | H | H | H | O | O | $CH_3$ | $OCH_2C(CH_3)=CH_2$ | CH | |
| $CH_3$ | H | H | H | O | O | $CH_3$ | $OCH_2C\equiv CH$ | CH | |
| $CH_3$ | H | H | H | O | O | $CH_3$ | $OCH_2C\equiv CCH_3$ | CH | |
| $CH_3$ | H | H | H | O | O | $CH_3$ | $OCH_2CH_2OCH_3$ | CH | |
| $CH_3$ | H | H | H | O | O | $CH_3$ | $OCH_2CH_2OCH_3$ | N | |
| $CH_3$ | H | H | H | O | O | $CH_3$ | $OCH_2(CH_2)_2Cl$ | CH | |
| $CH_3$ | H | H | H | O | O | $CH_3$ | $OCH_2(CH_2)_3Cl$ | CH | |
| $CH_3$ | H | H | H | O | O | H | $CH_3$ | CCl | |
| $CH_3$ | H | H | H | O | O | Cl | $CH_3$ | $OCH_2CH_2Cl$ | |
| $CH_3$ | H | H | H | O | O | $OCH_3$ | $OCH_3$ | $CH_3$ | |
| $CH_3$ | H | H | H | O | O | $OCH_3$ | $OCH_3$ | $CH_2CH_3$ | |

TABLE I (Continued)

| $R_1$ | $R_2$ | $R_3$ | $R_4$ | Q | W | Y | X | Z | m.p. (°C) |
|---|---|---|---|---|---|---|---|---|---|
| $CH_3$ | H | H | H | O | O | $OCH_3$ | $OCH_3$ | $CCH_2CH{=}CH_2$ | |
| $CH_3$ | 5–Cl | H | H | O | O | $OCH_3$ | $CH_3$ | CH | 179–181 |
| $CH_3$ | 5–Cl | H | H | O | O | $CH_3$ | $CH_3$ | CH | 204–205 |
| $CH_3$ | 5–Cl | H | H | O | O | $OCH_3$ | $OCH_3$ | N | 180–186 |
| $CH_3$ | 5–Cl | H | H | O | O | $OCH_3$ | $CH_3$ | N | 184–187 |
| $CH_3$ | 5–Cl | H | H | O | O | H | $CH_3$ | CCl | 210–211 |
| $CH_3$ | 5–$CH_3$ | H | H | O | O | $OCH_3$ | $OCH_3$ | CH | 171–174 |
| $CH_3$ | 5–$CH_3$ | H | H | O | O | $OCH_3$ | $CH_3$ | CH | 181–188 |
| $CH_3$ | 5–$CH_3$ | H | H | O | O | $CH_3$ | $CH_3$ | CH | 180–183 |
| $CH_3$ | 5–$CH_3$ | H | H | O | O | $OCH_3$ | $CH_3$ | N | 159–161 |
| $CH_3$ | 5–$CH_3$ | H | H | O | O | $OCH_3$ | $OCH_3$ | N | 178–181 |
| $CH_3$ | 5–$CH_3$ | H | H | O | O | $CH_3$ | $CH_3$ | N | |
| $CH_3$ | 5–$CH_3$ | H | $CH_3$ | O | O | $OCH_3$ | $OCH_3$ | N | 133–136 |
| $CH_3$ | 5–$CH_2CH_3$ | H | H | O | O | $OCH_3$ | $CH_3$ | CH | |
| 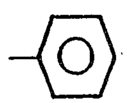 | H | H | H | O | O | $OCH_3$ | $OCH_3$ | CH | 221–223 |
| 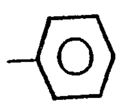 | H | H | H | O | O | $CH_3$ | $CH_3$ | CH | 182–186 |

TABLE I (Continued)

| $R_1$ | $R_2$ | $R_3$ | $R_4$ | Q | W | Y | X | Z | m.p. (°C) |
|---|---|---|---|---|---|---|---|---|---|
| 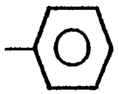 | H | H | H | O | O | $OCH_3$ | $CH_3$ | N | 184–186 |
| 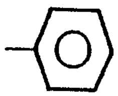 | H | H | H | O | O | $OCH_3$ | $OCH_3$ | N | 169–172 |
| 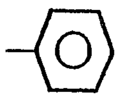 | H | H | H | O | O | $CH_3$ | $CH_3$ | N | |
| 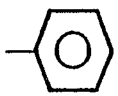 | H | H | H | O | O | H | $CH_3$ | CCl | 193–195 |
| 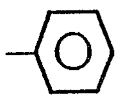 | H | H | $CH_3$ | O | O | $OCH_3$ | $OCH_3$ | N | 143–145 |
| $CH_3$ | H | H | $OCH_3$ | O | O | $OCH_3$ | $OCH_3$ | CH | |
| 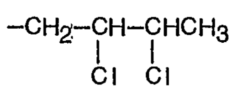 | H | H | H | O | O | $OCH_3$ | $OCH_3$ | CH | |
| $CF_3$ | H | H | H | O | O | $OCH_3$ | $CH_3$ | CH | 135–140 (dec) |
| $CF_3$ | H | H | H | O | O | $CH_3$ | $CH_3$ | CH | 190–193 (dec) |

TABLE I (Continued)

| $R_1$ | $R_2$ | $R_3$ | $R_4$ | Q | W | Y | X | Z | m.p. (°C) |
|---|---|---|---|---|---|---|---|---|---|
| $CF_3$ | H | H | H | O | O | $OCH_3$ | $OCH_3$ | N | |
| $CF_3$ | H | H | H | O | O | $OCH_3$ | $CH_3$ | N | |
| $CF_3$ | H | H | H | O | O | $CH_3$ | $CH_3$ | N | 200 (dec) |
| $CF_3$ | H | H | $CH_3$ | O | O | $OCH_3$ | $OCH_3$ | N | 144–148 (dec) |
| $CF_3$ | H | H | H | $N-CH_3$ | O | $OCH_3$ | $OCH_3$ | CH | |
| $CF_3$ | H | H | H | $N-CH_3$ | O | $OCH_3$ | $CH_3$ | CH | |
| $CF_3$ | H | H | H | $N-CH_3$ | O | $CH_3$ | $CH_3$ | CH | |
| $CF_3$ | H | H | H | $N-CH_3$ | O | $OCH_3$ | $OCH_3$ | N | |
| $CF_3$ | H | H | H | $N-CH_3$ | O | $OCH_3$ | $CH_3$ | N | |
| $CF_3$ | H | H | H | $N-CH_3$ | O | $CH_3$ | $CH_3$ | N | |
| $CF_3$ | H | H | $CH_3$ | $N-CH_3$ | O | $OCH_3$ | $OCH_3$ | N | |

TABLE II

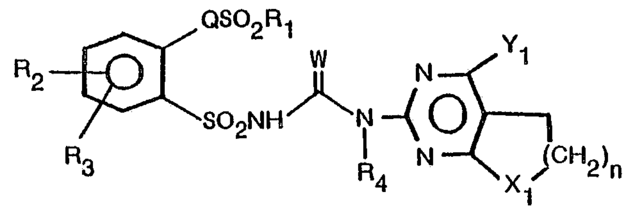

| $R_1$ | $R_2$ | $R_3$ | $R_4$ | Q | W | $X_1$ | $Y_1$ | n | m.p. (°C) |
|---|---|---|---|---|---|---|---|---|---|
| $CH_3$ | H | H | H | O | O | O | H | 1 | |
| $CH_3$ | H | H | H | O | O | O | $CH_3$ | 1 | |
| $CH_3$ | H | H | H | O | O | O | $OCH_3$ | 1 | |
| $CH_3$ | H | H | H | O | O | O | H | 2 | |
| $CH_3$ | H | H | H | O | O | O | $CH_3$ | 2 | |
| $CH_3$ | H | H | H | O | O | O | $OCH_3$ | 2 | |
| $CH_3$ | H | H | H | $-N(CH_3)-$ | O | O | $OCH_3$ | 2 | |
| $CH_3$ | 5–Cl | H | H | O | O | O | $CH_3$ | 1 | |
| $CH_3$ | 5–Cl | H | H | O | O | O | $OCH_3$ | 1 | |
| $CH_3$ | 5–Cl | H | H | O | O | $CH_2$ | $CH_3$ | 1 | |
| $CH_3$ | 5–Cl | H | H | O | O | $CH_2$ | $OCH_3$ | 1 | |

TABLE II (Continued)

| $R_1$ | $R_2$ | $R_3$ | $R_4$ | Q | W | $X_1$ | $Y_1$ | n | m.p. (°C) |
|---|---|---|---|---|---|---|---|---|---|
| $CH_3$ | 5–Cl | H | H | O | O | O | $CH_3$ | 2 | |
| $CH_3$ | 5–Cl | H | H | O | O | O | $OCH_3$ | 2 | |
| $-(CH_2)_3CH_3$ | H | H | H | O | O | O | $OCH_3$ | 1 | |
| $(CH_2)_2CH_2Cl$ | H | H | H | O | O | O | $OCH_3$ | 1 | |
| 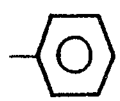 | H | H | H | O | O | O | $OCH_3$ | 1 | |
| 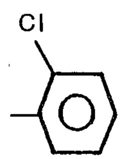 | H | H | H | O | O | O | $OCH_3$ | 1 | |
| 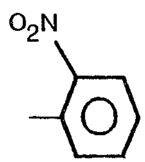 | H | H | H | O | O | O | $OCH_3$ | 1 | |
| $(CH_2)_2CH_3$ | H | H | H | O | O | O | $OCH_3$ | 1 | |
| $CH_2CH_2Cl$ | H | H | H | O | O | O | $OCH_3$ | 1 | |
| $CH_2CH_2Cl$ | H | H | H | O | O | O | $CH_3$ | 1 | |
| $CH_2CH_2Cl$ | H | H | H | O | O | O | $OCH_3$ | 2 | |
| $CH_3$ | 5–F | H | H | O | O | O | $OCH_3$ | 1 | |

TABLE II (Continued)

| $R_1$ | $R_2$ | $R_3$ | $R_4$ | Q | W | $X_1$ | $Y_1$ | n | m.p. (°C) |
|---|---|---|---|---|---|---|---|---|---|
| $CH_3$ | 5–Cl | 4–Cl | H | O | O | O | $OCH_3$ | 1 | |
| $CH_3$ | 6–$CH_3$ | H | H | O | O | O | $OCH_3$ | 1 | |
| $CH_3$ | H | H | H | $-N(CH_3)-$ | O | O | $OCH_3$ | 2 | |
| $CH_3$ | H | H | H | $-N(CH_3)-$ | O | O | $CH_3$ | 1 | |
| $CH_3$ | H | H | H | O | S | O | $OCH_3$ | 1 | |
| $CH_3$ | H | H | H | O | S | O | $CH_3$ | 1 | |
| $CH_3$ | H | H | $CH_3$ | O | O | O | $CH_3$ | 1 | |
| $CH_3$ | H | H | $CH_3$ | O | O | $CH_2$ | $CH_3$ | 1 | |
| $CF_3$ | H | H | H | O | O | O | $CH_3$ | 1 | |
| $-CH_2CH_2OCH_3$ | H | H | H | O | O | O | $CH_3$ | 1 | |
| $CH_3$ | H | H | $OCH_3$ | O | O | O | $CH_3$ | 1 | |
| $CH_3$ | H | H | H | O | O | $CH_2$ | $OCH_3$ | 2 | |
| $CH_3$ | H | H | H | O | O | O | Cl | 1 | |
| $CH_3$ | H | H | H | O | O | O | Cl | 2 | |

TABLE III

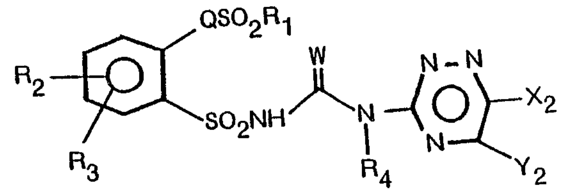

| $R_1$ | $R_2$ | $R_3$ | $R_4$ | Q | W | $X_2$ | $Y_2$ | m.p. (°C) |
|---|---|---|---|---|---|---|---|---|
| $CH_3$ | H | H | H | O | O | $OCH_3$ | $OCH_3$ | |
| $CH_3$ | H | H | H | O | O | $OCH_3$ | $CH_3$ | |
| $CH_3$ | H | H | H | O | S | $OCH_3$ | $OCH_3$ | |
| $CH_3$ | H | H | $CH_3$ | O | O | $OCH_3$ | $OCH_3$ | |
| $CH_3$ | H | H | H | O | O | $CH_3$ | $CH_3$ | |
| $CH_3$ | H | H | $CH_3$ | O | O | $CH_3$ | $CH_3$ | |
| $CH_3$ | H | H | H | $N-CH_3$ | O | $OCH_3$ | $OCH_3$ | |
| $CF_3$ | H | H | H | O | O | $OCH_3$ | $OCH_3$ | |
| $CH_2CH_2OCH_3$ | H | H | H | O | O | $OCH_3$ | $OCH_3$ | |
| 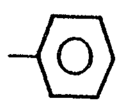 | H | H | H | O | O | $OCH_3$ | $OCH_3$ | |
| $CH_3$ | 5–Cl | H | H | O | O | $OCH_3$ | $OCH_3$ | |
| $CH_3$ | 5–$CH_3$ | H | H | O | O | $OCH_3$ | $OCH_3$ | |
| $CH_3$ | H | H | $OCH_3$ | O | O | $OCH_3$ | $OCH_3$ | |

TABLE IV

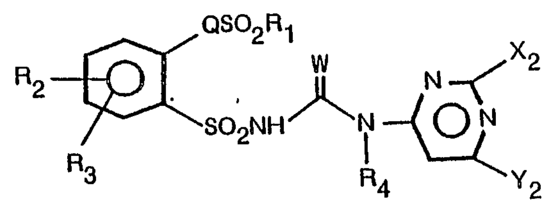

| $R_1$ | $R_2$ | $R_3$ | $R_4$ | Q | W | $X_2$ | $Y_{2'}$ | m.p. (°C) |
|---|---|---|---|---|---|---|---|---|
| $CH_3$ | H | H | H | O | O | $OCH_3$ | $OCH_3$ | |
| $CH_3$ | H | H | H | O | O | $OCH_3$ | $CH_3$ | |
| $CH_3$ | H | H | H | O | S | $OCH_3$ | $OCH_3$ | |
| $CH_3$ | H | H | $CH_3$ | O | O | $OCH_3$ | $OCH_3$ | |
| $CH_3$ | H | H | H | O | O | $CH_3$ | $CH_3$ | |
| $CH_3$ | H | H | $CH_3$ | O | O | $CH_3$ | $CH_3$ | |
| $CH_3$ | H | H | H | N–$CH_3$ | O | $OCH_3$ | $OCH_3$ | |
| $CF_3$ | H | H | H | O | O | $OCH_3$ | $OCH_3$ | |
| $CH_2CH_2OCH_3$ | H | H | H | O | O | $OCH_3$ | $OCH_3$ | |
| 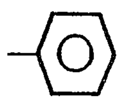 | H | H | H | O | O | $OCH_3$ | $OCH_3$ | |
| $CH_3$ | 5–Cl | H | H | O | O | $OCH_3$ | $OCH_3$ | |
| $CH_3$ | 5–$CH_3$ | H | H | O | O | $OCH_3$ | $OCH_3$ | |
| $CH_3$ | H | H | $OCH_3$ | O | O | $OCH_3$ | $OCH_3$ | |

As indicated above, the compounds of general formula I according to the invention exhibit herbicidal properties.

Thus, in another aspect, the invention provides a composition suitable for controlling the growth of undesired vegetation which comprises at least one compound of formula I according to the invention in association with at least one of the following: surfactant, solid diluent and liquid diluent.

Formulations

Useful formulations of the compounds of Formula I can be prepared in conventional ways. They include dusts, granules, pellets, solutions, suspensions, emulsions wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

TTABLE V

| | Weight Percent* Active Ingredient | Diluent(s) | Surfactant(s) |
|---|---|---|---|
| Wettable Powders | 20—90 | 0—74 | 1—10 |
| Oil Suspensions, Emulsions, Solutions, (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering*, December 4, 1967, pp. 147ff. and "Perry's Chemical Engineer's Handbook", 5th Ed., McGraw-Hill, New York, 1973, pp. 8—56ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

### Example 5

Wettable Powder

| | |
|---|---|
| N-[(4,6-dimethoxypyrimidin-2-yl)aminocarbonyl]-2-hydroxy benzenesulfonamide, methanesulfonate | 80% |
| Sodium alkylnaphthalenesulfonate | 2% |
| sodium ligninsulfonate | 2% |
| synthetic amorphous silica | 3% |
| Kaolinite | 13% |

The ingredients are blended, hammer-milled until all the solids are essentially under 50 microns and then reblended.

### Example 6

Wettable Powder

| | |
|---|---|
| 2-hydroxy-N-[(4-methoxy-6-methylpyrimidin-2-yl)aminocarbonyl]-benzenesulfonamide, methanesulfonate | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

### Example 7

Granule

| | |
|---|---|
| Wettable Powder of Example 6 | 5% |
| attapulgite granules (U.S.S. 20—40 mesh; 0.84—0.42 | 95% |

A slurry of wettable powder containing 25% solids is sprayed on the surface of attapulgite granules in a double-cone blender. The granules are dried and packaged.

### Example 8

Extruded Pellet

| | |
|---|---|
| 2-hydroxy-N-[(4-methoxy-6-methyl-1,3,5-triazin-2-yl)aminocarbonyl]-benzenesulfonamide, methanesulfonate | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to product pellets about 3 mm long. These may be used directly after drying, or the dried pellets may be crushed to pass a U.S.S. No. 20 sieve (0.84 mm

openings). The granules held on a U.S.S. No. 40 sieve (0.42 mm openings) may be packaged for use and the fines recycled.

Example 9

Oil Suspension

| | |
|---|---|
| N-[(4,6-dimethylpyrimidin-2-yl)aminocarbonyl]-2-hydroxybenzene-sulfonamide, methanesulfonate | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 10

Wettable Powder

| | |
|---|---|
| N-[(4,6-dimethoxy-1,3,5-triazin-2-yl)aminocarbonyl]-2-hydroxy-benzenesulfonamide, methanesulfonate | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 11

Low Strength Granule

| | |
|---|---|
| N-[(4,6-dimethoxy-1,3,5-triazin-2-yl)(methyl)aminocarbonyl]-2-hydroxybenzenesulfonamide, methanesulfonate | 1% |
| N,N-dimethylformamide | 9% |
| attapulgite granules (U.S.S. 20—40 sieve | 90% |

The active ingredient is dissolved in the solvent and the solution is sprayed upon dedusted granules in a double cone blender. After spraying of the solution has been completed, the blender is allowed to run for a short period and then the granules are packaged.

Example 12

Aqueous Suspension

| | |
|---|---|
| N-[(4,6-dimethyl-1,3,5-triazin-2-yl)aminocarbonyl]-2-hydroxy-benzenesulfonamide, methanesulfonate | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 13

Solution

| | |
|---|---|
| N-[(4,6-dimethylpyrimidin-2-yl)aminocarbonyl]-2-hydroxybenzene-sulfonamide, methanesulfonate, sodium salt | 5% |
| water | 95% |

The salt is added directly to the water with stirring to produce the solution, which may then be packaged for use.

Example 14

Low Strength Granule

| | |
|---|---|
| 2-hydroxy-N-[(4-methoxy-6-methylpyrimidin-2-yl)aminocarbonyl]-benzenesulfonamide, methanesulfonate | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 15

Granule

| | |
|---|---|
| N-[(4,6-dimethyoxypyrimidin-2-yl)aminocarbonyl]-2-hydroxybenzene-sulfonamide, methanesulfonate | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 16

High Strength Concentrate

| | |
|---|---|
| N-[(4,6-dimethoxy-1,3,5-triazin-2-yl)aminocarbonyl]-2-hydroxy-benzenesulfonamide, methanesulfonate | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 17

Wettable Powder

| | |
|---|---|
| 2-hydroxy-N-[(4-methoxy-6-methyl-1,3,5-triazin-2-yl)aminocarbonyl]-benzenesulfonamide, methanesulfonate | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 18

Wettable Powder

| | |
|---|---|
| N-[(4,6-dimethyl-1,3,5-triazin-2-yl)aminocarbonyl]-2-hydroxybenzene-sulfonamide, methanesulfonate | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 19

Oil Suspension

| | |
|---|---|
| 2-hydroxy-N-[(4-methoxy-6-methyl-1,3,5-triazin-2-yl)-aminocarbonyl]-benzenesulfonamide, 1-propanesulfonate | 35% |
| blend of polyalcohol carboxylic esters and oil soluble petroleum-sulfonates | 6% |
| xylene | 59% |

The ingredients are combined and ground together in a sand mill to produce particles essentially all below 5 microns. The product can be used directly, extended with oils, or emulsified in water.

Example 20

Dust

| | |
|---|---|
| N-[(4,6-dimethoxypyrimidin-2-yl)aminocarbonyl]-2-hydroxybenzene-sulfonamide, 1-propanesulfonate | 10% |
| attapulgite | 10% |
| Pyrophyllite | 80% |

The active ingedient is blended with attapulgite and then passed through a hammer-mill to produce particles substantially all below 200 microns. The ground concentrate is then blended with powdered pyrophyllite until homogeneous.

Utility

The compounds of the present invention are active herbicides. They have utility for broad-spectrum pre- and/or post-emergence weed control in areas where complete control of all vegetation is desired, such as around fuel storage tanks, ammunition depots, industrial storage areas, oil-well sites, drive-in theaters, around billboards, highway and railroad structures. By properly selecting rate, time and method of application, compounds of this invention may also be used to modify plant growth beneficially, and also to selectively control weeds in crops such as wheat, barley, rice, corn and soybeans.

The precise amount of the compounds of Formula I to be used in any given situation will vary according to the particular end result desired, the amount of foliage present, the weeds to be controlled, the crop species involved, the soil type, the formulation and mode of application, weather conditions, etc. Since so many variables play a role, it is not possible to state a rate of application suitable for all situations. Broadly speaking, the compounds of this invention are used at levels of about 0.001 to 20 kg/ha with a preferred range of 0.01 to 10 kg/ha. In general, the higher rates of application from within this range will be selected for adverse conditions or where extended persistence in soil is desired.

The compounds of Formula I may be combined with other herbicides and are particularly useful in combination with the ureas: such as 3-(3,4-dichlorophenyl)-1,1-dimethylurea (diuron); the triazines: such as 2-chloro-4-(ethylamino)-6-(isopropylamino)-*s*-triazine (atrazine); the uracils: such as 5-bromo-3-*sec*-butyl-6-methyluracil (bromacil); N-(phosponomethyl)glycine (glyphosate); 3-cyclohexyl-1-methyl-6-dimethylamino-*s*-triazine-2,4(1H, 3H)-dione (hexazinone); N,N-dimethyl-2,2-diphenylacetamide (diphenamid); 2,3-dichlorophenoxyacetic acid (2,4-D) (and closely related compounds); 4-chloro-2-butynyl-3-chlorophenylcarbamate (barban); S-(2,3-dichloroallyl)-diisopropylthiocarbamate (diallate); S-(2,3,3-trichloroallyl)-diisopropylthiocarbamate (triallate); 1,2-dimethyl-3,5-diphenyl-1H-pyrazolium methyl sulfate (difenzoquat methyl sulfate); methyl 2-[4-(2,4-dichlorophenoxy)phenoxy]propanoate (dichlofop methyl); 4-amino-6-*tert*-butyl-3-(methylthio)-1,2,4-triazin-5(4H)-one (metribuzin); 3-(3,4-dichlorophenyl)-1-methoxy-1-methylurea (linuron); 3-isopropyl-1H-2,1,3-benzothiodiazin-4(3H)-one-2,2-dioxide (bentazon); α,α,α-trifluoro-2,6-dinitro-N,N-dipropyl-*p*-toluidine (trifluralin); 1,1′-dimethyl-4,4′-bipyridinium ion (paraquat); monosodium methanearsonate (MSMA); 2-chloro-2′,6′-diethyl (methoxymethyl)acetanilide (alachlor); 1,1-

dimethyl-3-(α,α,α-trifluoro-*m*-tolyl)urea (fluometuron); and 5-[2-chloro-4-(trifluoromethyl)phenoxy]-2-nitrobenzoic acid, methyl ester (acifluorfen-methyl).

The activity of the compounds of formula I was investigated in a number of greenhouse tests. The tests are described and data resulting from them are shown below.

Test A

Seeds of crabgrass (*Digitaria* spp.), barnyardgrass (*Echinochloa crusgalli*), wild oats (*Avena fatua*), cassia (*Cassia tora*), morningglory (*Ipomoea* sp.), cocklebur (*Xanthium* spp.), sorghum, corn, soybean, rice, wheat and nutsedge tubers (*Cyperus rotundus*) were planted in a growth medium and treated preemergence with the chemicals dissolved in a non-phytotoxic solvent solution of the compounds of Table A. At the same time, cotton having five leaves (including cotyledonary ones), bush beans with the third trifoliate leaf expanding, crabgrass and barnyardgrass with two leaves, wild oats with two leaves, cassia with three leaves (including cotyledonary ones), morningglory and cocklebur with four leaves (including the cotyldonary ones), sorghum and corn with four leaves, soybean with two cotyledonary leaves, rice with three leaves, wheat with one leaf, and nutsedge with three-five leaves were sprayed with a non-phytotoxic solvent solution of the compounds of Table A. Other containers of the above mentioned weeds and crops were treated pre- or post-emergence with the same non-phytotoxic solvent so as to provide a solvent control. A set of untreated control plants was also included for comparison. Pre-emergence and post-emergence treated plants and controls were maintained in a greenhouse for sixteen days, then all treated plants were compared with their respective controls and rated visually for response to treatment.

The following rating system was used:

- 0 = no effect
- 10 = maximum effect
- C = chlorosis or necrosis
- D = defoliation
- E = emergence inhibition
- G = growth retardation
- H = formative effects
- U = unusual pigmentation
- 6Y = abscised buds or flowers
- P = terminal bud kill
- B = burn

COMPOUND 1

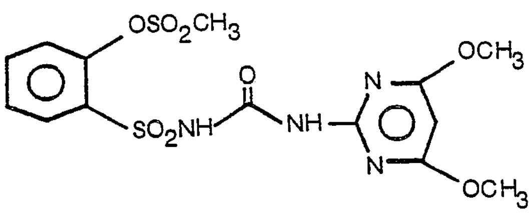

COMPOUND 2

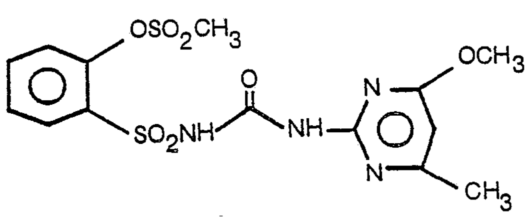

COMPOUND 3

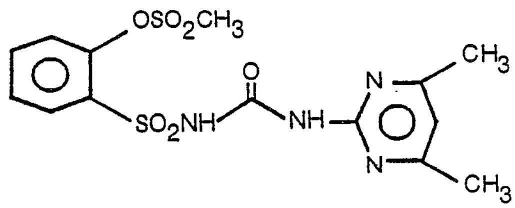

COMPOUND 4

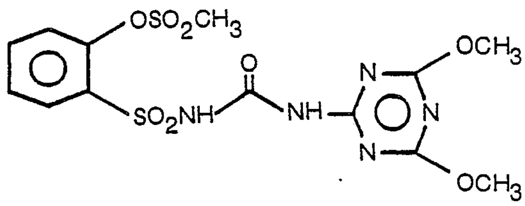

COMPOUND 5

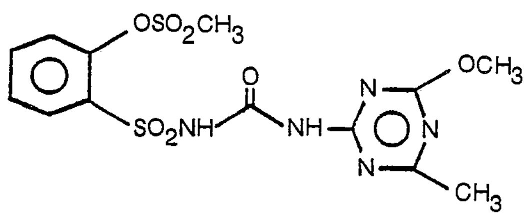

COMPOUND 6

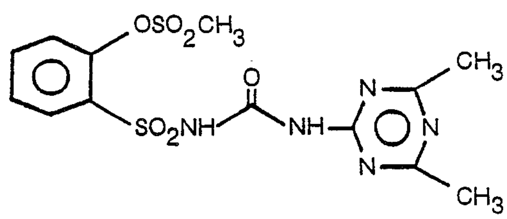

COMPOUND 7

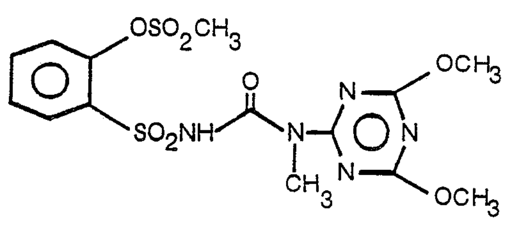

COMPOUND 8

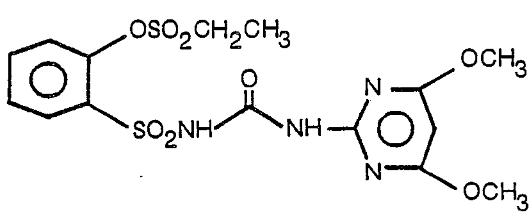

COMPOUND 9

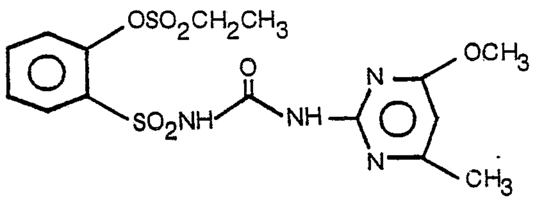

COMPOUND 10

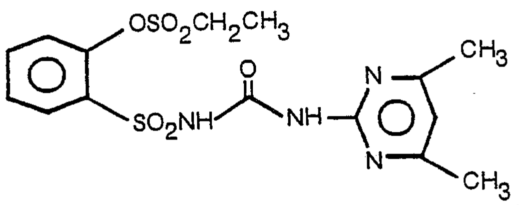

COMPOUND 11

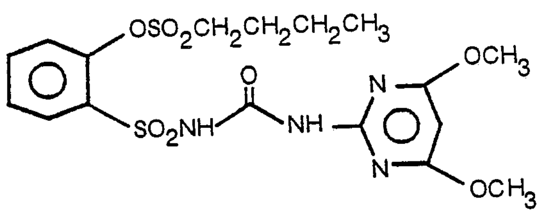

COMPOUND 12

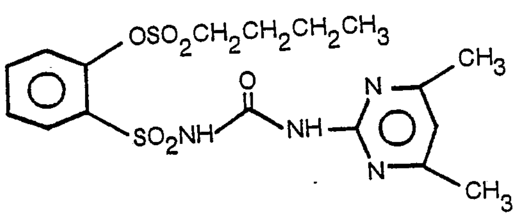

COMPOUND 13

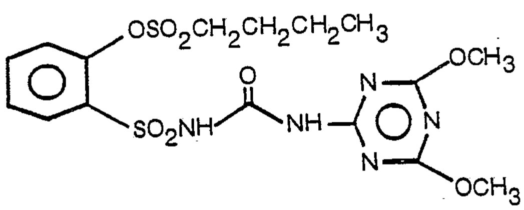

COMPOUND 14

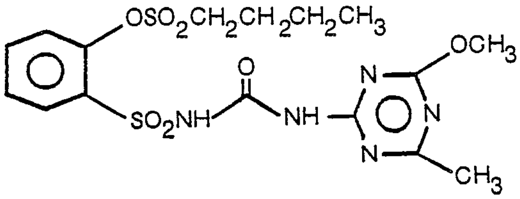

COMPOUND 15

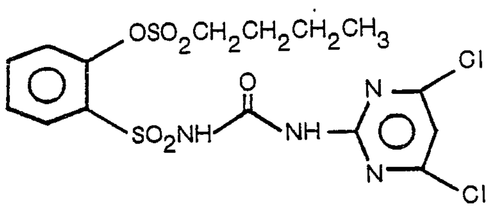

COMPOUND 16

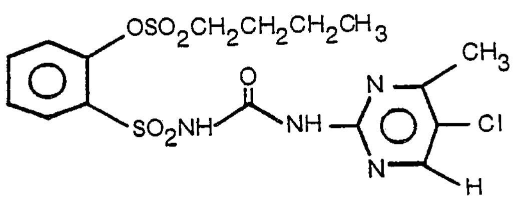

COMPOUND 17

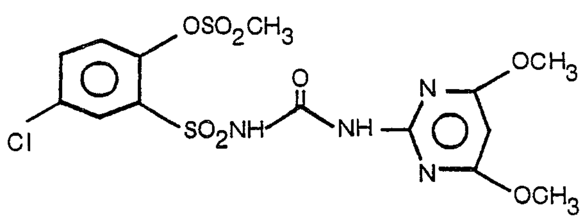

COMPOUND 18

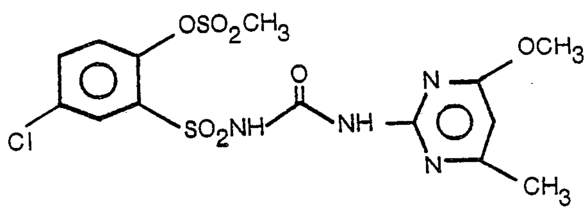

COMPOUND 19

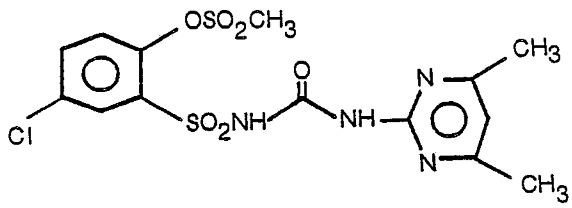

COMPOUND 20

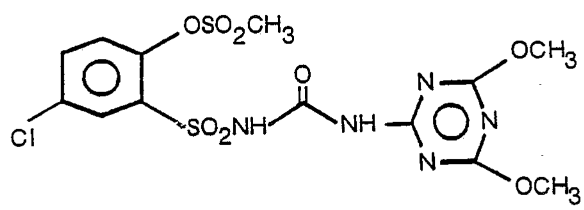

COMPOUND 21

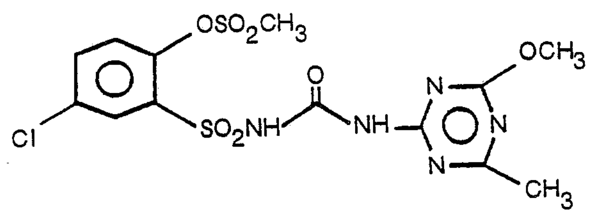

COMPOUND 22

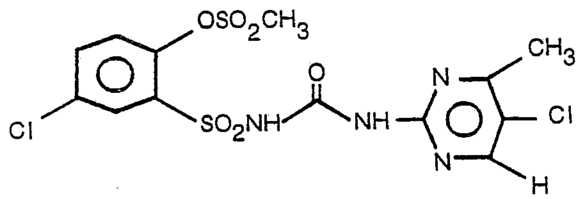

COMPOUND 23

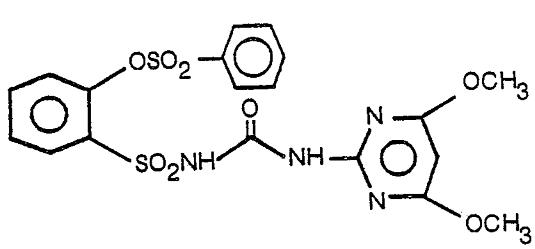

COMPOUND 24

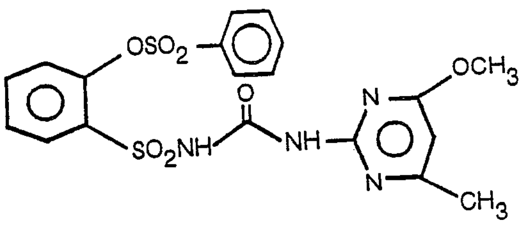

COMPOUND 25

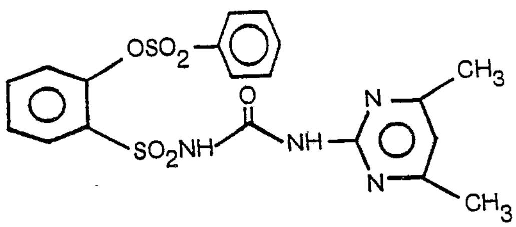

COMPOUND 26

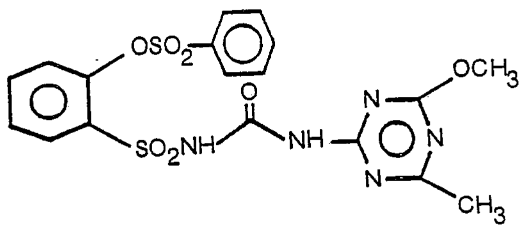

COMPOUND 27

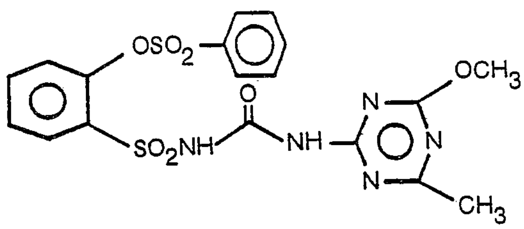

COMPOUND 28

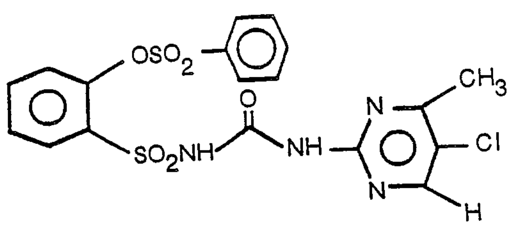

COMPOUND 29

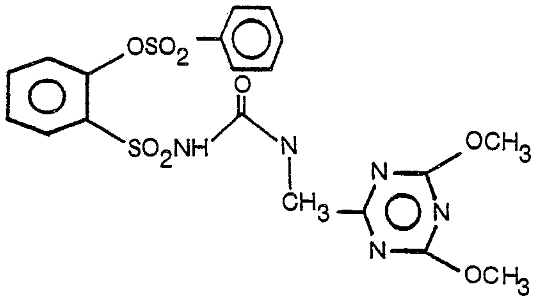

COMPOUND 30

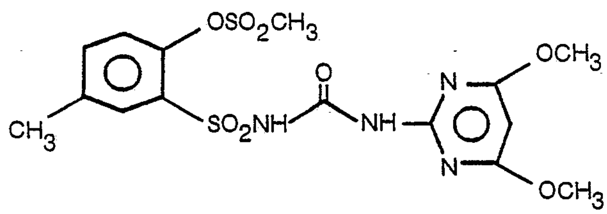

COMPOUND 31

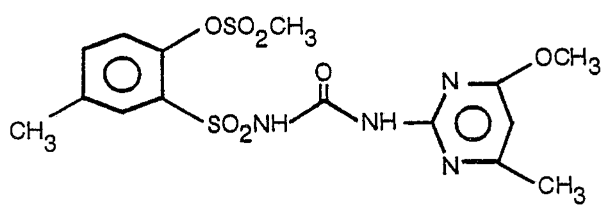

COMPOUND 32

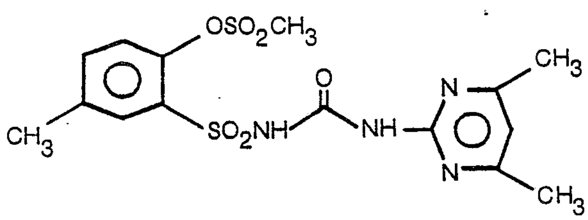

COMPOUND 33

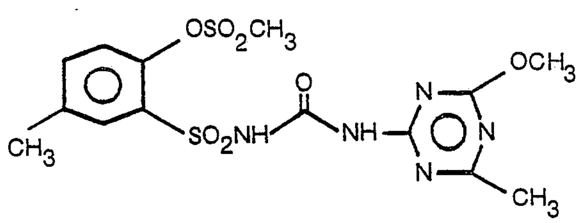

COMPOUND 34

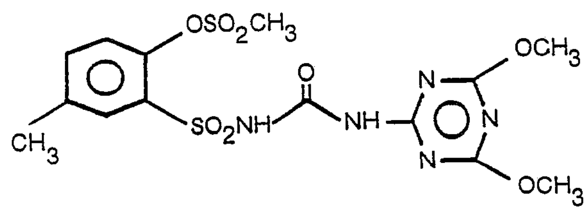

COMPOUND 35

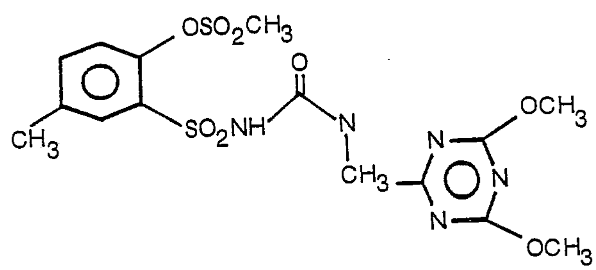

COMPOUND 36

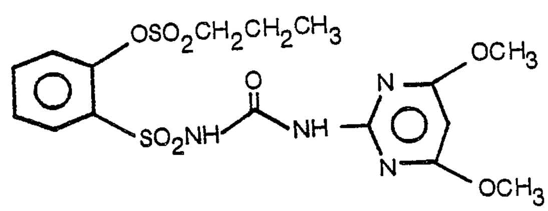

COMPOUND 37

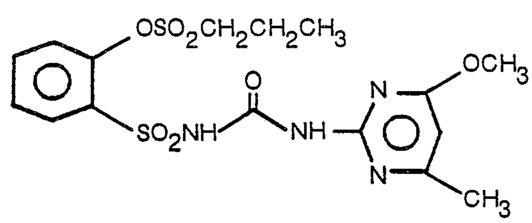

COMPOUND 38

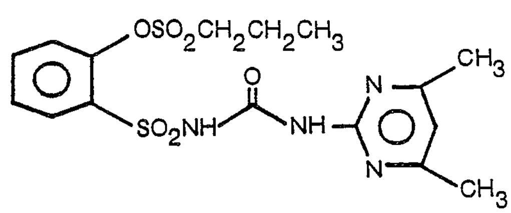

COMPOUND 39

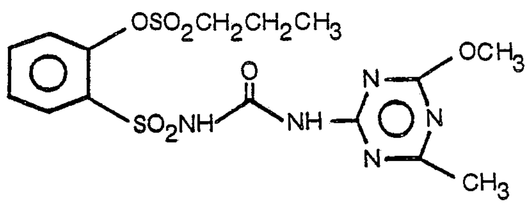

COMPOUND 40

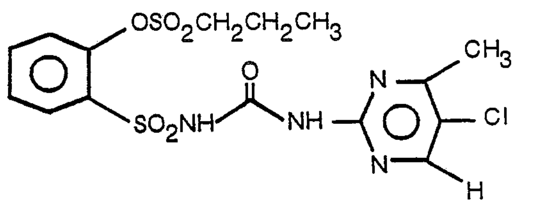

COMPOUND 41

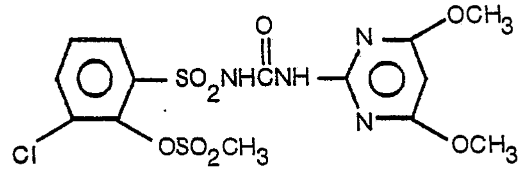

COMPOUND 42

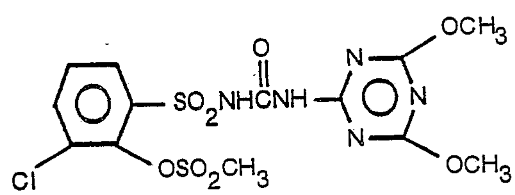

COMPOUND 43

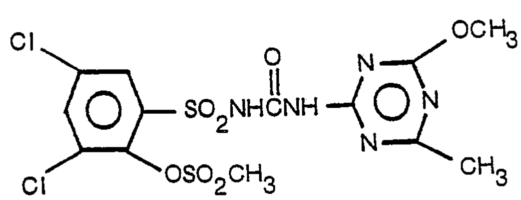

COMPOUND 44

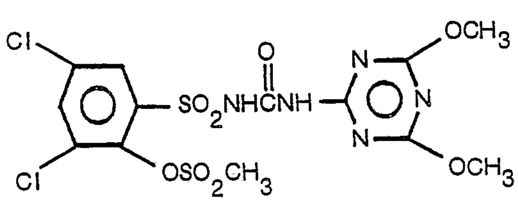

TABLE A

| | Cmpd. 1 | Cmpd. 2 | Cmpd. 3 | Cnpd. 4 |
|---|---|---|---|---|
| Rate kg/ha | 0.1 | 0.1 | 0.1 | 0.1 |
| POST-EMERGENCE | | | | |
| Bush Bean | 9C | 9C | 9C | 9C |
| Cotton | 10C | 9C | 10C | 9C |
| Morningglory | 10C | 10C | 10C | 10C |
| Cocklebur | 10C | 10C | 9C | 10C |
| Cassia | 9C | 9C | 9C | 9C |
| Nutsedge | 9C | 9C | 9C | 1C, 5G |
| Crabgrass | 9C | 9C | 9C | 4C, 9G |
| Barnyardgrass | 10C | 10C | 10C | 4C, 9H |
| Wild Oats | 2C, 9G | 4C, 9G | 5C, 9G | 0 |
| Wheat | 1C,2G | 5C, 9G | 5C, 9G | 0 |
| Corn | 5C, 9G | 9C | 9C | 4U, 9G |
| Soybean | 9C | 9C | 9C | 9C |
| Rice | 9C | 9C | 9C | 9C |
| Sorghum | 9C | 10C | 9C | 2C, 9H |
| PRE-EMERGENCE | | | | |
| Morningglory | 9G | 9G | 9C | 9C |
| Cocklebur | 9H | 9H | 9H | 9H |
| Cassia | 9C | 5C, 9G | 6C, 9G | 6C, 9G |
| Nutsedge | 10E | 10E | 10E | 1C, 7G |
| Crabgrass | 9C | 6C, 9G | 2C, 8G | 2C, 5G |
| Barnyardgrass | 5C, 9H | 9C | 3C, 9H | 7C, 9H |
| Wild Oats | 1U, 9G | 5C, 9H | 4C, 9H | 8G |
| Wheat | 5G | 5C, 9H | 1C, 9H | 3G |
| Corn | 2U, 9G | 10E | 10E | 3C, 9H |
| Soybean | 9H | 9H | 9H | 9H |
| Rice | 10E | 10E | 10E | 10E |
| Sorghum | 7C, 9H | 6C, 9H | 10H | 1C, 9G |

TABLE A (Continued)

| | Cmpd. 5 | Cmpd. 6 | Cmpd. 7 |
|---|---|---|---|
| Rate kg/ha | 0.1 | 0.1 | 0.1 |
| POST-EMERGENCE | | | |
| Bush Bean | 9C | 9C | 1C,2G |
| Cotton | 9C | 9C | 9C |
| Morningglory | 10C | 10C | 9C |
| Cocklebur | 9C | 9C | 9C |
| Cassia | 9C | 6C,9G | 5C,9G |
| Nutsedge | 5G | 1C,5G | 2C,5G |
| Crabgrass | 2C,9G | 9C | 3G |
| Barnyardgrass | 5C,9H | 9C | 0 |
| Wild Oats | 1C,4G | 3C,9G | 0 |
| Wheat | 0 | 1C | 0 |
| Corn | 6C,9G | 6C,9H | 3C,9H |
| Soybean | 9C | 9C | 9C |
| Rice | 9C | 9C | 6G |
| Sorghum | 2C,9H | 6C,9G | 1C,3G |
| PRE-EMERGENCE | | | |
| Morningglory | 9C | 9C | 1C,3G |
| Cocklebur | 9H | 9H | 8H |
| Cassia | 6G,9G | 6C,9G | 2C,2H |
| Nutsedge | 1C,6G | 6G | 0 |
| Crabgrass | 2C,6G | 1C,6G | 0 |
| Barnyardgrass | 2C,9H | 7C,9H | 0 |
| Wild Oats | 1C,7G | 3C,9G | 0 |
| Wheat | 0 | 8G | 0 |
| Corn | 9H | 9H | 3G |
| Soybean | 9H | 9H | 1C,2H |
| Rice | 10E | 10E | 1C |
| Sorghum | 5C,9H | 7C,9H | 0 |

TABLE A (Continued)

| | Cmpd. 8 | Cmpd. 9 | Cmpd. 10 | Cmpd. 11 |
|---|---|---|---|---|
| Rate kg/ha | 0.05 | 0.05 | 0.05 | 0.05 |
| POST-EMERGENCE | | | | |
| Bush Bean | 10G, 9C | 9G, 3H, 6C | 7G, 3H, 7C | 2C, 2H |
| Cotton | 10G, 9C | 10G, 8C | 10G, 8C | 3C, 2H, 6G |
| Sorghum | 10G, 4C | 10C | 10G, 9C | 0 |
| Corn | 7G, 5H | 10G, 8C | 10G, 3H | 0 |
| Soybean | 10G, 7C | 10G, 8C | 7G, 7C | 2H, 6G |
| Wheat | 0 | 3C, 2C | 3G, 2C | 0 |
| Wild Oats | 0 | 9G, 3C | 10G, 6C | 0 |
| Rice | 8G, 3C | 10C | 10C | 1C |
| Barnyardgrass | 10G, 6C | 10C | 10G, 9C | 2H |
| Crabgrass | 0 | 8G, 3C | 0 | 0 |
| Morningglory | 10G, 9C | 7G, 9C | 10G, 5C | 9C |
| Cocklebur | 10G, 9C | 10C | 10G, 7C | 3C, 9G |
| Cassia | 10C | 10G, 8C | 10G, 8C | 0 |
| Nutsedge | 10G, 7C | 10G, 6C | 10G, 7C | 0 |
| PRE-EMERGENCE | | | | |
| Sorghum | 8G, 5G | 9G, 9C | 9G, 9C | 0 |
| Corn | 6G | 9G | 9G, 9C | 0 |
| Soybean | 8G, 5H | 9G, 5H | 8G, 5C | 0 |
| Wheat | 0 | 7G | 7G | 0 |
| Wild Oats | 5G, 3H | 8G, 3C | 7G, 5H | 0 |
| Rice | 8G, 6C | 8G, 8C | 10E | 3G |
| Barnyardgrass | 8G, 5H | 9G, 6C | 8G, 5H | 0 |
| Crabgrass | 3G | 5G, 3H | 3G | 0 |
| Morningglory | 8G, 5C | 8G, 9C | 8G, 9C | 7G |
| Cocklebur | 8G, 3H | 9G, 9P | 9G, 9P | 5H |
| Cassia | 9G, 5C | 9G, 9C | 8C, 9G | 0 |
| Nutsedge | 10E | 10E | 10E | 0 |

TABLE A (Continued)

| | Cmpd. 12 | Cmpd. 13 | Cmpd. 14 |
|---|---|---|---|
| Rate kg/ha | 0.05 | 0.05 | 0.05 |
| POST-EMERGENCE | | | |
| Bush Bean | 1C, 2H | 6C, 9G, 6Y | 6C, 9G, 6Y |
| Cotton | 1C | 3C, 3H, 9G | 3C, 3H, 9G |
| Sorghum | 6H | 0 | 0 |
| Corn | 0 | 0 | 0 |
| Soybean | 1H | 2H, 6G | 2H, 5G |
| Wheat | 0 | 0 | 0 |
| Wild Oats | 0 | 0 | 0 |
| Rice | 0 | 0 | 0 |
| Barnyardgrass | 0 | 0 | 0 |
| Crabgrass | 0 | 0 | 0 |
| Morningglory | 1C, 5G | 4C, 9H | 3C, 9H |
| Cocklebur | 1C | 4C, 9H | 2C, 8G |
| Cassia | 0 | 3C | 1C |
| Nutsedge | 0 | 0 | 0 |
| PRE-EMERGENCE | | | |
| Sorghum | 0 | 0 | 0 |
| Corn | 0 | 0 | 0 |
| Soybean | 0 | 0 | 0 |
| Wheat | 0 | 0 | 0 |
| Wild Oats | 0 | 0 | 0 |
| Rice | 0 | 0 | 0 |
| Barnyardgrass | 0 | 0 | 0 |
| Crabgrass | 0 | 0 | 0 |
| Morningglory | 0 | 3C, 9G | 4C, 9G |
| Cocklebur | 0 | 8H, 3C | 7H, 3C |
| Cassia | 0 | 2C | 0 |
| Nutsedge | 0 | 0 | 0 |

TABLE A (Continued)

| | Cmpd. 17 | Cmpd. 18 | Cmpd. 19 |
|---|---|---|---|
| Rate kg/ha | 0.05 | 0.05 | 0.05 |
| POST-EMERGENCE | | | |
| Bush Bean | 3C, 5G, 6Y | 9C, 7G | 6C, 7G, 6Y |
| Cotton | 7C, 6G | 8C, 7G | 9C, 6G |
| Sorghum | 3C, 4G | 2C, 4G | 2C, 4G |
| Corn | 4C, 3U, 4G | 5C, 6G | 0 |
| Soybean | 6C, 7G | 10C | 7C, 6G |
| Wheat | 0 | 0 | 2C, 2G |
| Wild Oats | 2C | 3C, 4G | 3G |
| Rice | 2C, 3G | 3C, 4G | 3C,5G |
| Barnyardgrass | 10C | 7C, 6G | 2C, 3G |
| Crabgrass | 2C, 3G | 0 | 0 |
| Morningglory | 10C | 5C, 4G | 10C |
| Cocklebur | 10C | 8C, 7G | 10C |
| Cassia | 8G, 7G | 3C, 4G | 3C, 4G |
| Nutsedge | 10C | 6C, 4G | 5C, 4G |
| PRE-EMERGENCE | | | |
| Sorghum | 3C, 6G | 5C, 8G | 7C, 7G |
| Corn | 3C, 6G | 7C, 6G | 0 |
| Soybean | 7C, 8G | 7C, 8G | 7C, 6G |
| Wheat | 0 | 3C, 5G | 0 |
| Wild Oats | 2C | 7C, 6G | 2C, 6G |
| Rice | 10E | 8C, 8G | 8C, 7G |
| Barnyardgrass | 7C, 8G | 7C, 7G | 7C, 5G |
| Crabgrass | 2C, 2G | 3G, 2C | 0 |
| Morningglory | 7C, 7G | 10C | 7C, 6G |
| Cocklebur | 8C, 7G | 3C, 5G | 5C, 6G |
| Cassia | 8C, 8G | 7C, 6G | 5C, 4G |
| Nutsedge | 10E | 10E | 10E |

TABLE A (Continued)

| | Cmpd. 20 | Cmpd. 21 | Cmpd. 30 |
|---|---|---|---|
| Rate kg/ha | 0.05 | 0.05 | 0.05 |
| POST-EMERGENCE | | | |
| Bush Bean | 2G | 9C, 6G, 6Y | 3C, 9G, 6Y |
| Cotton | 5C, 4G | 9C, 5G | 5G, 9G |
| Sorghum | 4C, 5G | 6C, 5G | 10C |
| Corn | 10C | 7C, 6G | 10C |
| Soybean | 4C, 5G | 5C, 6G | 2C, 9G |
| Wheat | 0 | 2C, 3G | 9C |
| Wild Oats | 4C, 5G | 8C, 7G | 2C, 8G |
| Rice | 3C, 4G | 2C,3G | 10C |
| Barnyardgrass | 7C, 6G | 10C | 10C |
| Crabgrass | 2C, 2G | 7C, 6G | 6C, 9G |
| Morningglory | 10C | 10C | 9C |
| Cocklebur | 10C | 10C | 9C |
| Cassia | 2C | 5C, 3G | 9C |
| Nutsedge | 0 | 3C, 4G | 9C |
| PRE-EMERGENCE | | | |
| Sorghum | 7C, 8G | 7C, 8G | 1C, 9H |
| Corn | 7C, 8G | 8C, 8G | 1C, 9G |
| Soybean | 6C, 7G | 7C, 6G | 1C, 9G |
| Wheat | 0 | 0 | 9G |
| Wild Oats | 2C,5G | 5C, 6G | 8G |
| Rice | 10E | 10E | 10E |
| Barnyardgrass | 7C, 7G | 8C, 8G | 5C, 9H |
| Crabgrass | 4C, 5G | 5C, 7G | 2C, 8G |
| Morningglory | 10C | 10C | 9G |
| Cocklebur | 10C | 10C | 9H |
| Cassia | 5G, 6C | 7C, 6G | 2C, 9G |
| Nutsedge | 0 | 7C, 5G | 10E |

TABLE A (Continued)

| | Cmpd. 31 | Cmpd. 32 | Cmpd. 33 | Cmpd. 34 |
|---|---|---|---|---|
| Rate kg/ha | 0.05 | 0.05 | 0.05 | 0.05 |
| POST-EMERGENCE | | | | |
| Bush Bean | 9C | 9D, 9G, 6Y | 9D, 9G, 6Y | 4C, 6G, 6Y |
| Cotton | 6C, 9G | 3H, 6C, 9G | 6C, 9G | 3H, 6C, 9G |
| Sorghum | 2C, 9G | 9C | 3C, 9G | 1C, 6H |
| Corn | 7U, 9G | 1C, 9G | 3U, 9G | 1C, 5G |
| Soybean | 4C, 9G | 5C, 9G | 3C, 9G | 2C, 9G |
| Wheat | 2C, 7G | 9C | 1C, 3G | 0 |
| Wild Oats | 1C, 8G | 9G | 1C, 2G | 0 |
| Rice | 3C, 9G | 9C | 2C, 9G | 8G |
| Barnyardgrass | 9C | 10C | 10C | 3C, 7H |
| Crabgrass | 6G | 2C, 5G | 1C, 3G | 1C, 3G |
| Morningglory | 10C | 10C | 3C, 9G | 9C |
| Cocklebur | 10C | 9C | 6C, 9G | 6C, 9G |
| Cassia | 9C | 9C | 3C, 7G | 3C, 5G |
| Nutsedge | 1C, 9G | 4C, 9G | 2C, 7G | 1C, 3G |
| PRE-EMERGENCE | | | | |
| Sorghum | 4C, 9G | 3C, 9H | 2C, 9G | 2C, 7G |
| Corn | 2C, 9G | 2C, 9H | 1C, 9G | 1C, 8G |
| Soybean | 8H | 3C, 7H | 9H | 1C, 9H |
| Wheat | 9G | 3C | 0 | 0 |
| Wild Oats | 2C, 9H | 1C, 9G | 1C, 5G | 0 |
| Rice | 2C, 9H | 10E | 9H | 2C, 5G |
| Barnyardgrass | 2C, 9H | 2C, 9H | 3C, 8H | 1C, 5H |
| Crabgrass | 2C, 9G | 1H, 3G | 1C, 3G | 0 |
| Morningglory | 9C | 9G | 1C, 9H | 2C, 9G |
| Cocklebur | 9H | 9H | 9H | 9H |
| Cassia | 2C, 9G | 2C, 9G | 9G, 3C | 6C, 9G |
| Nutsedge | 10E | 10E | 1C, 9G | 3G |

TABLE A (Continued)

| | Cmpd. 35 | Cmpd. 36 | Cmpd. 37 | Cmpd. 38 |
|---|---|---|---|---|
| Rate kg/ha | 0.1 | 0.05 | 0.05 | 0.05 |
| POST-EMERGENCE | | | | |
| Bush Bean | 2G | 5C, 9G, 6Y | 9C | 4C, 9G, 6Y |
| Cotton | 3C, 3H, 7G | 9C | 9C | 5C, 9G |
| Sorghum | 1C, 3G | 9C | 3U | 10C |
| Corn | 0 | 1C, 2G | 2C | 2C, 4G |
| Soybean | 2C, 9G | 2C,8G | 2C | 1H, 6G |
| Wheat | 0 | 0 | 0 | 0 |
| Wild Oats | 0 | 2G | 1C | 5G |
| Rice | 0 | 4C, 9G | 2C | 7G |
| Barnyardgrass | 0 | 3C, 9H | 10C | 3C, 7H |
| Crabgrass | 0 | 2C, 4G | 3C | 2C, 5G |
| Morningglory | 6C, 9G | 9C | 9C | 2C, 9G |
| Cocklebur | 4C, 9G | 10C | 10C | 9C |
| Cassia | 2C | 5C, 9G | | 5G |
| Nutsedge | 1C, 2G | 9G | 1C | 8G |
| PRE-EMERGENCE | | | | |
| Sorghum | 0 | 2C, 9H | 2C,9H | 2C, 9H |
| Corn | 0 | 2C, 5G | 2C,8H | 2C,7H |
| Soybean | 0 | 1C, 4H | 2C, 5G | 2G |
| Wheat | 0 | 0 | 3G | 0 |
| Wild Oats | 0 | 1C, 6G | 1C, 8G | 1C, 6G |
| Rice | 0 | 3C, 9H | 2C, 8H | 2C, 6G |
| Barnyardgrass | 0 | 2C, 8H | 2C, 9H | 3C, 5G |
| Crabgrass | 0 | 1C | 1C, 3G | 1C |
| Morningglory | 0 | 9G | 9G | 1C, 6G |
| Cocklebur | 0 | 9H | 9H | 9H |
| Cassia | 0 | 2C, 8G | 2C, 8H | 1C |
| Nutsedge | 0 | 9G | 9G | 5G |

TABLE A (Continued)

| | Cmpd. 39 | Cmpd. 40 |
|---|---|---|
| Rate kg/ha | 0.05 | 0.4 |
| POST-EMERGENCE | | |
| Bush Bean | 9C | 0 |
| Cotton | 9C | 1C |
| Sorghum | 1C | 4G |
| Corn | 1C | 0 |
| Soybean | 1H | 0 |
| Wheat | 0 | 0 |
| Wild Oats | 2G | 0 |
| Rice | 0 | 2G |
| Barnyardgrass | 2G | 0 |
| Crabgrass | 1C | 5G |
| Morningglory | 9C | 3G |
| Cocklebur | 10C | 3G |
| Cassia | | 0 |
| Nutsedge | 2G | 3G |
| PRE-EMERGENCE | | |
| Sorghum | 1C, 7G | 0 |
| Corn | 1C, 3G | 0 |
| Soybean | 1C | 1H |
| Wheat | 0 | 0 |
| Wild Oats | 0 | 0 |
| Rice | 2C | 0 |
| Barnyardgrass | 1C, 4H | 0 |
| Crabgrass | 1C | 2G |
| Morningglory | 9G | 3G |
| Cocklebur | 9H | 5H |
| Cassia | 2C, 9H | 0 |
| Nutsedge | 2G | 0 |

TABLE A (Continued)

| | Cmpd. 26 | Cmpd. 27 |
|---|---|---|
| Rate kg/ha | 0.05 | 0.05 |
| POST-EMERGENCE | | |
| Bush Bean | 3C, 9G, 6Y | 1C, 2G, 6Y |
| Cotton | 2C | 1C |
| Sorghum | 0 | 3G |
| Corn | 2C, 3G | 1C |
| Soybean | 2H | 2H |
| Wheat | 0 | 0 |
| Wild Oats | 0 | 0 |
| Rice | 0 | 4G |
| Barnyardgrass | 0 | 0 |
| Crabgrass | 0 | 1C |
| Morningglory | 2C, 6H | 1C |
| Cocklebur | 2C, 6G | 3C, 6H |
| Cassia | | |
| Nutsedge | 0 | 1C |
| PRE-EMERGENCE | | |
| Sorghum | 0 | 0 |
| Corn | 0 | 0 |
| Soybean | 0 | 0 |
| Wheat | 0 | 0 |
| Wild Oats | 0 | 0 |
| Rice | 0 | 0 |
| Barnyardgrass | 0 | 0 |
| Crabgrass | 0 | 0 |
| Morningglory | 0 | 0 |
| Cocklebur | 0 | 9H |
| Cassia | 0 | 0 |
| Nutsedge | 0 | 0 |

TABLE A (Continued)

| | Cmpd. 41 | Cmpd. 42 |
|---|---|---|
| Rate kg/ha | 0.4 | 0.4 |
| POST-EMERGENCE | | |
| Bush Bean | 9C | 9C |
| Cotton | 10C | 4C, 3H, 9G |
| Sorghum | 6U, 9G | 2C, 9G |
| Corn | 2U, 9H | 5U, 9G |
| Soybean | 9C | 9C |
| Wheat | 2G | 0 |
| Wild Oats | 1C,8G | 0 |
| Rice | 6C, 9G | 6C, 9G |
| Barnyardgrass | 5C,9H | 1C, 6G |
| Crabgrass | 2C, 6G | 4G |
| Morningglory | 6C, 9G | 6C, 9G |
| Cocklebur | 10C | 9C |
| Cassia | 9C | 4C, 7H |
| Nutsedge | 2C, 8G | 2C, 5G |
| PRE-EMERGENCE | | |
| Sorghum | 5C, 9H | 5C, 9G |
| Corn | 2U, 9G | 2C, 9G |
| Soybean | 9H | 4C, 8H |
| Wheat | 8G | 2C, 8G |
| Wild Oats | 4C, 8G | 2C, 8G |
| Rice | 10E | 10E |
| Barnyardgrass | 4C, 9H | 4C, 9G |
| Crabgrass | 5G | 1C, 4G |
| Morningglory | 9C | 9C |
| Cocklebur | 9H | 9H |
| Cassia | 4C, 9G | 3C, 7G |
| Nutsedge | 1C, 9G | 8G |

TABLE A (Continued)

| | Compound 43 | | Compound 44 | |
|---|---|---|---|---|
| Rate kg/ha | 2 | 6 | 2 | 6 |
| POST-EMERGENCE | | | | |
| Bush Bean | 0 | 3C | 0 | 0 |
| Cotton | 0 | 3C | 0 | 2C |
| Sorghum | 0 | 0 | 0 | 2C |
| Corn | 0 | 0 | 0 | 0 |
| Soybean | 0 | 1C | 0 | 3G |
| Wheat | 0 | 0 | 0 | 0 |
| Wild Oats | 0 | 0 | 0 | 0 |
| Rice | 0 | 0 | 0 | 0 |
| Barnyardgrass | 0 | 1C | 0 | 0 |
| Crabgrass | 0 | 0 | 0 | 0 |
| Morningglory | 0 | 0 | 0 | 1C, 3G |
| Cocklebur | 0 | 0 | 0 | 0 |
| Cassia | 0 | 1C | 0 | 0 |
| Nutsedge | 0 | 0 | 0 | 0 |
| PRE-EMERGENCE | | | | |
| Sorghum | 0 | 1C, 5G | 0 | 2C |
| Corn | 0 | 2C, 5G | 0 | 1C, 2G |
| Soybean | 0 | 0 | 0 | 0 |
| Wheat | 0 | 0 | 0 | 0 |
| Wild Oats | 0 | 1C, 4G | 0 | 0 |
| Rice | 0 | 1C | 0 | 1C |
| Barnyardgrass | 0 | 5C, 8H | 0 | 5C |
| Crabgrass | 0 | 2G | 0 | 0 |
| Morningglory | 0 | 3C | 0 | 2C |
| Cocklebur | 0 | 2C, 8H | 0 | 5H |
| Cassia | 0 | 3G | 0 | 5G |
| Nutsedge | 0 | 0 | 0 | 0 |

Test B

Two plastic bulb pans were filled with fertilized and limed Fallsington silt loam soil. One pan was planted with corn, sorghum, Kentucky bluegrass and several grassy weeds. The other pan was planted with cotton, soybeans, purple nutsedge (*Cyperus rotundus*), and several broadleaf weeds. The following grassy and broadleaf weeds were planted: crabgrass (*Digitaria sanguinalis*), barnyardgrass (*Echinochloa crusgalli*), wild oats (*Avena fatua*), johnsongrass (*Sorghum halepense*), dallisgrass (*Paspalum dilatatum*), giant foxtail (*Setaria faberii*), cheatgrass (*Bromus secalinus*), mustard (*Brassica arvensis*), cocklebur (*Xanthium pensylvanicum*), pigweed (*Amaranthus retroflexus*), morningglory (*Ipomoea hederacea*), cassia (*Cassia tora*), teaweed (*Sida spinosa*), velvetleaf (*Abutilon theophrasti*), and jimsonweed (*Datura stramonium*). A 12.5 cm diameter plastic pot was also filed with prepared soil and planted with rice and wheat. Another 12.5 cm pot was planted with sugarbeets. The above four containers were treated pre-emergence with several test compounds within the scope of the invention.

Twenty-eight days after treatment, the plants were evaluated and visually rated for response to the chemical treatments utilizing the rating system described previously for Test A. The data are summarized in Table B.

TABLE B

PRE-EMERGENCE ON FALLSINGTON SILT LOAM

| | Compound 1 | | Compound 2 | |
|---|---|---|---|---|
| Rate kg/ha | 0.03 | 0.12 | 0.03 | 0.12 |
| Crabgrass | 7G, 2H | 8G, 3H | 7G, 3H | 8G, 3H |
| Barnyardgrass | 8G, 3H | 9G, 9C | 9G, 8C | 10C |
| Sorghum | 9G, 9C | 10C | 10C | 10E |
| Wild Oats | 6G | 10C | 8G, 5C | 9G, 9C |
| Johnsongrass | 8G, 7C | 10C | 9G, 9C | 10C |
| Dallisgrass | 7G | 9G, 9C | 8G, 3H | 8G, 3H |
| Giant Foxtail | 6G, 3C | 8G, 5C | 5G, 3C | 10C |
| Ky. bluegrass | 8G, 9C | 10C | 8G, 9C | 10C |
| Cheatgrass | 8G, 9C | 9G, 9C | 10E | 10E |
| Sugarbeets | 10C | 10C | 10C | 10C |
| Corn | 5G, 3U | 8G, 5H | 7G, 5H | 10C |
| Mustard | 10C | 10C | 10C | 10C |
| Cocklebur | 6G | — | 5G | 10C |
| Pigweed | 10C | 10E | 7G | 10E |
| Nutsedge | 10E | 10E | 6G | 10E |
| Cotton | 8G | 9G, 5C | 6G, 3H | 9G, 8C |
| Morningglory | 5G | 9G, 9C | 7G | 9G, 5C |
| Cassia | 8G, 8C | 10C | 7G, 4C | 9G, 9C |
| Teaweed | 6G | 9G, 8C | 6G, 3H | 8G, 6C |
| Velvetleaf | 9G, 8C | 10C | 8G, 8C | 9G, 9C |
| Jimsonweed | 7G, 3C | 9G, 8C | 9G, 9C | 9G, 9C |
| Soybean | 8G, 5H | 9G, 8C | 7G, 5H | 9G, 8C |
| Rice | 8G, 3H | 10E | 8G,7C | 10E |
| Wheat | 0 | 3G | 5G | 8G, 9C |

TABLE B (Continued)

PRE-EMERGENCE ON FALLSINGTON SILT LOAM

| | Compound 3 | | | |
|---|---|---|---|---|
| Rate kg/ha | 0.007 | 0.015 | 0.03 | 0.12 |
| Crabgrass | 0 | 0 | 5G | 6G |
| Barnyardgrass | 2G | 2G | 8G, 3H | 9G, 9C |
| Sorghum | 2G | 4G | 10C | 10E |
| Wild Oats | 0 | 0 | 10C | 10C |
| Johnsongrass | 0 | 3G | 10C | 10C |
| Dallisgrass | 0 | 3G | 6G | 8G, 3H |
| Giant Foxtail | 0 | 3G | 3C | 7G, 3C |
| Ky. bluegrass | 0 | 2G | 10C | 8G, 8C |
| Cheatgrass | 0 | – | 10E | 10E |
| Sugarbeets | – | 4G | 10C | 10C |
| Corn | 0 | 0 | 10C | 10C |
| Mustard | 5G | 7G, 3C | 10C | 10C |
| Cocklebur | 0 | 0 | 7G | 8G, 5H |
| Pigweed | 0 | 10E | 9G, 9C | 10C |
| Nutsedge | 0 | 0 | 10E | 10E |
| Cotton | 0 | 0 | 8G, 3C | 9G, 7C |
| Morningglory | 3G | 3G | 8G | 9G, 7C |
| Cassia | 0 | 0 | 9G, 8C | 9G, 8C |
| Teaweed | – | 0 | 4G | 10C |
| Velvetleaf | 0 | 0 | 8G, 5E | 9G, 8C |
| Jimsonweed | 0 | 0 | 8G, 8C | 9G, 9C |
| Soybean | 0 | 0 | 8G, 5H | 9G, 8C |
| Rice | 3G | 6G, 3H | 10E | 10E |
| Wheat | 0 | 0 | 5G | 7G, 3C |

TABLE B (Continued)

PRE-EMERGENCE ON FALLSINGTON SILT LOAM

| | Compound 36 | | Compound 39 | |
|---|---|---|---|---|
| Rate kg/ha | 0.06 | 0.25 | 0.06 | 0.25 |
| Crabgrass | 2G | 4G | 2G | 2G |
| Barnyardgrass | 4G | 6G, 5H | 4G | 5G |
| Sorghum | 6G, 3H | 8G, 5H | 4G | 4G, 2H |
| Wild Oats | 4G | 4G | 3G | 3G |
| Johnsongrass | 4G, 3H | 6G, 3H | 4G | 3G |
| Dallisgrass | 4G | 5G | 3G | 0 |
| Giant Foxtail | 4G | 6G, 3H | 0 | 2G |
| Ky. bluegrass | 6G | 7G | 2G | 4G |
| Cheatgrass | 6G | 7G | 0 | 3G |
| Sugarbeets | 6G, 6C | 8G, 9C | 9G, 8C | 10C |
| Corn | 3G | 4G | 2G | 4G |
| Mustard | 10E | 10E | 8G, 8C | 8G, 9C |
| Cocklebur | – | 8G, 7C | 8G, 5H | 10C |
| Pigweed | 5G, 5C | 9G, 9C | 4G | 6G, 4C |
| Nutsedge | 7G | 10E | 4G | 6G |
| Cotton | 4G | 8G, 3H | 8G, 3H | 9G, 5C |
| Morningglory | 4G | 8G, 9C | 9G | 9G, 3C |
| Cassia | 6G | 8G, 8C | 5G, 3C | 7G, 8C |
| Teaweed | 5G, 3C | 6G, 3C | 6G, 2C | 6G, 3C |
| Velvetleaf | 5G, 3H | 8G, 8C | 7G, 3H | 8G, 9C |
| Jimsonweed | 3G | 5G, 5C | 7G, 3C | 8G, 5C |
| Soybean | 2G | 5G, 3H | 3G | 4G |
| Rice | 3G | 5G | 0 | 0 |
| Wheat | 0 | 0 | 0 | 0 |

Test C

The high herbicidal activity of two of the compounds from within the scope of the invention is evident from the results obtained in this test. The experiment concerned pre-emergence applications on soil. The containers used were 25 cm diameter plastic pots filled with Fallsington silt loam. One set of pots was planted to weeds, the seeds of which were uniformly mixed with the top 1.2 cm layer of soil. The species selected were: johnsongrass (*Sorghum halepense*), barnyardgrass (*Echinochloa crusgalli*), crabgrass (*Digitaria sanguinalis*), giant foxtail (*Setaria faberii*), velvetleaf (*Abutilon theophrasti*), jimsonweed (*Datura stramonium*), mustard (*Brassica arvensis*), and pigweed (*Amaranthus retroflexus*). Another set of pots were planted to the following crops with from one to four species per pot: corn (planting depth 3.7 cm), cotton, soybeans, sunflower, Clinton oats, wheat, Black Valentine beans, rice, sorghum, peas, flax, and peanuts (all at 2.5 cm depth), cucumbers, cabbage, alfalfa, safflower, sugarbeets, tomato, spinach, barley, and Kentucky bluegrass (all at 1.2 cm depth). Immediately after planting, the test chemical was applied to the soil surfaces dissolved in a non-phytotoxic solvent. One pot from the weed phase and one of each of the crop species were left untreated for the purpose of comparison. The treated and untreated pots were promptly watered with approximatley 4 mm of simultated rainfall and then held in a greenhouse for several weeks. Visual weed and crop response ratings were made 28 days after treatment utilizing the rating system as described above for Test A. The data are given in Table C.

TABLE C

PRE-EMERGENCE ON FALLSINGTON SILT LOAM SOIL

| | Compound 1 | | | |
|---|---|---|---|---|
| Rate kg/ha | 0.0035 | 0.0075 | 0.0150 | 0.03 |
| Corn | | | | 5G, 3C |
| Cotton | | | | 7G, 3H |
| Soybean | | | | 4G, 2IH |
| Peanut | | | | 6G |
| Sunflower | | | | 5G |
| Oats | | | | 4G |
| Wheat | | | | 2G |
| Sorghum | | | | 7G, 5H |
| Sugarbeet | | | | 9G, 9C |
| Pea | | | | 10E |
| Flax | | | | 6G |
| Alfalfa | | | | 8G, 6C |
| Bean | | | | 0 |
| Spinach | | | | 8G, 5H |
| Cabbage | | | | 8G, 6C |
| Tomato | | | | 4G |
| Rice | | | | 6G |
| Safflower | | | | 7G, 4C |
| Cucumber | | | | 5G |
| Ky. bluegrass | | | | 7G |
| Barley | | | | 3G |
| Broadleaves | 3G | 4G | 6G, 2H | – |
| Grasses | 0 | 0 | 2G | – |

TABLE C (Continued)

PRE-EMERGENCE ON FALLSINGTON SILT LOAM SOIL

| | Compound 2 | | | |
|---|---|---|---|---|
| Rate kg/ha | 0.0075 | 0.015 | 0.030 | 0.06 |
| Corn | | | | 9G, 9C |
| Cotton | | | | 10E |
| Soybean | | | | 9G, 5H |
| Peanut | | | | 8G, 3H |
| Sunflower | | | | 9G, 5H |
| Oats | | | | 6G, 2C |
| Wheat | | | | 5G |
| Sorghum | | | | 10E |
| Sugarbeet | | | | 10E |
| Pea | | | | 10E |
| Flax | | | | 10E |
| Alfalfa | | | | 8G, 6C |
| Bean | | | | 8G, 5H |
| Spinach | | | | 9G, 9C |
| Cabbage | | | | 8G, 7C |
| Tomato | | | | 5C, 8G |
| Rice | | | | 10E |
| Safflower | | | | 8G, 7C |
| Cucumber | | | | 9G, 5H |
| Ky. bluegrass | | | | 6G, 5H |
| Barley | | | | 8G, 7C |
| Broadleaves | 5G, 3C | 7G, 5C | 9G, 8C | – |
| Grasses | 6G, 3C | 8G, 4C | 9G, 6C | – |

Test D

Two ten-inch in diameter plastic pans lined with polyethylene liners were filled with prepared Fallsington slit loam soil. One pan was planted with seeds of wheat (*Triticum aestivum*), barley (*Hordeum vulgare), wild oats (Avena fatua*), downy brome (*Bromus tectorum*), cheatgrass (*Bromus secalinus*), blackgrass (*Alopecurus myosuroides*), annual bluegrass (*Poa annua), green foxtail (Setaria viridis*), quack grass (*Agropyron repens*), Italian ryegrass (*Lolium multiflorum*) and ripgut brome (*Bromus rigidus*). The other pan was planted with seeds of Russian thistle (*Salsola kali*), tansy mustard (*Descuraina pinnata*), smartweed (*Polygonum pensylvanicum*), tumble mustard (*Sisymbrium altissium*) kochia (*Kochia scoparia*), shepherd's purse (*Capsella bursa-pastoris*), *Matricaria inodora*, black nightshade (*Solanum nigrum*), yellow rocket (*Barbarea vulgaris*), wild mustard (*Brassica kaber*) and wild buckwheat (*Polygonum convolvulus*). The above two pans were treated pre-emergence. At the same time two pans in which the above plant species were growing were treated post-emergence. Plant height at the time of treatment ranged from 1—15 cm depending on plant species.

The compounds applied were diluted with a non-phytotoxic solvent and sprayed over-the-top of the pans. An untreated control and a solvent alone control were included for comparison. All treatments were maintained in the greenhouse for 20 days at which time the treatments were compared to the controls and the effects visually rated. The recorded data, presented in Table D, indicate that certain compounds from within the scope of the invention have utility for pre- and/or post-emergence weed control in cereal crops such as wheat and barley.

TABLE D

| | Compound 1 | | | |
|---|---|---|---|---|
| Rate kg/ha | 0.002 | 0.008 | 0.008 | 0.015 |
| PRE-EMERGENCE | | | | |
| Wheat | 0 | 0 | 0 | 0 |
| Barley | 0 | 0 | 0 | 1C,6G |
| Wild Oats | 0 | 0 | 0 | 3C,7G |
| Downy Brome | 3G | 2C,5G | 1G | 5C,7G |
| Cheatgrass | 5G | 4C,7G | 3G | 10C |
| Blackgrass | 3G | 5C,6G | 2G | 10C |
| Annual Bluegrass | 5G | 2C,6G | 1C,2G | 7C,8G |
| Green Foxtail | 0 | 2G | 3C,4G | 3C,5G |
| Quackgrass | 3G | 3C,5G | 0 | 8G |
| Italian Ryegrass | 0 | 3C,5G | 0 | 4G |
| Ripgut Brome | 2G | 2C,4G | 0 | 2C,5G |
| Russian Thistle | 0 | 0 | 1G | 8C,7G |
| Tansy Mustard | 6G | 8C,8G | 8C,8G | 10C |
| Smartweed | – | – | – | – |
| Tumble Mustard | 3C,8G | 10C | 7C,7G | 10C |
| Kochia | 1G | 5G | 3G | 9C,8G |
| Shepherd's Purse | 3C,5G | 8C,8G | 9C,8G | 10C |
| *Matricaria inodora* | 4G | 7C,8G | 7C,7G | 10C |
| Black Nightshade | 2C,4G | 4C,6G | 6C,7G | 8G |
| Yellow Rocket | 2C,7G | 4C,8G | 7C,7G | 10C |
| Wild Mustard | 5C,6G | 8C,8G | 7C,7G | 10C |
| Wild Buckwheat | 3G | 2C,4G | 1C,2G | 3C,6G |

TABLE D (Continued)

| | Compound 1 | | | |
|---|---|---|---|---|
| Rate kg/ha | 0.002 | 0.008 | 0.008 | 0.015 |
| POST-EMERGENCE | | | | |
| Wheat | 0 | 0 | 0 | 0 |
| Barley | 0 | 0 | 0 | 3C, 5G |
| Wild Oats | 0 | 1C | 0 | 3C, 2G |
| Downy Brome | 3G | 5C, 7G | 4C, 5G | 3G |
| Cheatgrass | 4C, 7G | 8C, 7G | 8C, 7G | 10C |
| Blackgrass | 6G | 4C, 5G | 7C, 7G | 10C |
| Annual Bluegrass | 3G | 3C, 6G | 3C, 5G | 5C, 4G |
| Green Foxtail | 4G | 7C, 7G | 7C, 7G | 3C, 4G |
| Quackgrass | 3G | 6G | 2C, 3G | 6C, 5G |
| Italian Ryegrass | 2G | 2G | 0 | 3G |
| Ripgut Brome | 2G | 5G | 0 | 2G |
| Russian Thistle | 2C,3G | 10C | 9C, 7G | 10C |
| Tansy Mustard | 10C | 10C | 10C | 10C |
| Smartweed | – | – | – | – |
| Tumble Mustard | 4C, 6G | 10C | 10C | 10C |
| Kochia | 3G | 4G | 10C | 8C, 7G |
| Shepherd's Purse | 10C | 10C | 10C | 10C |
| *Matricaria inodora* | 7C, 7G | 10C | 9C,7G | 10C |
| Black Nightshade | 4G | 4C, 6G | 1C,1G | 0 |
| Yellow Rocket | 3G | 10C | 10C | 10C |
| Wild Mustard | 10C | 10C | 10C | 10C |
| Wild Buckwheat | 2C, 4G | 5C, 5G | 6C, 5G | 10C |

TABLE D (Continued)

| | Compound 1 | | | |
|---|---|---|---|---|
| | Pre-Emergence | | Post-Emergence | |
| Rate kg/ha | 0.03 | 0.06 | 0.03 | 0.06 |
| Wheat | 0 | 2C, 3G | 0 | 1C, 2G |
| Barley | 1C, 1G | 6C, 8G | 2C, 2G | 7C, 6G |
| Wild Oats | 0 | 8C, 8G | 2G | 3C, 3G |
| Downy Brome | 2C, 7G | 10C | 7C, 7G | 3C, 4G |
| Cheatgrass | 5C, 8G | 10C | 10C | 10C |
| Blackgrass | 3C, 7G | 10C | 10C | 10C |
| Annual Bluegrass | 5C, 7G | 10C | 6C, 7G | 10C |
| Green Foxtail | 7C, 7G | 9C, 8G | 7C, 8G | 7C, 6G |
| Quackgrass | 2C, 3G | 10C | 3C, 6G | 7C, 6G |
| Italian Ryegrass | 1C, 3G | 4C, 7G | 2C, 4G | 4C, 5G |
| Ripgut Brome | 0 | 5C, 7G | 2C, 3G | 3C, 5G |
| Russian Thistle | 2C, 3G | 8C, 8G | 10C | 10C |
| Tansy Mustard | 10C | 10C | 10C | 10C |
| Smartweed | – | – | – | – |
| Tumble Mustard | 10C | 10C | 10C | 10C |
| Kochia | 7C, 8G | 10C | 10C | 10C |
| Shepherd's Purse | 10C | 10C | 10C | 10C |
| *Matricaria inodora* | 7C, 8G | 10C | 9C, 8G | 10C |
| Black Nightshade | 7C, 7G | 3C, 8G | 3C, 7G | 4C, 5G |
| Yellow Rocket | 8C, 9G | 10C | 10C | 10C |
| Wild Mustard | 8C, 8G | 10C | 10C | 10C |
| Wild Buckwheat | 3C, 4G | 4C, 8G | 7C, 6G | 10C |

TABLE D (Continued)

| | Compound 2 | | | |
|---|---|---|---|---|
| Rate kg/ha | 0.002 | 0.008 | 0.008 | 0.015 |
| PRE-EMERGENCE | | | | |
| Wheat | 0 | 0 | 0 | 2C,2G |
| Barley | 0 | 3G | 3C,4G | 5C,7G |
| Wild Oats | 0 | 2C,5G | 2G | 8C,8G |
| Downy Brome | 6G | 7C,7G | 3C,7G | 10C |
| Cheatgrass | 5C,7G | 10C | 5C,8G | 10C |
| Blackgrass | 3C,6G | 7C,7G | 3C, 7G | 10C |
| Annual Bluegrass | 1C,6G | 7C,7G | 2C,5G | 10C |
| Green Foxtail | 0 | 4C,5G | 2C,4G | 6C,7G |
| Quackgrass | 4G | 3C,6G | 4G | 8C,9G |
| Italian Ryegrass | 3G | 5C,7G | 5C,7G | 9C,9G |
| Ripgut Brome | 5G | 6G | 3C,4G | 9C,9G |
| Russian Thistle | 0 | 1C,1G | 3C,4G | 9C,9G |
| Tansy Mustard | 3C,8G | 8C,9G | 7C,8G | 10C |
| Smartweed | – | – | – | – |
| Tumble Mustard | 7C,8G | 10C | 10C | 10C |
| Kochia | 2G | 5G | 7C,8G | 8C,9G |
| Shepherd's Purse | 8C,8G | 8C,8G | 10C | 10C |
| *Matricaria inodora* | 5C,8G | 7C,8G | 7C,7G | 10C |
| Black Nightshade | 3C,4G | 3C,4G | 5C,6G | 2C,7G |
| Yellow Rocket | 8G | 2C,9G | 7C,7G | 10C |
| Wild Mustard | 7C,8G | 8C,8G | 7C,8G | 10C |
| Wild Buckwheat | 2C,1G | 3C,4G | 3C,2G | 3C,4G |

TABLE D (Continued)

| | Compound 2 | | | |
|---|---|---|---|---|
| Rate kg/ha | 0.002 | 0.008 | 0.008 | 0.015 |
| POST-EMERGENCE | | | | |
| Wheat | 0 | 1G | 1G | 5C, 5G |
| Barley | 0 | 3G | 2C, 3G | 7C, 6G |
| Wild Oats | 0 | 2C, 5G | 5G | 7C, 7G |
| Downy Brome | 3C, 5G | 7C, 7G | 7C, 7G | 7C, 7G |
| Cheatgrass | 6C, 7G | 10C | 10C | 10C |
| Blackgrass | 2C, 6G | 7C, 7G | 7C, 7G | 10C |
| Annual Bluegrass | 5C, 6G | 10C | 6C, 5G | 10C |
| Green Foxtail | 3C, 5G | 4C, 5G | 7C, 8G | 6C, 6G |
| Quackgrass | 1C, 6G | 4C, 6G | 6G | 7C, 6G |
| Italian Ryegrass | 1C, 5G | 3C, 6G | 3C, 6G | 10C |
| Ripgut Brome | 4G | 1C, 7G | 3G | 4C, 6G |
| Russian Thistle | 5C, 4G | 8C, 7G | 10C | 10C |
| Tansy Mustard | 10C | 10C | 10C | 10C |
| Smartweed | – | – | – | – |
| Tumble Mustard | 7C, 8G | 8C, 8G | 10C | 10C |
| Kochia | 5G | 10C | 10C | 7C, 6G |
| Shepherd's Purse | 10C | 10C | 10C | 10C |
| *Matricaria inodora* | 10C | 10C | 7C, 7G | 10C |
| Black Nightshade | 2G | 3C, 5G | 1C, 1G | 0 |
| Yellow Rocket | 10C | 10C | 8C, 8G | 10C |
| Wild Mustard | 10C | 10C | 10C | 10C |
| Wild Buckwheat | 2C, 2G | 3C, 5G | 6C, 6G | 10C |

TABLE D (Continued)

| | Compound 2 | | | |
|---|---|---|---|---|
| | Pre-Emergence | | Post-Emergence | |
| Rate kg/ha | 0.03 | 0.06 | 0.03 | 0.06 |
| Wheat | 2C,4G | 4C,6G | 1C,2G | 7C,7G |
| Barley | 6C,7G | 8C,8G | 4C,6G | 10C |
| Wild Oats | 7C,6G | 9C,8G | 5C,6G | 8C,7G |
| Downy Brome | 7C,8G | 10C | 8C,7G | 10C |
| Cheatgrass | 10C | 10C | 10C | 10C |
| Blackgrass | 8C,7G | 10C | 10C | 10C |
| Annual Bluegrass | 5C,6G | 10C | 10C | 10C |
| Green Foxtail | 4C,6G | 9C,9G | 8C,8G | 7C,7G |
| Quackgrass | 7C,7G | 9C,9G | 4C,6G | 10C |
| Italian Ryegrass | 7C,8G | 10C | 5C,7G | 10C |
| Ripgut Brome | 6C,7G | 9C,9G | 4C,4G | 9C,8G |
| Russian Thistle | 5C,6G | 10C | 10C | 10C |
| Tansy Mustard | 9C,9G | 10C | 10C | 10C |
| Smartweed | – | – | – | – |
| Tumble Mustard | 10C | 10C | 10C | 10C |
| Kochia | 9C,8G | 9C,9G | 10C | 10C |
| Shepherd's Purse | 10C | 10C | 10C | 10C |
| *Matricaria inodora* | 7C,8G | 10C | 8C,7G | 10C |
| Black Nightshade | 7C,6G | 5C,8G | 3C,4G | 2G |
| Yellow Rocket | 10C | 10C | 10C | 10C |
| Wild Mustard | 9C,8G | 10C | 10C | 10C |
| Wild Buckwheat | 4C,5G | 5C,6G | 7C,8G | 10C |

TABLE D (Continued)

| | Compound 3 | | | |
|---|---|---|---|---|
| | Pre-Emergence | | Post-Emergence | |
| Rate kg/ha | 0.008 | 0.03 | 0.008 | 0.03 |
| Wheat | 0 | 1C, 2G | 0 | 1C, 2G |
| Barley | 2G | 7C, 6G | 0 | 4C, 6G |
| Wild Oats | 1G | 7C, 7G | 0 | 7C, 5G |
| Downy Brome | 4G | 10C | 6C, 7G | 7C, 7G |
| Cheatgrass | 2C, 5G | 10C | 7C, 7G | 10C |
| Blackgrass | 7C, 7G | 10C | 4C, 5G | 10C |
| Annual Bluegrass | 3C, 4G | 5C, 6G | 4C, 6G | 5C, 7G |
| Green Foxtail | 2C, 3G | 5C, 4G | 2C, 4G | 6C, 7G |
| Quackgrass | 0 | 3C, 4G | 2C, 3G | 4C, 6G |
| Italian Ryegrass | 3G | 7C, 7G | 3C, 6G | 5C, 6G |
| Ripgut Brome | 0 | 10C | 1C, 2G | 3C, 4G |
| Russian Thistle | 0 | 7C, 7G | 3C, 2G | 10C |
| Tansy Mustard | 4C, 5G | 9C, 9G | 8C, 7G | 10C |
| Smartweed | – | – | – | – |
| Tumble Mustard | 7G | 10C | 10C | 10C |
| Kochia | 6G | 10C | 4G | 10C |
| Shepherd's Purse | 7C, 7G | 10C | 9C, 8G | 10C |
| *Matricaria inodora* | 6C, 7G | 7C, 7G | 6C, 5G | 9C, 8G |
| Black Nightshade | 3C, 4G | 5C, 6G | 4G | 8C, 8G |
| Yellow Rocket | 7G | 7C, 7G | 2C, 5G | 10C |
| Wild Mustard | 5C, 7G | 7C, 8G | 10C | 10C |
| Wild Buckwheat | 0 | 2C, 3G | 2C, 2G | 5C, 6G |

TABLE D (Continued)

Compound 36

| | Pre-Emergence | | Post-Emergence | |
|---|---|---|---|---|
| Rate kg/ha | 0.015 | 0.06 | 0.015 | 0.06 |
| Wheat | 0 | 0 | 0 | 0 |
| Barley | 0 | 0 | 0 | 0 |
| Wild Oats | 0 | 0 | 0 | 0 |
| Downy Brome | 3G | 6G | 0 | 3G |
| Cheatgrass | 7G | 10C | 1G | 3C, 5G |
| Blackgrass | 6G | 3C, 7G | 2G | 5C, 4G |
| Annual Bluegrass | 6G | 7C, 7G | 2G | 3G |
| Green Foxtail | 2G | 3C, 5G | 1G | 2G |
| Quackgrass | 0 | 2G | 0 | 0 |
| Italian Ryegrass | 1G | 3G | 0 | 0 |
| Ripgut Brome | 2G | 2C, 7G | 0 | 2G |
| Russian Thistle | 0 | 1G | 0 | 2G |
| Tansy Mustard | 2G | 2C, 5G | 5C, 4G | 7C, 8G |
| Smartweed | – | – | – | – |
| Tumble Mustard | 5G | 3C, 7G | 10C | 10C |
| Kochia | 0 | 5C, 6G | 0 | 0 |
| Shepherd's Purse | 7G | 3C, 8G | 7C, 8G | 10C |
| *Matricaria inodora* | 3C, 8G | 7C, 8G | 7C, 7G | 9C, 8G |
| Black Nightshade | 4G | 1C, 5G | 2G | 4C, 5G |
| Yellow Rocket | 3C, 8G | 10C | 7C, 7G | 10C |
| Wild Mustard | 7C, 8G | 10C | 10C | 10C |
| Wild Buckwheat | 3G | 1C, 4G | 1C, 2G | 5C, 4G |

Test E

Test samples were formulated and applied directly to the paddy water three days after transplanting of rice. The test was maintained in a greenhouse, and plant response ratings were taken at the stated intervals after application. The data indicate:

COMPOUND 1

| Rate g/ha | Rice 4 Days | Rice 4 Weeks | Barnyardgrass* 4 Weeks | Water Chestnut** 4 Weeks |
|---|---|---|---|---|
| 1 | 0 | 0 | 0 | 7G |
| 4 | 0 | 0 | 0 | 8G |
| 8 | 0 | 0 | 0 | 9G |

COMPOUND 36

| Rate, g/ha | Rice 1 Week | Rice 8 Weeks | Barnyardgrass* 8 Weeks | Water Chestnut** 8 Weeks | Arrowhead*** 8 Weeks |
|---|---|---|---|---|---|
| 25 | 0 | 0 | 0 | 10C | 0 |
| 100 | 0 | 0 | 0 | 10C | 0 |

* *Echinochloa* spp.

** *Eleocharis* spp.

*** *Sagittaria* spp.

It may be seen that compounds from within the scope of the invention can be used for selective weed control in rice.

Test F

The test chemicals, dissolved in a non-phytotoxic solvent, were applied in an overall spray to the foliage and surrounding oil of selected plant species. One day after treatment, plants were observed for rapid burn injury. Approximately fourteen days after treatment, all species were visually compared to untreated controls and rated for response to treatment. The rating system was as described previously for Test A. The data are presented in Table F.

All plant species were seeded in Woodstown sandy loam soil and grown in a greenhouse. The following species were grown in soil contained in plastic pots (25 cm diameter by 13 cm deep): soybeans, cotton, alfalfa, corn, rice, wheet, sorghum, velvetleaf (*Abutilon theophrasti*), sesbania (*Sesbania exaltata*), Cassia (*Cassia tora*), morningglory (*Ipomoea hederacea*), jimsonweed (*Datura stramonium*), cocklebur (*Xanthium pensylvanicum*), crabgrass (*Digitaria* spp.), nutsedge (*Cyperus rotundus*), barnyardgrass (*Echinochloa crusgalli*), giant foxtail (*Setaria faberii*) and wild oats (*Avena fatua*). The following species were grown in soil in a paper cup (12 cm diameter by 13 cm deep): sunflower, sugarbeets, and mustard. All plants were sprayed approximately 14 days after planting.

Some of the compounds tested by this procedure are useful for the post-emergence control of weeds in wheet. Compound 39 has utility for the post-emergence control of weeds in soybeans.

TABLE F

OVER-THE-TOP SOIL/FOLIAGE TREATMENT

| | Compound 1 | | | |
|---|---|---|---|---|
| Rate kg/ha | 0.06 | 0.015 | 0.007 | 0.003 |
| Soybeans | 10G, 7C | 10G, 7C | 9G, 5C | 9G, 5C |
| Velvetleaf | 10G, 7C | 8G, 3C | 9G, 9C | 10G, 9C |
| Sesbania | 9C | 9C | 10C | 10C |
| Cassia | 10G, 7C | 8G, 2C | 9G, 5C | 10G, 5C |
| Cotton | 10G, 7C | 7G, 3C | 10G, 9C | 10C |
| Morningglory | 10G, 5C | 9G, 4C | 10G, 8C | 10G, 8C |
| Alfalfa | 9C | 5C | 6G, 2C | 6G, 2C |
| Jimsonweed | 9C | 7G, 3C | 0 | 0 |
| Cocklebur | 10G, 7C | 9G, 3C | 7G | 5G |
| Corn | 10C | 9G, 7C | 8G, 2H | 9G, 1H |
| Crabgrass | 8G, 4C | 2C | 5G | 0 |
| Rice | 8G, 3C | 3G, 1C | 8G, 5C | 7G, 2C |
| Nutsedge | 10C | 10C | 5G | 3G |
| Barnyardgrass | 10C | 10C | 9G, 4C | 9G, 2C |
| Wheat | 0 | 0 | 0 | 1G |
| Giant Foxtail | 10G, 3C | 7G, 3C | 5G | 5G |
| Wild Oats | 6G | 3G | 3G | 3G |
| Sorghum | 10C | 9C | 8G, 3C | 7G, 4U |
| Mustard | – | – | 10C | 10C |
| Sunflower | – | – | 10C | 10C |
| Sugarbeets | – | – | 10C | 9G, 9C |

TABLE F (Continued)

OVER-THE-TOP SOIL/FOLIAGE TREATMENT

| | Compound 2 | |
|---|---|---|
| Rate kg/ha | 0.06 | 0.015 |
| Soybeans | 9C | 10G, 8C |
| Velvetleaf | 10G, 4C | 5G |
| Sesbania | 10C | 9C |
| Cassia | 10G, 7C | 7G, 3C |
| Cotton | 10G, 6C | 7G, 3C |
| Morningglory | 10C | 8G, 2C |
| Alfalfa | 10C | 7C |
| Jimsonweed | 10C | – |
| Cocklebur | 10G, 8C | 10G, 7C |
| Corn | 10C | 10G,7U |
| Crabgrass | 10C | 6G, 1C |
| Rice | 10C | 6G, 3C |
| Nutsedge | 10C | 2C |
| Barnyardgrass | 10C | 6G, 3C |
| Wheat | 9C | 1C |
| Giant Foxtail | 10C | – |
| Wild Oats | 10C | 5C, 3C |
| Sorghum | 10C | 8G, 5C |
| Mustard | – | – |
| Sunflower | – | – |
| Sugarbeets | – | – |

TABLE F (Continued)

OVER-THE-TOP SOIL/FOLIAGE TREATMENT

| | Compound 3 | | | |
|---|---|---|---|---|
| Rate kg/ha | 0.06 | 0.015 | 0.007 | 0.003 |
| Soybeans | 10G, 6C | 10G, 4C | 9G, 7C | 10G, 5C |
| Velvetleaf | 7G, 2C | 5G | 8G, 8C | 7G |
| Sesbania | 9C | 9C | 8G, 5C | 9G, 8C |
| Cassia | 10G, 7C | 5G, 3C | 10G, 5C | 8G, 4C |
| Cotton | 10G, 6C | 10G, 6C | 10C | 8G, 2C |
| Morningglory | 9G, 4C | 9G, 3C | 10G, 8C | 7G, 1H |
| Alfalfa | 9C | 8C | 7G | 6G, 2C |
| Jimsonweed | 9G, 3C | – | – | 0 |
| Cocklebur | 10G, 7C | 10G, 7C | 4G, 5C | 3G |
| Corn | 10C | 9G, 6C | 9G | 9G, 1H |
| Crabgrass | 7G, 4C | 0 | 5G, 1C | 0 |
| Rice | 8G, 5C | 7G, 3C | 9G, 7C | 9G, 7C |
| Nutsedge | 9G, 4C | 9G, 3C | 7G | 5G |
| Barnyardgrass | 10C | 9C | 9G, 4C | 9G, 2C |
| Wheat | 8C | 1C | 6G | 3G |
| Giant Foxtail | – | – | 0 | 0 |
| Wild Oats | 10C | 7G, 3C | 5G | 4G |
| Sorghum | 10C | 9C | 7G, 3U | 9G, 6U |
| Mustard | – | – | 10C | 9G, 9C |
| Sunflower | – | – | 10C | 10G, 9C |
| Sugarbeets | – | – | 10C | 9G, 9C |

TABLE F (Continued)

OVER-THE-TOP SOIL/FOLIAGE TREATMENT

| | Compound 36 | | |
|---|---|---|---|
| Rate kg/ha | 0.12 | 0.06 | 0.015 |
| Soybeans | 10C | 9G, 9C | 9G, 7C |
| Velvetleaf | 10C | 10C | 8G, 7C |
| Sesbania | 10C | 10C | 10C |
| Cassia | 10C | 9G, 3C | 8G, 3C |
| Cotton | 9G, 9C | 8G, 8C | 4G, 4C |
| Morningglory | 10C | 10C | 8G, 4C |
| Alfalfa | 9G | 5G, 3C | 7G |
| Jimsonweed | 0 | 0 | 0 |
| Cocklebur | 10C | 10C | 10C |
| Corn | 2G, 1C | 1G, 2C | 1G, 1C |
| Crabgrass | 5G | 0 | 0 |
| Rice | 5G | 4G | 2G |
| Nutsedge | 7G, 5C | 7G, 5C | 0 |
| Barnyardgrass | 9G, 5C | 8G, 5C | 6G, 2C |
| Wheat | 2G | 0 | 0 |
| Giant Foxtail | 3G, 2C | 2C | 0 |
| Wild Oats | 3G | 0 | 0 |
| Sorghum | 7G, 3C | 5G, 2C | 2G, 2C |
| Mustard | 10C | 10C | 10C |
| Sunflower | 10C | 10C | 10C |
| Sugarbeets | 9G, 3C | 9G, 5C | 8G |

TABLE F (Continued)

OVER-THE-TOP SOIL / FOLIAGE TREATMENT

| | Compound 39 | | | | | | |
|---|---|---|---|---|---|---|---|
| Rate kg/ha | 0.12 | 0.06 | 0.03 | 0.015 | 0.015 | 0.015 | 0.007 |
| Soybeans | 10G, 7C | 9G, 9C | 4G | 0 | 0 | 3G | 0 |
| Velvetleaf | 9G, 7C | 8G, 5C | 10G, 5C | 8G, 4C | 8G, 3C | 8G, 3C | 7G, 3C |
| Sesbania | 10C | 9G, 4C | 10G, 7C | 8G, 6C | 9G, 4C | 5G, 4C | 7G, 4C |
| Cassia | 9G, 7C | 5G, 5C | 8G, 5C | 5G, 5C | 8G, 4C | 4G, 6C | 5G, 3C |
| Cotton | 9G, 7C | 9G, 6C | 9G, 5C | 8G, 4C | 8G, 4C | 8G, 4C | 6G, 3C |
| Morningglory | 9G, 8C | 9G, 5C | 9G, 4C | 9G, 5C | 9G, 4C | 10G, 3C | 9G, 6C |
| Alfalfa | 9G, 8C | 10C | 9G, 5C | 5G, 2C | 9G, 5C | 7G | 7G, 3C |
| Jimsonweed | 0 | – | – | – | 8G | 2C | – |
| Cocklebur | 10G, 7C | 10G, 5C | 10G, 5C | 9G, 5C | 10G, 5C | 10G, 4C | 9G, 4C |
| Corn | 0 | 0 | 3G,2C | 0 | 1G, 2C | 0 | 2G |
| Crabgrass | 0 | 0 | 0 | 0 | 0 | 0 | 0 |
| Rice | 0 | 0 | 4G | 1G | 3G | 0 | 2G |
| Nutsedge | 1G | 0 | 3G | 0 | 0 | 0 | 5G |
| Barnyardgrass | 0 | 0 | 3G | 0 | 0 | 0 | 2G |
| Wheat | 0 | 0 | 0 | 0 | 0 | 0 | 0 |
| Giant Foxtail | 0 | 0 | 2G | 0 | 2G | 0 | 1G |
| Wild Oats | 0 | 0 | 2G | 0 | 1G | 2G | 0 |
| Sorghum | 0 | 0 | 5G | 0 | 4G | 5G | 3G |
| Mustard | 10C | 10C | 10G, 5C | 10G,5C | 10G, 5C | 9G | 10G, 5C |
| Sunflower | 10C | 10C | 10G, 8C | 10G, 8C | 10G, 8C | 10C | 10G, 8C |
| Sugarbeets | 10G, 5C | 10C | 9G, 3C | 10G, 5C | 9G, 3C | 9G | 10G, 4C |

Test G

This test demonstrates the broadleaf herbicidal properties of compound 39. Approximately 12 soybean plants were grown in a 25 cm. diameter plastic pot. In another pot of equal size the following broadleaf plant species were grown: sugarbeets, velvetleaf, *Sesbania, Cassia*, mustard, morningglory, alfalfa, jimsonweed, and cocklebur. The plant foliage and surrounding soil were treated with the test chemical dissolved in a non-phytotoxic solvent as an overall spray at the rate of 0.015 kg/ha, when the plants were 17 days old. Visual ratings of herbicidal effects were made 22 days after treatment utilizing the rating system described for Test A.

TABLE G

| Rate | 0.015 |
|---|---|
| Soybeans | 0 |
| Sugarbeets | 9G |
| Velvetleaf | 9G, 9C |
| Sesbania | 8G, 8C |
| Cassia | 7G, 4C |
| Mustard | 10C |
| Morningglory | 9G, 9C |
| Alfalfa | 8G, 4C |
| Jimsonweed | 0 |
| Cocklebur | 10P, 2C |

The data indicate that the compound tested provides excellent post-emergence control of broadleaved weeds in soybeans.

**Claims for the Contracting States: BE CH DE FR GB IT LI LU NL SE**

1. A compound of the formula:

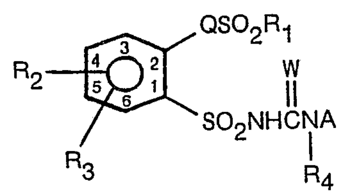

I

where
W is O or S;
Q is O or $NR_5$;
$R_1$ is $C_1$—$C_4$ alkyl, $C_1$—$C_4$ alkyl substituted with 1—3 atoms of F, Cl or Br, $CH_2CH_2OCH_3$, $CH_2CH_2CH_2OCH_3$ or

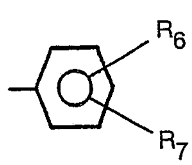

;

$R_2$ is H, F, Cl, Br, $OCH_3$, $NO_2$ or $C_1$—$C_2$ alkyl;
$R_3$ is H, F, Cl, Br or $CH_3$;
$R_4$ is H, $CH_3$ or $OCH_3$;
$R_5$ is $C_1$—$C_4$ alkyl;
$R_6$ and $R_7$ are independently H, F, Cl, Br, $CH_3$, $CF_3$, $NO_2$ or $OCH_3$;
A is

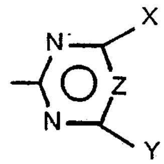 , 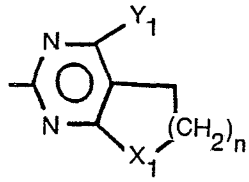 ,

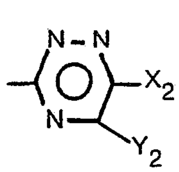 or 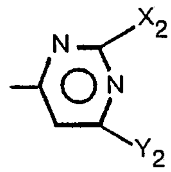 ;

X is $NH_2$, $N(CH_3)_2$, $NHCH_3$, $C_1$—$C_4$ alkyl, $C_1$—$C_4$ alkyl substituted with 1—3 atoms of F, Cl or Br, $CH_2OCH_3$, $CH_2OCH_2CH_3$, $C_1$—$C_4$ alkoxy, $C_1$—$C_2$ alkylthio, $C_3$—$C_4$ alkenyloxy, $C_3$—$C_4$ alkynyloxy, $OCH_2CH_2OCH_3$ or $C_2$—$C_4$ alkoxy substitued with 1—3 atoms of F, Cl or Br;

n is 1 or 2;

Y is H, $CH_3$, $OCH_3$ or Cl;

$X_1$ is O or $CH_2$;

$Y_1$ is H, $CH_3$, $OCH_3$ or Cl;

$X_2$ and $Y_2$ are independently $CH_3$ or $OCH_3$; and

Z is CH, N, $CCH_3$, CBr, CCl, CF, CI, $CC_2H_5$, $CCH_2CH_2Cl$ or $CCH_2CH{=}CH_2$;

provided that

(1) when Y is Cl, then Z is other than N and X is $NH_2$, $NHCH_3$, $N(CH_3)_2$, $CH_3$ or $OCH_3$;

(2) when Y is H, then X is $OCH_3$, $CH_3$ or $CH_2OCH_3$, and Z is other than N; and

(3) when W is S, then $R_4$ is H.

2. A compound of Claim 1 where $R_5$ is $CH_3$ W is O, and $R_4$ is H or $CH_3$.

3. A compound of Claim 2 where $R_1$ is $C_1$—$C_4$ alkyl, $CF_3$, $CH_2CH_2OCH_3$ or $CH_2CH_2CH_2OCH_3$.

4. A compound of Claim 3 where $R_2$ is H.

5. A compound of Claim 4 where $R_3$ is H.

6. A compound of Claim 5 where

$R_1$ is $C_1$—$C_3$ alkyl or $CF_3$;

Q is O;

A is

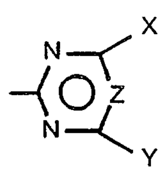 ;

and Z is CH or N.

7. A compound of Claim 6 where $R_4$ is H.

8. A compound of Claim 7 where X and Y are independently $CH_3$ or $OCH_3$, and $R_1$ is $CH_3$.

9. The compound of Claim 1 which is N-[(4,6-dimethoxypyrimidin-2-yl)aminocarbonyl]-2-hydroxybenzenesulfonamide, methanesulfonate.

10. The compound of Claim 1 which is 2-hydroxy-N-[(4-methoxy-6-methylpyrimidin-2-yl)aminocarbonyl]benzenesulfonamide, methanesulfonate.

11. The compound of Claim 1 which is N-[(4,6-dimethylpyrimidin-2-yl)aminocarbonyl]-2-hydroxybenzenesulfonamide, methanesulfonate.

12. The compound of Claim 1 which is N-[(4,6-dimethoxy-1,3,5-triazin-2-yl)aminocarbonyl]-2-hydroxybenzenesulfonamide, methanesulfonate.

13. The compound of Claim 1 which is 2-hydroxy-N-[(4-methoxy-6-methyl-1,3,5-triazin-2-yl)-aminocarbonyl]benzenesulfonamide, methanesulfonate.

14. The compound of Claim 1 which is N-[(4,6-dimethyl-1,3,5-triazin-2-yl)aminocarbonyl]-2-hydroxybenzenesulfonamide, methanesulfonate.

15. The compound of Claim 1 which is N-[(4,6-dimethoxy-1,3,5-triazin-2-yl)(methyl)aminocarbonyl]-2-hydroxybenzenesulfonamide, methanesulfonate.

16. The compound of Claim 1 which is N-[(4,6-dimethoxy-pyrimidin-2-yl)aminocarbonyl]-2-hydroxybenzenesulfonamide, 1-propanesulfonate.

17. The compound of Claim 1 which is 2-hydroxy-N-[(4-methoxy-6-methyl-1,3,5-triazin-2-yl)-aminocarbonyl]benzenesulfonamide, 1-propanesulfonate.

18. A compound of claim 1 wherein:

$R_1$ is $C_1$—$C_4$ alkyl, $C_1$—$C_4$ alkyl substituted with 1—3 atoms of F, Cl or Br or

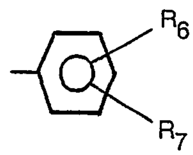 ;

$R_2$ is H, F, Cl, Br, $OCH_3$, $NO_2$ or $CH_3$;
$R_3$ is H, Cl or $CH_3$;
$R_4$ is H or $CH_3$;
$R_7$ is H, F, Cl, Br, $CH_3$, $CF_3$ or $OCH_3$;
A is

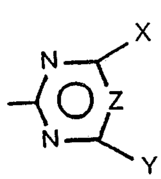 or 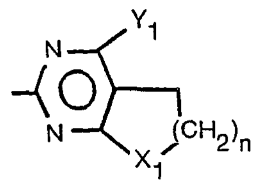 ,

where X is $C_1$—$C_4$ alkyl, $C_1$—$C_4$ alkyl substituted with 1—3 atoms of F, Cl or Br; $CH_2OCH_3$; $CH_2OCH_2CH_3$; $C_1$—$C_4$ alkoxy, $C_1$—$C_2$ alkylthio; $OCH_2CH{=}CH_2$; $OCH_2C{\equiv}CH$; $OCH_2C{\equiv}CCH_3$; $OCH_2CH_2OCH_3$; or $C_2$—$C_4$ alkoxy substituted with 1—3 atoms of F, Cl, or Br;
Y is $CH_3$, $OCH_3$ or Cl, or can be H when X is $CH_3$ or $OCH_3$;
$Y_1$ is H, $CH_3$ or $OCH_3$;
and W, Q, $R_5$, $R_6$, n, $X_1$ and Z are as defined in claim 1.

19. A process for the preparation of compounds of general formula I as defined in claim 1 which comprises (a) reacting a compound of formula

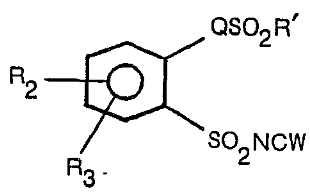

(wherein Q, W, $R_1$, $R_2$ and $R_3$ are as defined in Claim 1) with a compound of formula

HNA
|
$R_4$

(wherein A and $R_4$ are as defined above); or

(b) reacting a sulfonamide of formula

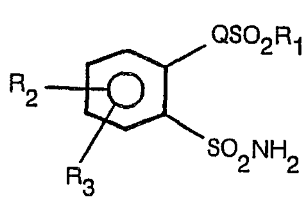

wherein $R_1$, $R_2$, $R_3$ and Q are as defined in Claim 1, with a 4,6-dichloroheterocyclic isocyanate of formula

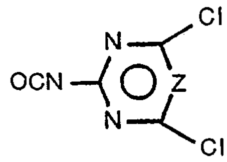

wherein Z is as defined in Claim 1;

whereafter one or both chlorine atoms on the heterocyclic ring in the resultant product are displaced by a suitable alkoxide to yield a compound of claim 1 wherein X is $OR_8$, where $R_8$ is $C_1$—$C_4$ alkyl, $C_3$—$C_4$ alkenyl, $C_3$—$C_4$ alkynyl, $CH_2CH_2OCH_3$ or $C_2$—$C_4$ alkyl substituted with 1—3 atoms of F, Cl or Br; and Y is Cl or $OCH_3$.

20. A composition suitable for controlling the growth of undesired vegetation which comprises at least one compound of formula I as defined in claims 1 or 18 in association with at least one of the following: surfactant, solid diluent and liquid diluent.

21. A composition according to claim 20, wherein the compound of formula I is a compound as defined in any one of Claims 2 to 17.

22. A method for controlling the growth of undesired vegetation in a locus which comprises applying to the locus an effective amount of at least one compound of formula I as defined in Claims 1 or 18.

23. A method according to Claim 22 wherein the compound of formula I is a compound as defined in any one of Claims 2 to 17.

24. A method for regulating the growth of plants in a locus which comprises applying to the locus an effective but substantially non-phytotoxic amount of a plant growth regulant selected from compounds of formula I as defined in any of Claims 1 to 18.

25. A compound of formula

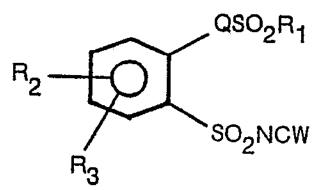

(where W. Q. $R_1$, $R_2$ and $R_3$ are as defined in Claim 1).

26. A compound of Claim 25 where Q is O, $R_2$ and $R_3$ are H, $R_1$ is $CH_3$ or $CF_3$, and W is O.

27. A compound of Claim 25 where Q is $NCH_3$, $R_2$ and $R_3$ are H, $R_1$ is $CH_3$ or $CF_3$, W is O.

28. A compound of formula

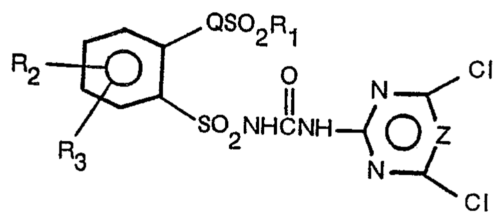

where

Q is O or $NR_5$;

$R_1$ is $C_1$—$C_4$ alkyl, $C_1$—$C_4$ alkyl substituted with 1—3 atoms of F, Cl or Br, $CH_2CH_2OCH_3$, $CH_2CH_2CH_2OCH_3$, or

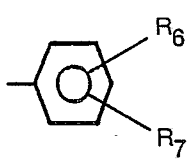 ;

$R_2$ is H, F, Cl, Br, $OCH_3$, $NO_2$ or $C_1$—$C_2$ alkyl;

$R_3$ is H, F, Cl, Br or $OCH_3$;

$R_5$ is $C_1$—$C_4$ alkyl;

$R_6$ and $R_7$ are independently H, F, Cl, Br, $CH_3$, $CF_3$, $NO_2$ or $OCH_3$; and

Z is CH or N.

**Claims for the Contracting State: AT**

1. A process for the preparation of a compound of formula:

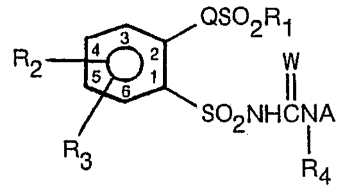

where

W is O or S;

Q is O or $NR_5$;

$R_1$ is $C_1$—$C_4$ alkyl, $C_1$—$C_4$ alkyl substituted with 1—3 atoms of F, Cl or Br, $CH_2CH_2OCH_3$, $CH_2CH_2CH_2OCH_3$ or

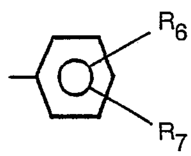

;

$R_2$ is H, F, Cl, Br, $OCH_3$, $NO_2$ or $C_1$—$C_2$ alkyl;

$R_3$ is H, F, Cl, Br or $CH_3$;

$R_4$ is H, $CH_3$ or $OCH_3$;

$R_5$ is $C_1$—$C_4$ alkyl;

$R_6$ and $R_7$ are independently H, F, Cl, Br, $CH_3$, $CF_3$, $NO_2$ or $OCH_3$;

A is

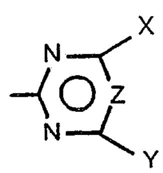

,

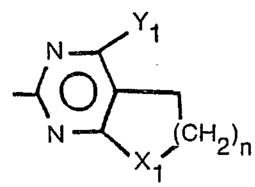

,

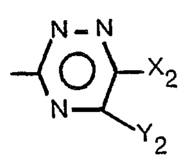

or

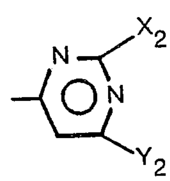

;

X is $NH_2$, $N(CH_3)_2$, $NHCH_3$, $C_1$—$C_4$ alkyl, $C_1$—$C_4$ alkyl substituted with 1—3 atoms of F, Cl or Br, $CH_2OCH_3$, $CH_2OCH_2CH_3$, $C_1$—$C_4$ alkoxy, $C_1$—$C_2$ alkylthio, $C_3$—$C_4$ alkenyloxy, $C_3$—$C_4$ alkynyloxy, $OCH_2CH_2OCH_3$ or $C_2$—$C_4$ alkoxy substitued with 1—3 atoms of F, Cl or Br;

n is 1 or 2;

Y is H, $CH_3$, $OCH_3$ or Cl;

$X_1$ is O or $CH_2$;

$Y_1$ is H, $CH_3$, $OCH_3$ or Cl;

$X_2$ and $Y_2$ are independently $CH_3$ or $OCH_3$; and

Z is CH, N, $CCH_3$, CBr, CCl, CF, CI, $CC_2H_5$, $CCH_2CH_2Cl$ or $CCH_2CH{=}CH_2$;

provided that

(1) when Y is Cl, then Z is other than N and X is $NH_2$, $NHCH_3$, $N(CH_3)_2$, $CH_3$ or $OCH_3$;

(2) when Y is H, then X is $OCH_3$, $CH_3$ or $CH_2OCH_3$, and Z is other than N; and

(3) when W is S, then $R_4$ is H which comprises (a) reacting a compound of formula

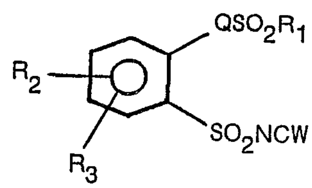

(wherein Q, W, $R_1$, $R_2$ and $R_3$ are as defined above) with a compound of formula

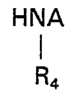

(wherein A and $R_4$ are as defined above); or

(b) reacting a sulfonamide of formula

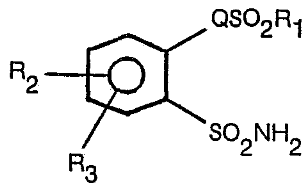

wherein $R_1$, $R_2$, $R_3$ and Q are as defined above, with a 4,6-dichloroheterocyclic isocyanate of formula

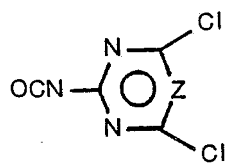

wherein Z is as defined above;

whereafter one or both chlorine atoms on the heterocyclic ring in the resultant product are displaced by a suitable alkoxide to yield a compound of formula I wherein X is $OR_8$, where $R_8$ is $C_1$—$C_4$ alkyl, $C_3$—$C_4$ alkenyl, $C_3$—$C_4$ alkynyl, $CH_2CH_2OCH_3$ or $C_2$—$C_4$ alkyl substituted with 1—3 atoms of F, Cl or Br; and Y is Cl or $OCH_3$.

2. A process according to Claim 1 wherein A in the compound of formula

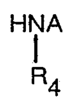 is 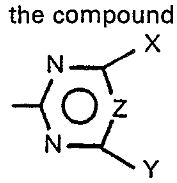

(wherein X, Y and Z are as defined in Claim 1).

3. A process according to Claim 1 wherein A in the compound of formula

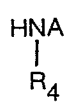 is 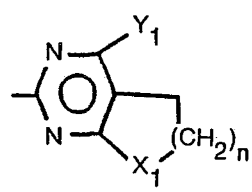

(wherein $X_1$, $Y_1$ and n are as defined in Claim 1).

4. A process according to Claim 1 wherein A in the compound of formula

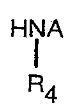 is 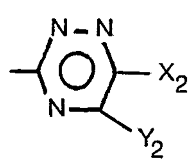

(wherein $X_2$ and $Y_2$ are as defined in Claim 1).

5. A process according to Claim 1 wherein A in the compound of formula

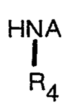 is 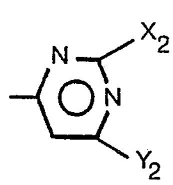

(wherein $X_2$ and $Y_2$ are as defined in Claim 1).

6. A process according to any one of Claims 1 to 5 for the preparation of a compound of formula I in which $R_5$ is $CH_3$, W is O and $R_4$ is H or $CH_3$.

7. A process according to claim 6 for the preparation of a compound of formula I in which $R_1$ is $C_1$—$C_4$ alkyl, $CF_3$, $CH_2CH_2OCH_3$ or $CH_2CH_2CH_2OCH_3$.

8. A process according to Claim 7 for the preparation of a compound of formula I in which $R_2$ is H.

9. A process according to Claim 8 for the preparation of a compound of formula I in which $R_3$ is H.

10. A process according to Claim 9 for the preparation of a compound of formula I in which $R_1$ is $C_1$—$C_3$ alkyl or $CF_3$, Q is O, and A is

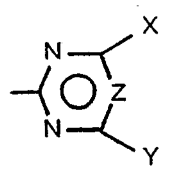 ;

(in which X and Y are as defined in Claim 1, and Z is CH or N).

11. A process according to Claim 10 for the preparation of a compound of formula I in which $R_4$ is H.

12. A process according to Claim 11 for the preparation of a compound of formula I in which X and Y are independently $CH_3$ or $OCH_3$, and $R_1$ is $CH_3$.

13. A process according to any of claims 1—3 or 6—12 wherein :

$R_1$ is $C_1$—$C_4$ alkyl, $C_1$—$C_4$ alkyl substituted with 1—3 atoms of F, Cl or Br or

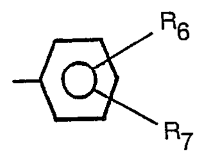 ;

$R_2$ is H, F, Cl, Br, $OCH_3$, $NO_2$ or $CH_3$;
$R_3$ is H, Cl or $CH_3$;
$R_4$ is H or $CH_3$;
$R_7$ is H, F, Cl, Br, $CH_3$, $CF_3$ or $OCH_3$;
A is

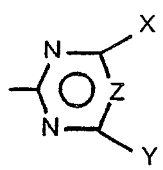 , or 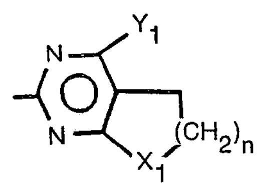 ,

where X is $C_1$—$C_4$ alkyl, $C_1$—$C_4$ alkyl substituted with 1—3 atoms of F, Cl or Br; $CH_2OCH_3$; $CH_2OCH_2CH_3$; $C_1$—$C_4$ alkoxy, $C_1$—$C_2$ alkylthio; $OCH_2CH{=}CH_2$; $OCH_2C{\equiv}CH$; $OCH_2C{\equiv}CCH_3$; $OCH_2CH_2OCH_3$; or $C_2$—$C_4$ alkoxy substituted with 1—3 atoms of F, Cl, or Br;

Y is $CH_3$, $OCH_3$ or Cl, or can be H when X is $CH_3$ or $OCH_3$;

$Y_1$ is H, $CH_3$ or $OCH_3$;

and W, Q, $R_5$, $R_6$, n, $X_1$ and Z are as defined in claim 1.

14. A process according to Claim 1 for the preparation of N-[(4,6-dimethoxypyrimidin-2-yl)aminocarbonyl]-2-hydroxybenzenesulfonamide, methanesulfonate.

15. A process according to Claim 1 for the preparation of 2-hydroxy-N-[(4-methoxy-6-methylpyrimidin-2-yl)aminocarbonyl]benzenesulfonamide, methanesulfonate.

16. A process according to Claim 1 for the preparation of N-[(4,6-dimethylpyrimidin-2-yl)aminocarbonyl]-2-hydroxybenzenesulfonamide, methanesulfonate.

17. A process according to Claim 1 for the preparation of N-[(4,6-dimethoxy-1,3,5-triazin-2-yl)aminocarbonyl]-2-hydroxybenzenesulfonamide, methanesulfonate.

18. A process according to Claim 1 for the preparation of 2-hydroxy-N-[(4-methoxy-6-methyl-1,3,5-triazin-2-yl)aminocarbonyl]benzenesulfonamide, methanesulfonate.

19. A process according to Claim 1 for the preparation of N-[(4,6-dimethyl-1,3,5-triazin-2-yl)aminocarbonyl]-2-hydroxybenzenesulfonamide, methanesulfonate.

20. A process according to Claim 1 for the preparation of N-[(4,6-dimethoxy-1,3,5-triazin-2-yl)(methyl)-aminocarbonyl]-2-hydroxybenzenesulfonamide, methanesulfonate.

21. A process according to Claim 1 for the preparation of N-[(4,5-dimethoxypyrimidin-2-yl)aminocarbonyl]-2-hydroxybenzenesulfonamide, 1-propanesulfonate.

22. A process according to Claim 1 for the preparation of 2-hydroxy-N-[(4-methoxy-6-methyl-1,3,5-triazin-2-yl)aminocarbonyl]benzenesulfonamide, 1-propanesulfonate.

23. A composition suitable for controlling the growth of undesired vegetation which comprises at least one compound of formula I as defined in any of Claims 1 to 22 in association with at least one of the following: surfactant, solid diluent and liquid diluent.

24. A method for controlling the growth of undesired vegetation in a locus which comprises applying to the locus an effective amount of at least one compound of formula I as defined in any of Claims 1 to 13.

25. A method according to Claim 24 wherein the compound of formula I is a compound as defined in any one of Claims 14 to 22.

26. A method for regulating the growth of plants in a locus which comprises applying to the locus an effective but substantially non-phytotoxic amount of a plant growth regulant selected from compounds of formula I as defined in any of Claims 1 to 22.

**Patentansprüche für die Vertragsstaaten: BE CH DE FR GB IT LI LU NL SE**

1. Verbindung mit der Formel

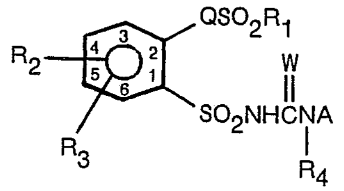

worin

W O oder S ist;

Q O oder $NR_5$ ist,

$R_1$ $C_1$—$C_4$-Alkyl, $C_1$—$C_4$-Alkyl substituiert mit 1—3 Atomen von F, Cl oder Br, $CH_2CH_2OCH_3$, $CH_2CH_2CH_2OCH_3$ oder

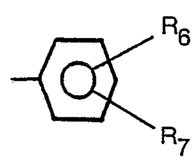

;

$R_2$ H, F, Cl, Br, $OCH_3$, $NO_2$ oder $C_1$—$C_2$-Alkyl ist,

$R_3$ H, F, Cl, Br oder $CH_3$ ist;

$R_4$ H, $CH_3$ oder $OCH_3$ ist;

$R_5$ $C_1$—$C_4$-Alkyl ist;

$R_6$ und $R_7$ unabhängig H, F, Cl, Br, $CH_3$, $CF_3$, $NO_2$ oder $OCH_3$ sind;

A

A is

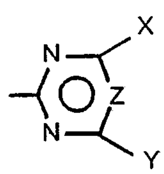

,

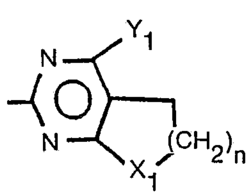

,

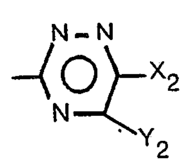

oder

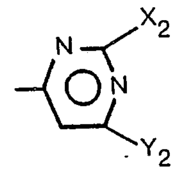

ist;

X $NH_2$, $N(CH_3)_2$, $NHCH_3$, $C_1$—$C_4$-Alkyl, $C_1$—$C_4$-Alkyl substituiert mit 1—3 Atomen von F, Cl oder Br, $CH_2OCH_3$, $CH_2OCH_2CH_3$, $C_1$—$C_4$-Alkoxy, $C_1$—$C_2$-Alkylthio, $C_3$—$C_4$-Alkenyloxy, $C_3$—$C_4$-Alkinyloxy, $OCH_2CH_2OCH_3$ oder $C_2$—$C_4$-Alkoxy substituiert mit 1—3 Atomen von F, Cl oder Br ist;

n 1 oder 2 ist;

Y H, $CH_3$, $OCH_3$ oder Cl ist;

$X_1$ O oder $CH_2$ ist;

$Y_1$ H, $CH_3$, $OCH_3$ oder Cl ist;

$X_2$ und $Y_2$ unabhängig $CH_3$ oder $OCH_3$ sind; und

Z CH, N, $CCH_3$, CBr, CCl, CF, Cl, $CC_2H_5$, $CCH_2CH_2Cl$ oder $CCH_2CH{=}CH_2$ ist;

vorausgesetzt, daß

1) wenn Y Cl ist, dann Z verschieden ist von N und X $NH_2$, $NHCH_3$, $N(CH_3)_2$, $CH_3$ oder $OCH_3$ ist;

2) wenn Y H ist, dann X $OCH_3$, $CH_3$ oder $CH_2OCH_3$ ist, und Z von N verschieden ist; und

3) wenn W S ist, dann $R_4$ H ist.

2. Verbindung nach Anspruch 1, worin $R_5$ $CH_3$ ist, W O ist, und $R_4$ H oder $CH_3$ ist.

3. Verbindung nach Anspruch 2, worin $R_1$ $C_1$—$C_4$-Alkyl, $CF_3$, $CH_2CH_2OCH_3$ oder $CH_2CH_2CH_2OCH_3$ ist.

4. Verbindung nach Anspruch 3, worin $R_2$ H ist.

5. Verbindung nach Anspruch 4, worin $R_3$ H ist.

6. Verbindung nach Anspruch 5, worin

$R_1$ $C_1$—$C_3$-Alkyl oder $CF_3$ ist;

Q O ist;

A

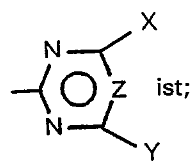

ist;

und Z CH oder N ist.

7. Verbindung nach Anspruch 6, worin $R_4$ H ist.

8. Verbindung nach Anspruch 7, worin X und Y unabhängig $CH_3$ oder $OCH_3$ sind, und $R_1$ $CH_3$ ist.

9. Verbindung nach Anspruch 1, nämlich N-[(4,6-Dimethoxypyrimidin-2-yl)aminocarbonyl]-2-hydroxy-benzolsulfonamid-methansulfonat.

10. Verbindung nach Anspruch 1, nämlich 2-Hydroxy-N-[(4-methoxy-6-methylpyrimidin-2-yl)-aminocarbonyl]-benzolsulfonamid-methansulfonat.

11. Verbindung nach Anspruch 1, nämlich N-[(4,6-Dimethylpyrimidin-2-yl)-aminocarbonyl]-2-hydroxybenzolsulfonamid-methansulfonat.

12. Verbindung nach Anspruch 1, nämlich N-[(4,6-Dimethoxy-1,3,5-triazin-2-yl)-aminocarbonyl]-2-hydroxy-benzolsulfonamid-methansulfonat.

13. Verbindung nach Anspruch 1, nämlich 2-Hydroxy-N-[(4-methoxy-6-methyl-1,3,5-triazin-2-yl)-aminocarbonyl]-benzolsulfonamid-methanesulfonat.

14. Verbindung nach Anspruch 1, nämlich N-[(4,6-Dimethyl-1,3,5-triazin-2-yl)-aminocarbonyl]-2-hydroxy-benzolsulfonamid-methansulfonat.

15. Verbindung nach Anspruch 1, nämlich N-[(4,6-Dimethoxy-1,3,5-triazin-2-yl)-(methyl)-aminocarbonyl]-2-hydroxy-benzolsulfonamid-methansulfonat.

16. Verbindung nach Anspruch 1, nämlich N-[(4,6-Dimethoxy-pyrimidin-2-yl)-aminocarbonyl]-2-hydroxy-benzolsulfonamid-1-propansulfonat.

17. Verbindung nach Anspruch 1, nämlich 2-Hydroxy-N-[4-methoxy-6-methyl-1,3,5-triazin-2-yl)-aminocarbonyl]-benzolsulfonamid-1-propansulfonat.

18. Verbindung nach Anspruch 1, worin

$R_1$ $C_1$—$C_4$-Alkyl, $C_1$—$C_4$-Alkyl substituiert mit 1—3 Atomen von F, Cl oder Br oder

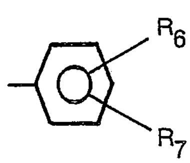

;

$R_2$ H, F, Cl, Br, $OCH_3$, $NO_2$ oder $CH_3$ ist;

$R_3$ H, Cl oder $CH_3$ ist;

$R_4$ H oder $CH_3$ ist;

$R_7$ H, F, Cl, Br, $CH_3$, $CF_3$ oder $OCH_3$ ist;

A

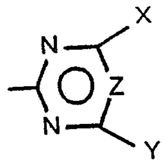 oder 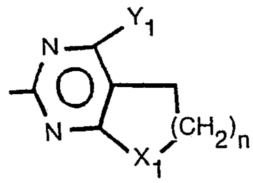

ist,

worin X $C_1$—$C_4$-Alkyl, $C_1$—$C_4$-Alkyl substituiert mit 1—3 Atomen von F, Cl oder Br; $CH_2OCH_3$; $CH_2OCH_2CH_3$; $C_1$—$C_4$-Alkoxy, $C_1$—$C_2$-Alkylthio; $OCH_2CH{=}CH_2$; $OCH_2C{\equiv}CH$; $OCH_2C{\equiv}CCH_3$; $OCH_2CH_2OCH_3$; oder $C_2$—$C_4$-Alkoxy substituiert mit 1—3 Atomen von F, Cl oder Br ist;

Y $CH_3$, $OCH_3$ oder Cl ist, oder H sein kann, wenn X $CH_3$ oder $OCH_3$ ist;

$Y_1$ H, $CH_3$ oder $OCH_3$ ist; und

W Q, $R_5$, $R_6$, n, $X_1$ und Z wie in Anspruch 1 definiert sind.

19. Verfahren zur Herstellung von Verbindungen der allgemeinen Formel I, wie in Anspruch 1 definiert, durch

(a) Reaktion einer Verbindung der Formel

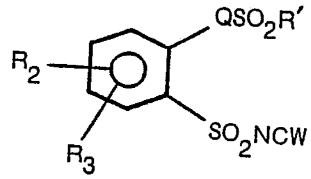

(worin Q, W, $R_1$, $R_2$ und $R_3$ wie in Anspruch 1 definiert sind) mit einer Verbindung der Formel

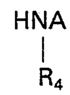

(worin A und $R_4$ wie vorstehend definiert sind); oder

(b) Reaktion eines Sulfonamids der Formel

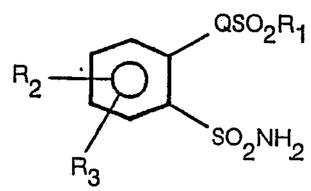

worin $R_1$, $R_2$, $R_3$ und Q wie in Anspruch 1 definiert sind, mit einem 4,6-dichlorheterocyclisch-Isocyanat der Formel

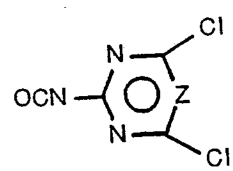

worin Z wie in Anspruch 1 definiert ist; worauf eines oder beide Chloratome an dem heterocyclischen Ring in dem resultierenden Produkt durch ein geeignetes Alkoxid ersetzt werden, unter Bildung einer Verbindung nach Anspruch 1, worin X $OR_8$ ist, worin $R_8$ $C_1$—$C_4$-Alkyl, $C_3$—$C_4$-Alkenyl, $C_3$—$C_4$-Alkinyl, $CH_2CH_2OCH_3$ oder $C_2$—$C_4$-Alkyl substituiert mit 1—3 Atomen von F, Cl oder Br ist; und Y Cl oder $OCH_3$ ist.

20. Zusammensetzung, geeignet zur Bekämpfung des Wachstums unerwünschter Vegetation, enthaltend mindestens eine Verbindung der Formel I, wie in den Ansprüche 1 oder 18 definiert, zusammen mit mindestens einem der folgenden: oberflächenaktives Mittel, festes Verdünnungsmittel und flüssiges Verdünnungsmittel.

21. Zusammensetzung nach Anspruch 20, worin die Verbindung der Formel I eine Verbindung, wie in einem der Ansprüche 2 bis 17 definiert, ist.

22. Verfahren zur Bekämpfung des Wachstums unerwüschter Vegetation an einem Ort, durch Auftragen auf den Ort von einer Wirksamen Menge mindstens einer Verbindung der Formel I, wie in den Ansprüchen 1 oder 18 definiert.

23. Verfahren nach Anspruch 22, bei dem die Verbindung der Formel I eine Verbindung wie in einem der Ansprüche 2 bis 17 definiert ist.

24. Verfahren zur Regulierung des Wachstums von Pflanzen an einem Ort, durch Auftrag auf den Ort von einer wirksamen, jedoch im wesentlichen nicht-phytotoxischen Menge, eines das Pflanzenwachstum regulierenden Mittels, ausgewählt aus den Verbindungen der Formel I, wie in einem der Ansprüche 1 bis 18 definiert.

25. Verbindung der Formel

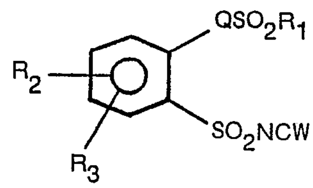

(worin W, Q, $R_1$, $R_2$ und $R_3$ wie in Anspruch 1 definiert sind).

26. Verbindung nach Anspruch 25, worin Q O ist, $R_2$ und $R_3$ H sind, $R_1$ $CH_3$ oder $CF_3$ ist, und W O ist.

27. Verbindung nach Anspruch 25, worin Q $NCH_3$ ist, $R_2$ und $R_3$ H sind, $R_1$ $CH_3$ oder $CF_3$ ist, und W O ist.

28. Verbindung der Formel

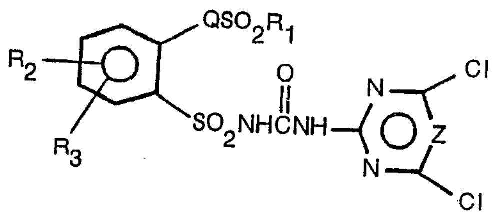

worin

Q O oder $NR_5$ ist;

$R_1$ $C_1$—$C_4$-Alkyl, $C_1$—$C_4$-Alkyl substituiert mit 1—3 Atomen von F, Cl oder Br, $CH_2CH_2OCH_3$, $CH_2CH_2CH_2OCH_3$ oder

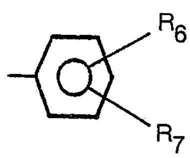 ist;

$R_2$ H, F, Cl, Br, $OCH_3$, $NO_2$ oder $C_1$—$C_2$-Alkyl ist;

$R_3$ H, F, Cl, Br oder $OCH_3$ ist;

$R_5$ $C_1$—$C_4$-Alkyl ist;

$R_6$ und $R_7$ unabhängig H, F, Cl, Br, $CH_3$, $CF_3$, $NO_2$ oder $OCH_3$ sind; und

Z CH oder N ist.

**Patentansprüche für den Vertragsstaat: AT**

1. Verfahren zur Herstellung einer Verbindung der Formel

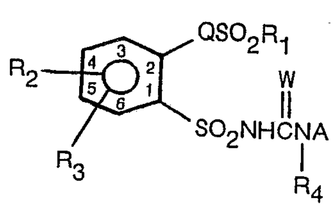

worin

W O oder S ist;

Q O oder $NR_5$ ist,

$R_1$ $C_1$—$C_4$-Alkyl, $C_1$—$C_4$-Alkyl substituiert mit 1—3 Atomen von F, Cl oder Br, $CH_2CH_2OCH_3$, $CH_2CH_2CH_2OCH_3$ oder

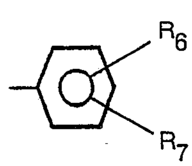 ist;

$R_2$ H, F, Cl, Br, $OCH_3$, $NO_2$ oder $C_1$—$C_2$-Alkyl ist,
$R_3$ H, F, Cl, Br oder $CH_3$ ist;
$R_4$ H, $CH_3$ oder $OCH_3$ ist;
$R_5$ $C_1$—$C_4$-Alkyl ist;
$R_6$ und $R_7$ unabhängig H, F, Cl, Br, $CH_3$, $CF_3$, $NO_2$ oder $OCH_3$ sind;
A

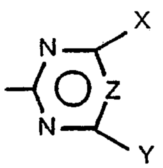 , 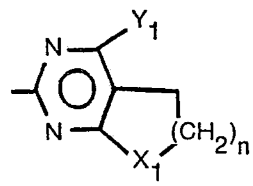 ,

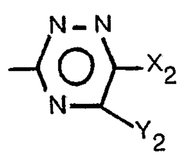 oder 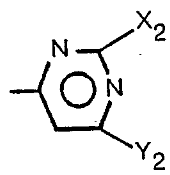

ist;

X $NH_2$, $N(CH_3)_2$, $NHCH_3$, $C_1$—$C_4$-Alkyl, $C_1$—$C_4$-Alkyl substituiert mit 1—3 Atomen von F, Cl oder Br, $CH_2OCH_3$, $CH_2OCH_2CH_3$, $C_1$—$C_4$-Alkoxy, $C_1$—$C_2$-Alkylthio, $C_3$—$C_4$-Alkenyloxy, $C_3$—$C_4$-Alkinyloxy, $OCH_2CH_2OCH_3$ oder $C_2$—$C_4$-Alkoxy substituiert mit 1—3 Atomen von F, Cl oder Br ist;
n 1 oder 2 ist;
Y H, $CH_3$, $OCH_3$ oder Cl ist;
$X_1$ O oder $CH_2$ ist;
$Y_1$ H, $CH_3$, $OCH_3$ oder Cl ist;
$X_2$ und $Y_2$ unabhängig $CH_3$ oder $OCH_3$ sind; und
Z CH, N, $CCH_3$, CBr, CCl, CF, CI, $CC_2H_5$, $CCH_2CH_2Cl$ oder $CCH_2CH{=}CH_2$ ist;
vorausgesetzt, daß
1) wenn Y Cl ist, dann Z von N verschieden ist, und X $NH_2$, $NHCH_3$, $N(CH_3)_2$, $CH_3$ oder $OCH_3$ ist;
2) wenn Y H ist, dann X $OCH_3$, $CH_3$ oder $CH_2OCH_3$ ist, und Z von N verschieden ist; und
3) wenn W S ist, dann $R_4$ H ist, durch
(a) Reaction einer Verbindung der Formel

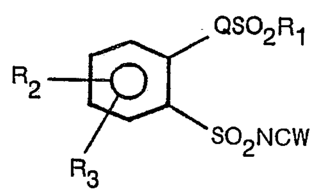

(worin Q, W, $R_1$, $R_2$ und $R_3$ wie vorstehend definiert sind) mit einer Verbindung der Formel

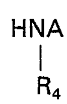

(worin A und $R_4$ wie vorstehend definiert sind); oder
(b) Reaktion eines Sulfonamids der Formel

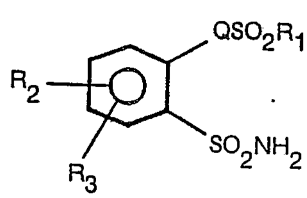

worin $R_1$, $R_2$, $R_3$ und Q wie vorstehend definiert sind, mit 4,6-dichlorheterocyclisch-Isocyanat der Formel

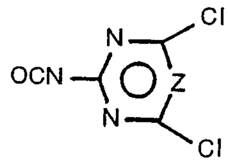

worin Z wie vorstehend definiert ist; worauf eines oder beide Chloratome an dem heterocyclischen Ring in dem resultierenden Produkt ersetzt werden durch ein geeignetes Alkoxid unter Erzielung einer Verbindung der Formel I, worin X $OR_8$ ist, worin $R_8$ $C_1$—$C_4$-Alkyl, $C_3$—$C_4$-Alkenyl, $C_3$—$C_4$-Alkinyl, $CH_2CH_2OCH_3$ oder $C_2$—$C_4$-Alkyl substituiert mit 1—3 Atomen von F, Cl oder Br ist; und Y Cl oder $OCH_3$ ist.

2. Verfahren nach Anspruch 1, worin A in der Verbindung der Formel

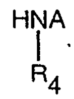

die Bedeutung hat von

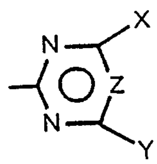

(worin X, Y und Z wie in Anspruch 1 definiert sind).

3. Verfahren nach Anspruch 1, worin A in der Verbindung der Formel

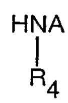

die Bedeutung hat von

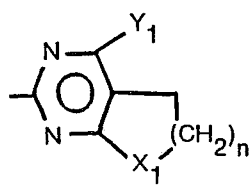

(worin $X_1$, $Y_1$ und n wie in Anspruch 1 definiert sind).

4. Verfahren nach Anspruch 1, worin A in der Verbindung der Formel

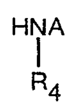

die Bedeutung hat von

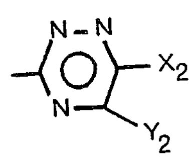

(worin $X_2$ und $Y_2$ wie in Anspruch 1 definiert sind).

5. Verfahren nach Anspruch 1, worin A in der Verbindung der Formel

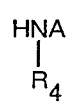

die Bedeutung hat von

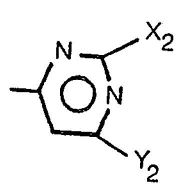

(worin $X_2$ und $Y_2$ wie in Anspruch 1 definiert sind).

6. Verfahren nach einem der Ansprüche 1 bis 5 zur Herstellung einer Verbindung der Formel I, worin $R_5$ $CH_3$ ist, W O ist, und $R_4$ H oder $CH_3$ ist.

7. Verfahren nach Anspruch 6, zur Herstellung einer Verbindung der Formel I, worin $R_1$ $C_1$—$C_4$-Alkyl, $CF_3$, $CH_2CH_2OCH_3$ oder $CH_2CH_2CH_2OCH_3$ ist.

8. Verfahren nach Anspruch 7, zur Herstellung einer Verbindung der Formel I, worin $R_2$ H ist.

9. Verfahren nach Anspruch 8, zur Herstellung einer Verbindung der Formel I, worin $R_3$ H ist.

10. Verfahren nach Anspruch 9, zur Herstellung einer Verbindung der Formel I, worin $R_1$ $C_1$—$C_3$-Alkyl oder $CF_3$ ist, Q O ist, und A

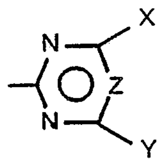 ;

ist, (worin X und Y wie in Anspruch 1 definiert sind, und Z CH oder N ist).

11. Verfahren nach Anspruch 10, zur Herstellung einer Verbindung der Formel I, worin $R_4$ H ist.

12. Verfahren nach Anspruch 11, zur Herstellung einer Verbindung der Formel I, worin X und Y unabhängig $CH_3$ oder $OCH_3$ sind, und $R_1$ $CH_3$ ist.

13. Verfahren nach einem der Ansprüche 1—3 oder 6—12, worin

$R_1$ $C_1$—$C_4$-Alkyl, $C_1$—$C_4$-Alkyl substituiert mit 1—3 Atomen von F, Cl oder Br oder

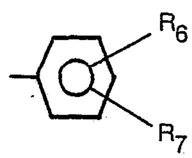 ist;

$R_2$ H, F, Cl, Br, $OCH_3$, $NO_2$ oder $CH_3$ ist;
$R_3$ H, Cl oder $CH_3$ ist;
$R_4$ H oder $CH_3$ ist;
$R_7$ H, F, Cl, Br, $CH_3$, $CF_3$ oder $OCH_3$ ist;
A

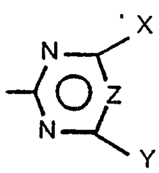 oder 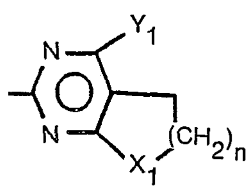

ist,

worin X $C_1$—$C_4$-Alkyl, $C_1$—$C_4$-Alkyl substituiert mit 1—3 Atomen von F, Cl oder Br; $CH_2OCH_3$; $CH_2OCH_2CH_3$; $C_1$—$C_4$-Alkoxy, $C_1$—$C_2$-Alkylthio; $OCH_2CH{=}CH_2$; $OCH_2C{\equiv}CH$; $OCH_2C{\equiv}CCH_3$; $OCH_2CH_2OCH_3$; oder $C_2$—$C_4$-Alkoxy substituiert mit 1—3 Atomen von F, Cl oder Br ist; und

Y $CH_3$, $OCH_3$ oder Cl ist, oder H sein kann, wenn X $CH_3$ oder $OCH_3$ ist;
$Y_1$ H, $CH_3$ oder $OCH_3$ ist; und
W Q, $R_5$, $R_6$, n, $X_1$ und Z wie in Anspruch 1 definiert sind.

14. Verfahren nach Anspruch 1 zur Herstellung von N-[(4,6-Dimethoxypyrimidin-2-yl)-aminocarbonyl]-2-hydroxybenzolsulfonamid-methansulfonat.

15. Verfahren nach Anspruch 1 zur Herstellung von 2-Hydroxy-N-[(4-methoxy-6-methylpyrimidin-2-yl)-aminocarbonyl]-benzolsulfonamid-methansulfonat.

16. Verfahren nach Anspruch 1 zur Herstellung von N-[(4,6-Dimethylpyrimidin-2-yl)-aminocarbonyl]-2-hydroxybenzolsulfonamid-methansulfonat.

17. Verfahren nach Anspruch 1 zur Herstellung von N-[(4,6-Dimethoxy-1,3,5-triazin-2-yl)-aminocarbonyl]-2-hydroxybenzolsulfonamid-methansulfonat.

18. Verfahren nach Anspruch 1 zur Herstellung von 2-Hydroxy-N-[(4-methoxy-6-methyl-1,3,5-triazin-2-yl)-aminocarbonyl]-benzolsulfonamid-methansulfonat.

19. Verfahren nach Anspruch 1 zur Herstellung von N-[(4,6-Dimethyl-1,3,5-triazin-2-yl)-aminocarbonyl]-2-hydroxybenzolsulfonamid-methansulfonat.

20. Verfahren nach Anspruch 1 zur Herstellung von N-[(4,6-Dimethoxy-1,3,5-triazin-2-yl)-(methyl)-aminocarbonyl]-2-hydroxybenzolsulfonamid-methansulfonat.

21. Verfahren nach Anspruch 1 zur Herstellung von N-[(4,5-Dimethoxypyrimidin-2-yl)-aminocarbonyl]-2-hydroxybenzolsulfonamid-1-propansulfonat.

22. Verfahren nach Anspruch 1 zur Herstellung von 2-Hydroxy-N-[4-methoxy-6-methyl-1,3,5-triazin-2-yl)-aminocarbonyl]-benzolsulfonamid-1-propansulfonat.

23. Zusammensetzung, geeignet zur Bekämpfung des Wachstums unerwünschter Vegetation, enthaltend mindestens eine Verbindung der Formel I, wie in einem der Ansprüche 1 bis 22 definiert, zusammen mit mindestens einem der folgenden: oberflächenaktives Mittel, festes Verdünnungsmittel und flüssiges Verdünnungsmittel.

24. Verfahren zur Bekämpfung des Wachstums unerwünschter Vegetation an einem Ort, durch Auftrag auf den Ort von einer wirksamen Menge mindestens einer Verbindung der Formel I, wie in einem der Ansprüche 1 bis 13 definiert.

25. Verfahren nach Anspruch 24, bei dem die Verbindung der Formel I eine Verbindung, wie in einem der Ansprüche 14 bis 22 definiert, ist.

26. Verfahren zur Regulierung des Wachstums von Pflanzen an einem Ort, durch Auftrag auf den Ort von einer wirksamen, jedoch im wesentlichen nicht-phytotoxischen Menge, eines das Pflanzenwachstum regulierenden Mittels, ausgewählt aus Verbindungen der Formel I, wie in einem der Ansprüche 1 bis 22 definiert.

**Revendications pour les Etats contractants: BE CH DE FR GB IT LI LU NL SE**

1. Un composé de la formule

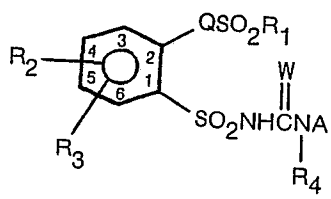

où

W est O ou S;

Q est O ou $NR_5$;

$R_1$ est un radical alkyle en $C_1$—$C_4$, alkyle en $C_1$—$C_4$ substitué par 1—3 atomes de F, Cl ou Br, $CH_2CH_2OCH_3$, $CH_2CH_2CH_2OCH_3$ ou

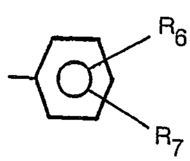 ;

$R_2$ est H, F, Cl, Br, $OCH_3$, $NO_2$ ou un radical alkyle en $C_1$—$C_2$;

$R_3$ est H, F, Cl, Br ou $CH_3$;

$R_4$ est H, $CH_3$ ou $OCH_3$;

$R_5$ est un radical alkyle en $C_1$—$C_4$;

$R_6$ et $R_7$ sont indépendamment H, F, Cl, Br, $CH_3$, $CF_3$, $NO_2$ ou $OCH_3$;

A est

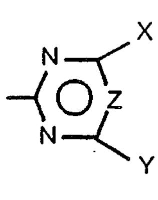 ,

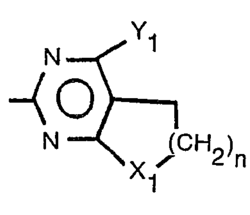 ,

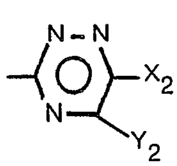 ou 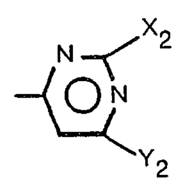 ;

X est $NH_2$, $N(CH_3)_2$, $NHCH_3$, un radical alkyle en $C_1$—$C_4$, alkyle en $C_1$—$C_4$ substitué par 1—3 atomes de F, Cl ou Br, $CH_2OCH_3$, $CH_2OCH_2CH_3$, alkoxy en $C_1$—$C_4$, alkylthio en $C_1$—$C_2$, alkényloxy en $C_3$—$C_4$, alkynyloxy en $C_3$—$C_4$, $OCH_2CH_2OCH_3$ ou alkoxy en $C_2$—$C_4$ substitué par 1—3 atomes de F, Cl ou Br;

n est 1 ou 2;

Y est H, $CH_3$, $OCH_3$ ou Cl;

$X_1$ est O ou $CH_2$;

$Y_1$ est H, $CH_3$ $OCH_3$ ou Cl;

$X_2$ est $Y_2$ sont indépendamment $CH_3$ ou $OCH_3$; et

Z est CH, N, $CCH_3$, CBr, CCl, CF, Cl, $CC_2H_5$, $CCH_2CH_2Cl$ ou $CCH_2CH{=}CH_2$;

avec les conditions que

(1) quand Y est Cl, alors Z est autre que N et X est $NH_2$; $NHCH_3$; $N(CH_3)_2$, $CH_3$ ou $OCH_3$;

(2) quand Y est H, alors X est $OCH_3$, $CH_3$ ou $CH_2OCH_3$, et Z est autre que N; et

(3) quand W est S, alors $R_4$ est H.

2. Un composé selon la revendication, dans lequel $R_5$ est $CH_3$, W est O et $R_4$ est H ou $CH_3$.

3. Un composé selon la revendication 2, dans lequel $R_1$ est un radical alkyle en $C_1$—$C_4$, $CF_3$, $CH_2CH_2OCH_3$ ou $CH_2CH_2CH_2OCH_3$.

4. Un composé selon la revendication 3, dans lequel $R_3$ est H.

5. Un composé selon la revendication 4, dans lequel $R_3$ est H.

6. Un composé selon la revendication 5, dans lequel $R_1$ est un radical alkyle en $C_1$—$C_3$ ou $CF_3$;

Q est O;

A est

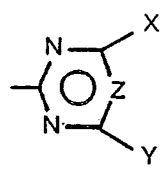

et Z est CH ou N.

7. Un composé selon la revendication 6, dans lequel $R_4$ est H.

8. Un composé selon la revendication 7, dans lequel X et Y sont indépendamment $CH_3$ ou $OCH_3$, et $R_1$ est $CH_3$.

9. Un composé selon la revendication 1, qui est le méthanesulfonate de N-[(4,6-diméthoxy-pyrimidin-2-yl)aminocarbonyl]-2-hydroxybenzènesulfonamide.

10. Un composé selon la revendication 1, qui est le méthanesulfonate de 2-hydroxy-N-[(4-méthoxy-6-méthylpyrimidin-2-yl)aminocarbonyl]benzènesulfonamide.

11. Un composé selon la revendication 1, qui est le méthanesulfonate de N-[(4,6-diméthyl-pyrimidin-2-yl)aminocarbonyl]-2-hydroxybenzènesulfonamide.

12. Un composé selon la revendication 1, qui est le méthanesulfonate de N-[(4,6-diméthoxy-1,3,5-triazin-2-yl)aminocarbonyl]-2-hydroxybenzènesulfonamide.

13. Un composé selon la revendication 1, qui est le méthanesulfonate de 2-hydroxy-N-[(4-méthoxy-6-méthyl-1,3,5-triazin-2-yl)aminocarbonyl]benzènesulfonamide.

14. Un composé selon la revendication 1, qui est le méthanesulfonate de N-[(4,6-diméthyl-1,3,5-triazin-2-yl)aminocarbonyl]-2-hydroxybenzènesulfonamide.

15. Un composé selon la revendication 1, qui est le méthanesulfonate de N-[(4,6-diméthoxy-1,3,5-triazin-2-yl) (méthyl)aminocarbonyl]-2-hydroxybenzènesulfonamide.

16. Un composé selon la revendication 1, qui est le 1-propanesulfonate de N-[(4,6-diméthoxy-pyrimidin-2-yl)aminocarbonyl]-2-hydroxybenzènesulfonamide.

17. Un composé selon la revendication 1, qui est le 1-propanesulfonate de 2-hydroxy-N-[(4-méthoxy-6-méthyl-1,3,5-triazin-2-yl)aminocarbonyl]benzènesulfonamide.

18. Un composé selon la revendication 1, dans lequel

$R_1$ est un radical alkyle en $C_1$—$C_4$, alkyle en $C_1$—$C_4$ substitué par 1—3 atomes de F, Cl ou Br ou

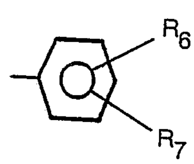

;

$R_2$ est H, F, Cl, Br, $OCH_3$, $NO_2$ ou $CH_3$;

$R_3$ est H, Cl ou $CH_3$;

$R_4$ est H ou $CH_3$;

$R_7$ est H, F, Cl, Br, $CH_3$, $CF_3$ ou $OCH_3$,

A est

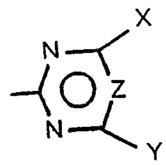

ou

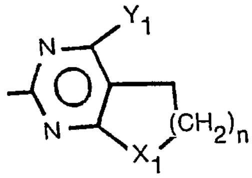

,

où X est un radical alkyle en $C_1$—$C_4$, alkyle en $C_1$—$C_4$ substitué par 1—3 atomes de F, Cl ou Br; $CH_2OCH_3$; $CH_2OCH_2CH_3$; alkoxy en $C_1$—$C_4$; alkylthio en $C_1$—$C_2$; $OCH_2CH{=}CH_2$; $OCH_2C{\equiv}CH$; $OCH_2C{\equiv}CCH_3$; $OCH_2CH_2OCH_3$; ou alkoxy en $C_2$—$C_4$ substitué par 1—3 atomes de F, Cl ou Br;

Y est $CH_3$, $OCH_3$ ou Cl, ou peut être H quand X est $CH_3$ ou $OCH_3$;

$Y_1$ est H, $CH_3$ ou $OCH_3$;

et W, Q, $R_5$, $R_6$, n, $X_1$ et Z sont tels que définis dans la revendication 1.

19. Un procédé pour la préparation de composés de formule générale I comme défini dans la revendication 1, qui comprend (a) la réaction d'un composé de formule

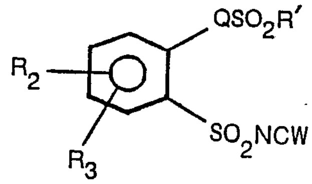

(où Q, W, $R_1$, $R_2$ et $R_3$ sont tels que définis dans la revendication 1) avec un composé de formule

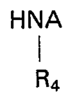

(où A et $R_4$ sont tels que définis ci-dessus); ou

(b) la réaction d'un sulfonamide de formule

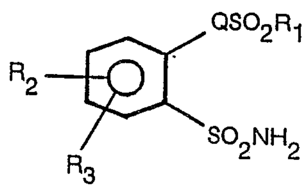

où $R_1$, $R_2$, $R_3$ et Q sont tels que définis dans la revendication 1, avec un isocyanate 4,6-dichlorohétérocyclique de formule

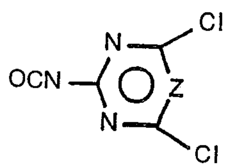

où Z est tel que défini dans la revendication 1;

après quoi un atome de chlore ou les deux sur le noyau hétérocyclique du produit résultant sont déplacés par un alcoolate approprié pour donner un composé selon la revendication 1 dans lequel X est $OR_8$, où $R_8$ est un radical alkyle en $C_1$—$C_4$, alkényle en $C_3$—$C_4$, alkynyle en $C_3$—$C_4$, $CH_2CH_2OCH_3$ ou alkyle en $C_2$—$C_4$ substitué par 1—3 atomes de F, Cl ou Br; et Y est Cl ou $OCH_3$.

20. Une composition utile pour lutter contre la croissance de végétation indésirable, qui comprend au moins un composé de formule I comme défini dans les revendications 1 ou 18 en association avec au moins l'un des ingrédients suivants: agent tensio-actif, diluant solide et diluant liquide.

21. Une composition selon la revendication 20, dans laquelle le composé de formule I est un composé tel que défini dans l'une quelconque des revendications 2 à 17.

22. Un procédé pour lutter contre la croissance de végétation indésirable à un endroit qui comprend l'application à cet endroit d'une quantité efficace d'au moins un composé de formule I comme défini dans les revendications 1 ou 18.

23. Un procédé selon la revendication 22, dans lequel le composé de formule I est un composé tel que défini dans l'une quelconque des revendications 2 à 17.

24. Un procédé pour régler la croissance de plantes à un endroit qui comprend l'application à cet endroit d'une quantité efficace mais substantiellement non-phytotoxique d'un régulateur de la croissance de plantes choisi parmi les composés de formule I comme défini dans l'une quelconque des revendications 1 à 18.

25. Un composé de formule

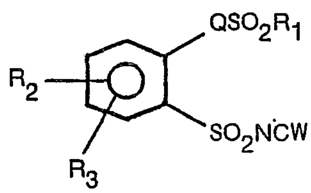

(où W, Q, $R_1$, $R_2$ et $R_3$ sont tels que définis dans la revendication 1).

26. Un composé selon la revendication 25, dans lequel Q est O, $R_2$ et $R_3$ sont H, $R_1$ est $CH_3$ ou $CF_3$ et W est O.

27. Un composé selon la revendication 25, dans lequel Q est $NCH_3$, $R_2$ et $R_3$ sont H, $R_1$ est $CH_3$ ou $CF_3$ et W est O.

28. Un composé de formule

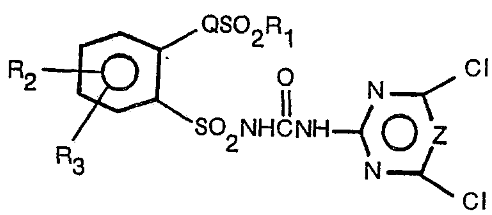

où

Q est O ou $NR_5$;

$R_1$ est un radical alkyle en $C_1$—$C_4$, alkyle en $C_1$—$C_4$ substitué par 1—3 atomes de F, Cl ou Br, $CH_2CH_2OCH_3$, $CH_2CH_2CH_2OCH_3$ ou

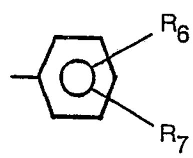 ;

$R_2$ est H, F, Cl, Br, $OCH_3$, $NO_2$ ou un radical alkyle en $C_1$—$C_2$;

$R_3$ est H, F, Cl, Br ou $OCH_3$;

$R_5$ est un radical alkyle en $C_1$—$C_4$;

$R_6$ et $R_7$ sont indépendamment H, F, Cl, Br, $CH_3$, $CF_3$, $NO_2$ ou $OCH_3$; et

Z est CH ou N.

**Revendications pour l'Etat contractant: AT**

1. Un procédé de préparation d'un composé de formule:

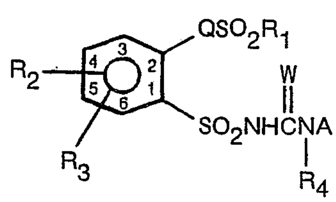

où

W est O ou S;

Q est O ou $NR_5$;

$R_1$ est un radical alkyle en $C_1$—$C_4$, alkyle en $C_1$—$C_4$ substitué par 1—3 atomes de F, Cl ou Br, $CH_2CH_2OCH_3$, $CH_2CH_2CH_2OCH_3$ ou

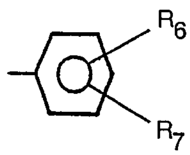

;

$R_2$ est H, F, Cl, Br, $OCH_3$, $NO_2$ ou un radical alkyle en $C_1$—$C_2$;
$R_3$ est H, F, Cl, Br ou $CH_3$;
$R_4$ est H, $CH_3$ ou $OCH_3$;
$R_5$ est un radical alkyle en $C_1$—$C_4$;
$R_6$ et $R_7$ sont indépendamment H, F, Cl, Br, $CH_3$, $CF_3$, $NO_2$ ou $OCH_3$;
A est

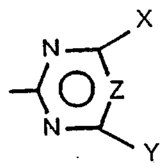 , 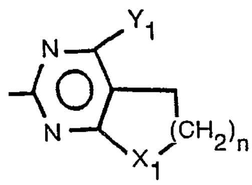 ,

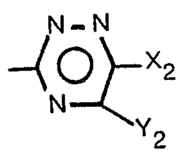 ou 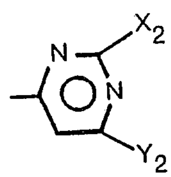 ;

X est $NH_2$, $N(CH_3)_2$, $NHCH_3$, un radical alkyle en $C_1$—$C_4$, alkyle en $C_1$—$C_4$ substitué par 1—3 atomes de F, Cl ou Br, $CH_2OCH_3$, $CH_2OCH_2CH_3$, alkoxy en $C_1$—$C_4$, alkylthio en $C_1$—$C_2$, alkényloxy en $C_3$—$C_4$, alkynyloxy en $C_3$—$C_4$, $OCH_2CH_2OCH_3$ ou alkoxy en $C_2$—$C_4$ substitué par 1—3 atomes de F, Cl ou Br;
n est 1 ou 2;
Y est H, $CH_3$, $OCH_3$ ou Cl;
$X_1$ est O ou $CH_2$;
$Y_1$ est H, $CH_3$, $OCH_3$ ou Cl;
$X_2$ et $Y_2$ sont indépendamment $CH_3$ ou $OCH_3$; et
Z est CH, N, $CCH_3$, CBr, CCl, CF, Cl, $CC_2H_5$, $CCH_2CH_2Cl$ ou $CCH_2CH{=}CH_2$;
avec les conditions que
(1) quand Y est Cl, alors Z est autre que N et X est $NH_2$; $NHCH_3$; $N(CH_3)_2$, $CH_3$ ou $OCH_3$;
(2) quand Y est H, alors X est $OCH_3$, $CH_3$ ou $CH_2OCH_3$, et Z est autre que N; et
(3) quand W est S, alors $R_4$ est H.
qui comprend (a) la réaction d'un composé de formule

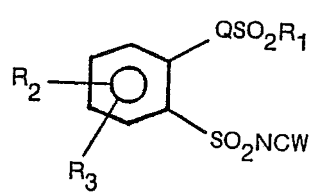

(où Q, W, $R_1$, $R_2$ et $R_3$ sont tels que définis dans la revendication 1) avec un composé de formule

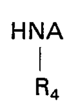

(où A et $R_4$ sont tels que définis ci-dessus); ou
(b) la réaction d'un sulfonamide de formule

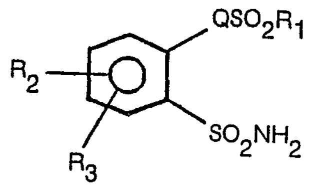

où $R_1$, $R_2$, $R_3$ et Q sont tels que définis dans la revendication 1, avec un isocyanate 4,6-dichlorohétérocyclique de formule

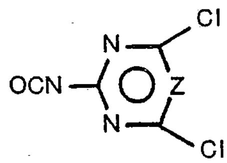

où Z est tel que défini dans la revendication 1;
après quoi un atome de chlore ou les deux sur le noyau hétérocyclique du produit résultant sont déplacés par un alcoolate approprié pour donner un composé selon la revendication 1 dans lequel X est $OR_8$, où $R_8$ est un radical alkyle en $C_1$—$C_4$, alkényle en $C_3$—$C_4$, alkynyle en $C_3$—$C_4$, $CH_2CH_2OCH_3$ ou alkyle en $C_2$—$C_4$ substitué par 1—3 atomes de F, Cl ou Br; et Y est Cl ou $OCH_3$.

2. Un procédé selon la revendication 1, dans lequel A dans le composé de formule

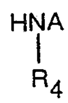 est 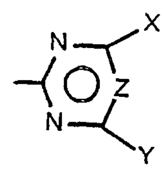

(où X, Y et Z sont tels que définis dans la revendication 1).

3. Un procédé selon la revendication 1, dans lequel A dans le composé de formule

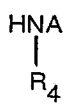 est 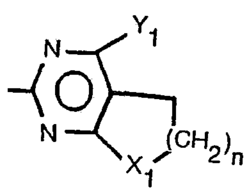

(où $X_1$, $Y_1$ et n sont tels que définis dans la revendication 1).

4. Un procédé selon la revendication 1, dans lequel A dans le composé de formule

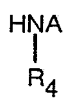 est 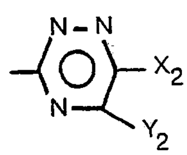

(où $X_2$, et $Y_2$ sont tels que définis dans la revendication 1).

5. Un procédé selon la revendication 1, dans lequel A dans le composé de formule

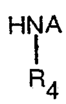 est 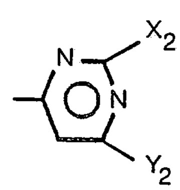

(où $X_2$ et $Y_2$ sont tels que définis dans la revendication 1).

6. Un procédé selon l'une quelconque des revendications 1 à 5 pour la préparation d'un composé de formule I dans lequel $R_5$ est $CH_3$, W est O et $R_4$ est H ou $CH_3$.

7. Un procédé selon la revendication 6 pour la préparation d'un composé de formule I dans lequel $R_1$ est un radical alkyle en $C_1$—$C_4$, $CF_3$, $CH_2CH_2OCH_3$ ou $CH_2CH_2CH_2OCH_3$.

8. Un procédé selon la revendication 7 pour la préparation d'un composé de formule I dans lequel $R_2$ est H.

9. Un procédé selon la revendication 8 pour la préparation d'un composé de formule I dans lequel $R_3$ est H.

10. Un procédé selon la revendication 9 pour la préparation d'un composé de formule I dans lequel $R_1$ est un radical alkyle en $C_1$—$C_3$ ou $CF_3$, Q est O et A est

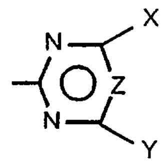

;

(où X et Y sont tels que définis dans la revendication 1 et Z est CH ou N).

11. Un procédé selon la revendication 10 pour la préparation d'un composé de formule I dans lequel $R_4$ est H.

12. Un procéde selon la revendication 11 pour la préparation d'un composé de formule I dans lequel X et Y sont indépendamment $CH_3$ ou $OCH_3$ et $R_1$ est $CH_3$.

13. Un procédé selon l'une quelconque des revendications 1—3 ou 6—12, dans lequel

$R_1$ est un radical alkyle en $C_1$—$C_4$, alkyle en $C_1$—$C_4$ substitué par 1—3 atomes de F, Cl ou Br ou

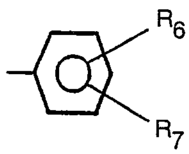

;

$R_2$ est H, F, Cl, Br, $OCH_3$, $NO_2$ ou $CH_3$;
$R_3$ est H, Cl ou $CH_3$;
$R_4$ est H ou $CH_3$;
$R_7$ est H, F, Cl, Br, $CH_3$, $CF_3$ ou $OCH_3$,
A est

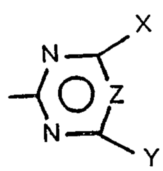

, ou

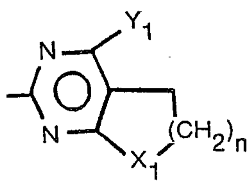

,

où X est un radical alkyle en $C_1$—$C_4$, alkyle en $C_1$—$C_4$ substitué par 1—3 atomes de F, Cl ou Br; $CH_2OCH_3$; $CH_2OCH_2CH_3$; alkoxy en $C_1$—$C_4$; alkylthio en $C_1$—$C_2$; $OCH_2CH{=}CH_2$; $OCH_2C{\equiv}CH$; $OCH_2C{\equiv}CCH_3$; $OCH_2CH_2OCH_3$; ou alkoxy en $C_2$—$C_4$ substitué par 1—3 atomes de F, Cl ou Br;
Y est $CH_3$, $OCH_3$ ou Cl, ou peut être H quand X est $CH_3$ ou $OCH_3$;
$Y_1$ est H, $CH_3$ ou $OCH_3$;
et W, Q, $R_5$, $R_6$, n, $X_1$ et Z sont tels que définis dans la revendication 1.

14. Un procédé selon la revendication 1 pour la préparation du méthanesulfonate de N-[(4,6-diméthoxypyrimidin-2-yl)aminocarbonyl]-2-hydroxybenzènesulfonamide.

15. Un procédé selon la revendication 1 pour la préparation du méthanesulfonate de 2-hydroxy-N-[(4-méthoxy-6-méthylpyrimidin-2-yl)aminocarbonyl]benzènesulfonamide.

16. Un procédé selon la revendication 1 pour la préparation du méthanesulfonate de N-[(4,6-diméthyl-pyrimidin-2-yl)aminocarbonyl]-2-hydroxybenzènesulfonamide.

17. Un procédé selon la revendication 1 pour la préparation du méthanesulfonate de N-[(4,6-diméthoxy-1,3,5-triazin-2-yl)aminocarbonyl]-2-hydroxybenzènesulfonamide.

18. Un procédé selon la revendication 1 pour la préparation du méthanesulfonate de 2-hydroxy-N-[(4-méthoxy-6-méthyl-1,3,5-triazin-2-yl)aminocarbonyl]benzènesulfonamide.

19. Un procédé selon la revendication 1 pour la préparation du méthanesulfonate de N-[(4,6-diméthyl-1,3,5-triazin-2-yl)aminocarbonyl]-2-hydroxybenzènesulfonamide.

20. Un procédé selon la revendication 1 pour la préparation du méthanesulfonate de N-[(4,6-diméthoxy-1,3,5-triazin-2-yl) (méthyl)aminocarbonyl]-2-hydroxybenzènesulfonamide.

21. Un procédé selon la revendication 1 pour la préparation du 1-propanesulfonate de N-[(4,5-diméthoxypyrimidin-2-yl)aminocarbonyl]-2-hydroxybenzènesulfonamide.

22. Un procédé selon la revendication 1 pour la préparation du 1-propanesulfonate de 2-hydroxy-N-[(4-méthoxy-6-méthyl-1,3,5-triazin-2-yl)aminocarbonyl]-benzènesulfonamide.

23. Une composition utile pour lutter contre la croissance de végétation indésirable, qui comprend au moins un composé de formule I comme défini dans l'une quelconque des revendications 1 à 22 en association avec au moins un des ingrédients suivants: agent tensio-actif, diluant solide et diluant liquide.

24. Un procédé pour lutter contre la croissance de végétation indésirable à un endroit, qui comprend l'application à cet endroit d'une quantité efficace d'au moins un composé de formule I comme défini dans l'une quelconque des revendications 1 à 13.

25. Un procédé selon la revendication 24, dans lequel le composé de formule I est un composé tel que défini dans l'une quelconque des revendicationa 14 à 22.

26. Un procédé pour régler la croissance de plantes à un endroit, qui comprend l'application à cet endroit d'une quantité efficace mais substantiellement non-phytotoxique d'un régulateur de la croissance de plantes choisi parmi les composés de formule I tels que définis dans l'une quelconque des revendications 1 à 22.